(19) Europäisches Patentamt / European Patent Office / Office européen des brevets

(11) **EP 4 495 264 A1**

(12) **EUROPEAN PATENT APPLICATION**

(43) Date of publication:
**22.01.2025 Bulletin 2025/04**

(21) Application number: **23382757.5**

(22) Date of filing: **21.07.2023**

(51) International Patent Classification (IPC):
*C12Q 1/6883* (2018.01)

(52) Cooperative Patent Classification (CPC):
**C12Q 1/6883;** C12Q 2600/112; C12Q 2600/124;
C12Q 2600/156

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
 • **NEIKER - Instituto Vasco de Investigación y Desarrollo Agrario, S.A.**
  **48160 Derio, Bizkaia (ES)**
 • **INIA-CSIC**
  **28040 Madrid (ES)**

(72) Inventors:
 • **ALONSO FERNÁNDEZ-PACHECO, Marta**
  **48160 Derio (ES)**

 • **GONZÁLEZ RECIO, Óscar**
  **28040 Madrid (ES)**
 • **BADÍA BRINGUÉ, Gerard**
  **48160 Derio (ES)**
 • **CANIVE RUÍZ, María**
  **48160 Derio (ES)**
 • **VÁZQUEZ ARBAIZAR, María Patricia**
  **48160 Derio (ES)**
 • **JUSTE JORDÁN, Ramón A.**
  **48160 Derio (ES)**
 • **GARRIDO URCULLU, Joseba**
  **48160 Derio (ES)**
 • **FERNÁNDEZ MUÑOZ, Almudena**
  **28040 Madrid (ES)**

(74) Representative: **Hoffmann Eitle**
 **Hoffmann Eitle S.L.U.**
 **Paseo de la Castellana 140, 3a planta**
 **Edificio LIMA**
 **28046 Madrid (ES)**

(54) **METHOD FOR CLASSIFYING AND/OR SELECTING CATTLE ACCORDING TO THEIR SUSCEPTIBILITY, TOLERANCE AND/OR RESISTANCE TO MYCOBACTERIUM AVIUM SUBSP. PARATUBERCULOSIS INFECTION**

(57)    The present invention refers to the veterinary field. Particularly, the present invention refers to a method for classifying and/or selecting cattle according to their susceptibility, tolerance and/or resistance to *Mycobacterium avium* subsp. paratuberculosis (MAP) infection.

EP 4 495 264 A1

**Description**

**FIELD OF THE INVENTION**

The present invention refers to the veterinary field. Particularly, the present invention refers to a method for classifying and/or selecting cattle according to their susceptibility, tolerance and/or resistance to *Mycobacterium avium* subsp. paratuberculosis (MAP) infection.

**STATE OF THE ART**

Paratuberculosis (PTB) or Johne's disease is a granulomatous enteritis of domestic and wild ruminants caused by *Mycobacterium avium* subsp. paratuberculosis (MAP). PTB is a major problem for animal health and must be reported to the World Organization for Animal Health. In Europe and North America, PTB is endemic in dairy cattle, with herd prevalence estimates higher than 50 %. PTB causes substantial economic losses to the dairy industry mainly due to decreased milk production, weight loss, and premature culling or death of the infected animals. Animals are infected early in life through the fecal-oral route, but clinical onset appears when animals are 18 months or older. Current diagnostic tests, ELISA and fecal PCR, often fail to detect MAP-infected cattle early in the course of infection. According to their extension, cellular infiltrate, and amount of MAP, PTB-associated histological lesions have been classified into the following categories: focal, multifocal, and diffuse (diffuse paucibacillary, diffuse intermediate, and diffuse multibacillary). High bacterial burden, clinical signs, and gross lesions are associated with the presence of diffuse lesions. MAP has been associated with Crohn's disease (CD) in humans and has been detected in samples of patients with CD, ulcerative colitis, and idiopathic inflammatory bowel disease (IBD)-associated colorectal cancer. MAP is also a possible trigger factor in some human autoimmune diseases such as rheumatoid arthritis, multiple sclerosis, and type I diabetes. Currently, no efficacious vaccine exists that confers protection against MAP. The current control measures include the removal of MAP-infected animals from the herd and the enhancement of farm-biosecurity measures. Genetic selection to enhance the resistance of dairy cattle to PTB and other diseases is extensively being explored.

The present invention is intended to use genetic markers (single nucleotide polymorphisms, SNPs) associated with susceptibility, tolerance and resistance to MAP infection to predict and classify cattle according to these phenotypes. This strategy would allow producers to select cattle less susceptible, more tolerant to PTB and resistant to MAP infection, and likely to other intracellular pathogens, ultimately reducing the prevalence of diseases and the dependence on antimicrobials, preventing economic losses, increasing the length of cattle productive life, and improving food safety. Preventing endemic and chronic diseases such as PTB by selecting tolerant and resistant cattle is important for sustainable and efficient dairy farming and the maintenance of the rural economy. Although this is a long-term control strategy, the benefits of breeding these animals are permanent and transferred to subsequent generations. In summary, the present invention is focused on a new PTB control strategy based on the use of certain SNPs for the selection of cattle according to their reduced susceptibility, and increased tolerance or resistance to MAP infection.

**DESCRIPTION OF THE INVENTION**

The present invention refers to a method for classifying and/or selecting cattle according to their susceptibility, tolerance and/or resistance to MAP infection.

Such as it is shown in **Table 1**, **Table 2** and **Table 3**, the SNPs included therein are statistically significantly associated with susceptibility to MAP **(Table 1)**, resistance to MAP **(Table 2)** or tolerance to PTB **(Table 3)**. Particularly, the presence of the alternative alleles of the SNPs of **Table 1A** is an indication that the cattle have a high probability of testing positive for MAP infection and are therefore susceptible to MAP, the presence of the alternative alleles of the SNPs of **Table 1B** is an indication that the cattle have a high probability of presenting diffuse lesions in gut tissues and are therefore susceptible to MAP, the presence of the alternative alleles of the SNPs of **Table 2A** is an indication that the cattle have a high probability of having low MAP load in macrophages and are therefore resistant to MAP, the presence of the alternative alleles of the SNPs of **Table 2B** is an indication that bovine blood samples have a high probability of producing high levels of IFN-gamma after stimulation with avian tuberculin and are therefore resistant to MAP, the presence of the alternative alleles of the SNPs of **Table 3A** is an indication that the cattle have a high probability of presenting no lesions in gut tissues despite testing positive for MAP infection and are therefore tolerant to PTB, and the presence of the alternative alleles of the SNPs of **Table 3B is** an indication that the cattle have a high probability of having multifocal, but not diffuse, lesions in gut tissues and are therefore tolerant to PTB.

**Table 4** shows the AUC value of the genetic predictive models (GBLUP) that allow the classification of cattle according to their susceptibility, tolerance or resistance to MAP infection.

It is important to note that, such as it is indicated in **Table 1, Table 2** and **Table 3,** by means of the p-value and false discovery rate (FDR), each individual SNP is statistically significantly associated with the specific phenotype (susceptibility, resistance or tolerance). This means that any individual SNP, preferably any combination of any number of the SNP included in **Table 1, Table 2** and **Table 3,** more preferably the combination of all the SNP included in **Tables 1, Table 2** and **Table 3,** can be used for classifying and/or selecting cattle according to their susceptibility, tolerance and/or resistance to MAP infection.

So, the first embodiment of the present invention refers to an *in vitro* method (hereinafter *method of the invention*) for classifying and/or selecting cattle according to their susceptibility, tolerance and/or resistance to *Mycobacterium avium* subsp. paratuberculosis (MAP) infection, which comprises:

a) determining the identity of at least one single nucleotide polymorphism (SNP) selected from the list shown in **Tables 1, 2 or 3** in a biological sample obtained from cattle, wherein determining the identity of the at least one SNP comprises determining the identity of the allele present at the SNP position or determining the identity of a genetic variant in linkage disequilibrium with the allele present at the SNP position,
b) wherein the identification of the alternative allele of at least one SNP selected from the list shown in **Table 1** is an indication that the cattle are susceptible to MAP; or
c) wherein the identification of the alternative allele of at least one SNP selected from the list shown in **Table 2** is an indication that the cattle are resistant to MAP; or
d) wherein the identification of the alternative allele of at least one SNP selected from the list shown in **Table 3** is an indication that the cattle are tolerant to PTB.

In a preferred embodiment, the method is for classifying and/or selecting cattle according to their susceptibility to MAP, wherein susceptibility is determined according to their probability of testing positive for MAP infection, which comprises:

a) determining the identity of at least one SNP selected from the list shown in **Table 1A** in a biological sample obtained from cattle, wherein determining the identity of the at least one SNP comprises determining the identity of the allele present at the SNP position or determining the identity of a genetic variant in linkage disequilibrium with the allele present at the SNP position,
b) wherein the identification of the alternative allele of at least one SNP selected from the list shown in **Table 1A**, is an indication that the cattle have a high probability of testing positive for MAP infection and are therefore susceptible to MAP.

In a preferred embodiment, the method is for classifying and/or selecting cattle according to their susceptibility to MAP, wherein susceptibility is determined according to their probability of presenting diffuse lesions in gut and associated lymphoid tissues, which comprises:

a) determining the identity of at least one SNP selected from the list shown in **Table 1B** in a biological sample obtained from cattle, wherein determining the identity of the at least one SNP comprises determining the identity of the allele present at the SNP position or determining the identity of a genetic variant in linkage disequilibrium with the allele present at the SNP position,
b) wherein the identification of the alternative allele of at least one SNP selected from the list shown in **Table 1B** is an indication that the cattle have a high probability of presenting diffuse lesions in gut tissues and are therefore susceptible to MAP.

In a preferred embodiment, the method is for classifying and/or selecting cattle according to their resistance to MAP, wherein resistance is determined according to their probability of having low MAP load in macrophages, which comprises:

a) determining the identity of at least one SNP selected from the list shown in **Table 2A** in a biological sample obtained from cattle, wherein determining the identity of the at least one SNP comprises determining the identity of the allele present at the SNP position or determining the identity of a genetic variant in linkage disequilibrium with the allele present at the SNP position,
b) wherein the identification of the alternative allele of at least one SNP selected from the list shown in **Table 2A** is an indication that the cattle have a high probability of having low MAP load in macrophages and are therefore resistant to MAP.

In a preferred embodiment, the method is for classifying and/or selecting cattle according to their resistance to MAP, wherein resistance is determined according to their probability of producing high levels of IFN-gamma in blood stimulated with avian tuberculin, which comprises:

a) determining the identity of at least one SNP selected from the list shown in **Table 2B** in a biological sample obtained from cattle, wherein determining the identity of the at least one SNP comprises determining the identity of the allele present at the SNP position or determining the identity of a genetic variant in linkage disequilibrium with the allele present at the SNP position,

b) wherein the identification of the alternative allele of at least one SNP selected from the list shown in **Table 2B** is an indication that blood samples from the cattle have a high probability of producing high levels of IFN-gamma after stimulation with avian tuberculin and are therefore resistant to MAP.

In a preferred embodiment, the method is for classifying and/or selecting cattle according to their tolerance to PTB, wherein tolerance is determined according to their probability of having no lesions in gut tissues despite testing positive for MAP infection, which comprises:

a) determining the identity of at least one SNP selected from the list shown in **Table 3A** in a biological sample obtained from cattle, wherein determining the identity of the at least one SNP comprises determining the identity of the allele present at the SNP position or determining the identity of a genetic variant in linkage disequilibrium with the allele present at the SNP position,

b) wherein the identification of the alternative allele of at least one SNP selected from the list shown in **Table 3A** is an indication that the cattle have a high probability of presenting no lesions in gut tissues despite testing positive for MAP infection and are therefore tolerant to PTB.

In a preferred embodiment, the method is for classifying and/or selecting cattle according to their tolerance to PTB, wherein tolerance is determined according to their probability of having multifocal, but not diffuse, lesions in gut tissues, which comprises:

a) determining the identity of at least one SNP selected from the list shown in **Table 3B** in a biological sample obtained from cattle, wherein determining the identity of the at least one SNP comprises determining the identity of the allele present at the SNP position or determining the identity of a genetic variant in linkage disequilibrium with the allele present at the SNP position,

b) wherein the identification of the alternative allele of at least one SNP selected from the list shown in **Table 3B** is an indication that the cattle have a high probability of having multifocal, but not diffuse, lesions in gut tissues and are therefore tolerant to PTB.

In a preferred embodiment, the method of the invention comprises:

a) determining the identity of all the SNPs selected from the list shown in **Tables 1, 2 or** 3 in a biological sample obtained from cattle, wherein determining the identity of the at least one SNP comprises determining the identity of the allele present at the SNP position or determining the identity of a genetic variant in linkage disequilibrium with the allele present at the SNP position,

b) wherein the identification of the alternative allele of all the SNPs of **Table 1**, is an indication that the cattle are susceptible to MAP; or

c) wherein the identification of the alternative allele of all the SNPs of **Table 2**, is an indication that the cattle are resistant to MAP; or

d) wherein the identification of the alternative allele of all the SNPs of **Table 3,** is an indication that the cattle are tolerant to PTB.

In a preferred embodiment, the test for MAP infection is performed by ELISA, tissue PCR, bacteriological culture for MAP detection, histopathological analysis, Interferon gamma release assay (IGRA) and/or MGIA (Mycobacterial growth inhibition assay), and the step a) is performed by using PCR, qPCR, allele-specific PCR, allele-specific qPCR, SNP array, whole genome sequencing, genomic prediction and/or genotype imputation.

In a preferred embodiment, the cattle are selected from the group comprising: a cow, a bull, heifers, bullocks, steers, beef cattle and dairy cattle.

In a preferred embodiment, the biological sample is a nucleic acid sample, preferably a nucleic acid sample derived from peripheral blood.

The second embodiment of the present invention refers to the *in vitro* use of at least one or all the SNPs selected from the list shown in **Tables 1, 2 or 3**, or of a genetic variant in linkage disequilibrium with the at least one or all the SNPs, or of a kit comprising reagents for the determination of at least one or all the SNPs selected from the list shown in **Tables 1, 2 or 3,** or of a genetic variant in linkage disequilibrium with at least one or all the SNPs, for classifying and/or selecting cattle according to their susceptibility, tolerance and/or resistance to MAP infection.

In a preferred embodiment, the present invention refers to the *in vitro* use of at least one or all the SNPs selected from the list shown in **Table 1A**, or of a genetic variant in linkage disequilibrium with the at least one or all the SNPs, or of a kit comprising reagents for the determination of at least one or all the SNPs selected from the list shown in **Table 1A**, or of a genetic variant in linkage disequilibrium with the at least one or all the SNPs, for classifying and/or selecting cattle according to their susceptibility to MAP infection by assessing their probability of testing positive for MAP infection; or use of at least one or all the SNPs selected from the list shown in **Table 1B**, or of a genetic variant in linkage disequilibrium with the at least one or all the SNPs, or of a kit comprising reagents for the determination of at least one or all the SNPs selected from the list shown in **Table 1B**, or of a genetic variant in linkage disequilibrium with the at least one or all the SNPs, for classifying and/or selecting cattle according to their susceptibility to MAP infection by assessing their probability of presenting diffuse lesions in gut tissues.

In a preferred embodiment, the present invention refers to the *in vitro* use of at least one or all the SNPs selected from the list shown in **Table 2A**, or of a genetic variant in linkage disequilibrium with the at least one or all the SNPs, or of a kit comprising reagents for the determination of at least one or all the SNPs selected from the list shown in **Table 2A**, or of a genetic variant in linkage disequilibrium with the at least one or all the SNPs, for classifying and/or selecting cattle according to their resistance to MAP infection by assessing their probability of having low MAP load in macrophages; or use of at least one or all the SNPs selected from the list shown in **Table 2B**, or of a genetic variant in linkage disequilibrium with the at least one or all the SNPs, or of a kit comprising reagents for the determination of at least one or all the SNPs selected from the list shown in **Table 2B**, or of a genetic variant in linkage disequilibrium with the at least one or all the SNPs, for classifying and/or selecting cattle according to their resistance to MAP infection by assessing their probability of producing high levels of IFN-gamma in response to avian tuberculin.

In a preferred embodiment, the present invention refers to the *in vitro* use of at least one or all the SNPs selected from the list shown in **Table 3A**, or of a genetic variant in linkage disequilibrium with the at least one or all the SNPs, or of a kit comprising reagents for the determination of at least one or all the SNPs selected from the list shown in **Table 3A**, or of a genetic variant in linkage disequilibrium with the at least one or all the SNPs, for classifying and/or selecting cattle according to their tolerance to PTB by assessing their probability of having no lesions in gut tissues despite testing positive for MAP infection; or use of at least one or all the SNPs selected from the list shown in **Table 3B**, or of a genetic variant in linkage disequilibrium with the at least one or all the SNPs, or of a kit comprising reagents for the determination of at least one or all the SNPs selected from the list shown in **Table 3B**, or of a genetic variant in linkage disequilibrium with the at least one or all the SNPs, for classifying and/or selecting cattle according to their tolerance to PTB by assessing their probability of having multifocal, but not diffuse, lesions in gut tissues.

In a preferred embodiment, the present invention refers to the *in vitro* use of at least one or all the alternative alleles of the SNPs.

The present invention also refers to a method for detecting SNPs in a test sample from cattle with susceptibility, tolerance, and/or resistance to MAP infection by following the methods described in the present invention.

The present invention also refers to a method for analysing a biological sample isolated from cattle in order to detect detecting susceptibility, tolerance and/or resistance to MAP which comprises: determining the identity of at least one single nucleotide polymorphism (SNP) selected from the list shown in **Tables 1, 2 or 3** in a biological sample obtained from cattle, wherein determining the identity of the at least one SNP comprises determining the identity of the allele present at the SNP position or determining the identity of a genetic variant in linkage disequilibrium with the allele present at the SNP position, wherein the identification of the alternative allele of at least one SNP of **Table 1** is an indication of susceptibility to MAP, wherein the identification of the alternative allele of at least one SNP of **Table 2** is an indication of resistance to MAP, and wherein the identification of the alternative allele of at least one SNP of **Table 3** is an indication of tolerance to PTB.

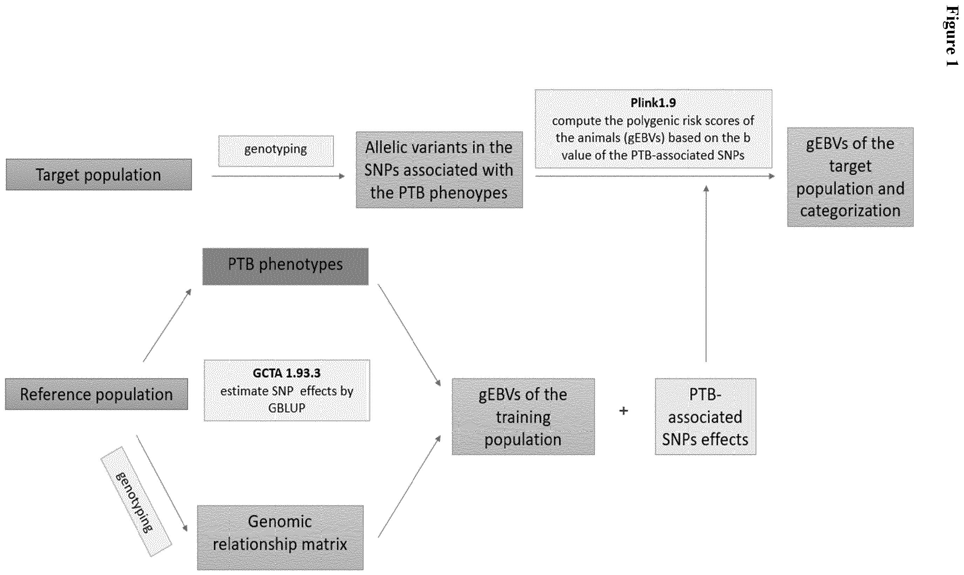

In a preferred embodiment, the present invention is a computer-implemented invention, wherein a processing unit (hardware) and a software are configured to: a) Receive the information about the identity of at least one SNP, b) process information for categorizing the animals as described above, and c) provide an output through a terminal display **(Figure 1)**.

For the purpose of the present invention the following terms are defined:

- The term "comprising" means including, but not limited to, whatever follows the word "comprising". Thus, use of the term "comprising" indicates that the listed elements are required or mandatory, but that other elements are optional and may or may not be present.
- The term "consisting of" means including, and limited to, whatever follows the phrase "consisting of". Thus, the phrase "consisting of" indicates that the listed elements are required or mandatory, and that no other elements may be present.
- The term, "identification of the alternative allele" refers to the identification of the allele of the SNP that is associated to the indicated trait (e. g. susceptibility, resistance, tolerance). The alternative alleles of each SNP are represented in **Table 1, 2** and **3** (see table legend). The determination of the identity of an allele can be performed by determining the genetic variant present at the SNP position or by determining the identity of a genetic variant in linkage disequilibrium with the allele present at the SNP position. In the context of the present application, the alternative allele is considered identified when it has been identified through either of these methods.

- The term "susceptibility, tolerance and/or resistance to MAP infection" refers to susceptibility to MAP infection, resistance to MAP infection, and tolerance to the disease caused by MAP, which is PTB. Cattle are considered susceptible when they have a positive ELISA, PCR and culture result and/or when they show diffuse lesions in gut tissues. Cattle are considered tolerant to the disease when they show multifocal lesions in gut tissues or when they do not show histopathological lesions in gut tissues despite testing positive for MAP infection. Cattle are considered resistant to MAP infection when they have low MAP load within *ex vivo* infected macrophages and/or when they are able to produce high levels of IFNγ in response to avian tuberculin stimulation.

**Description of the figures**

**Figure 1.** Pipeline for the prediction of the estimated breeding value of each individual attributed to the aggregative effect of the SNPs associated with susceptibility, tolerance or resistance to MAP infection.

**Detailed description of the invention**

The present invention is illustrated by means of the Examples set below without the intention to limit its scope of protection.

**Example 1. Materials and methods**

**Example 1.1. Ethical statement**

Animals used in the present invention were not submitted to any *in vivo* experimentation. Blood sampling was conducted with the approval of the Ethics Committee of Animal Experimentation of NEIKER (OEBA-2022-010), according to pertinent Basque (Basque Government Decree 454/1994), Spanish (Spanish Government Law 32/2007/7 and Royal decree 53/2013/1), and European (Community Council Animal Welfare Guidelines 2010/63/EU) regulations.

**Example 1.2. Animals and blood samples**

Example 1.2.1. Animals used for susceptibility assessment

*Animals used for PTB diagnosis by ELISA, and tissue PCR and culture*

The Spanish Holstein population consisted of 983 culled Holstein cattle from several herds located in eight regions: Basque Country (N= 415, 42.21%), Catalonia (N= 220, 22.38 %), Navarre (N= 204, 20.75%), Cantabria (N= 70, 7.12%), Aragon (N= 38, 3.87 %), Castile and Leon (N= 25, 2.54 %), La Rioja (N= 7, 0.71 %) and Asturias (N= 4, 0.41 %). Only cows (2 years or older, 5.6 years mean age) were included in the analyses as PTB has a long incubation period and older animals are, therefore, more likely to show clinical signs and positive diagnostic results.

*Animals used for histopathological analysis*

The Spanish Holstein population consisted of 813 culled Holstein cattle from several herds located in eight regions: Basque Country (N= 212, 26.08 %), Catalonia (N= 318, 39.11 %), Navarre (N= 170, 20.91 %), Cantabria (N= 59, 7.26 %), Aragon (N= 24, 2.95 %), Castile and Leon (N= 21, 2.58 %), La Rioja (N= 6, 0.74 %) and Asturias (N= 3, 0.37 %). Only cows (18 months years or older, 5.5 years mean age) were included in the analyses as PTB has a long incubation period and older animals are, therefore, more likely show clinical signs.

Example 1.2.2. Animals used for resistance assessment

*Animals used for the assessment of MAP load in macrophages*

The reference population consisted of 75 Spanish Holstein cows from a single farm located in the Basque Country. In 2023, all the cows had a negative fecal PCR result. Only adult cows (2 years or older, 4.5 years mean age) were included in the study. All the animals included in this study were registered with the Spanish Federation of Holstein cattle (CONAFE; www.conafe.com.) and had negative fecal PCR result when blood samples were collected and in subsequent annual samplings. In addition, the animals used for the study were not diagnosed with any other pathogen.

*Animals used for the assessment of IFN gamma production*

The study population consisted of 152 culled Holstein cattle from several herds located in seven Spanish regions: Basque Country (N = 45), Catalonia (N = 67), Navarre (N = 1), Cantabria (N = 30), Aragon (N = 1), Castile and Leon (N = 5) and Asturias (N = 1); along with 2 slaughtered cows of unknown origin. All the cows were older than 2 years. In each weekly visit to the abattoirs, from two to six Holstein cows older than two-year-old were sampled.

Example 1.2.3. Animals used for tolerance assessment

The study population consisted of 685 culled Spanish Holstein cattle from several herds located on seven regions: Basque Country (N= 286, 41.75%), Catalonia (N= 157, 22.92 %), Navarre (N= 147, 21.46 %), Cantabria (N= 49, 7.15 %), Aragon (N= 22, 3.21 %), Castile and Leon (N= 16, 2.34 %), La Rioja (N= 5, 0.73 %) and Asturias (N=3, 0.44%). All cows were 18 months years or older (5.6 years mean age).

**Example 1.3. Histopathological examination.**

Post-mortem tissue sampling was performed. Briefly, samples from ileocecal lymph node, jejunal lymph node, ileocecal valve (ICV), jejunum, and terminal ileum were collected aseptically from each animal and placed in formalin within 24 h after arrival at the laboratory. The samples were preferentially taken from areas of the preselected tissues that showed gross lesions, thickened mucosa and enlarged lymphatic nodes, if present. The samples were fixed in 10% neutral buffered formalin for 72 h, dehydrated through graded alcohols and xilol, embedded in paraffin, and cut into 4 $\mu$m sections. Sections were, mounted on treated microscope slides, stained with haematoxylin and eosin (HE) and with Ziehl-Neelsen (ZN) and examined for pathological lesions and for the presence of acid-fast bacteria, respectively. According to their location and extension, inflammatory cell type, and mycobacterial load, PTB-associated histopathological lesions were classified in focal, multifocal, and diffuse lesions.

**Example 1.4. Tissue PCR and bacteriological culture, and ELISA.**

A pool (2 gr) of ICV, distal ileum and the jejunal caudal lymph nodes was decontaminated with 38 ml of hexadecyl pyridinium chloride at a final concentration of 0.75% (Sigma, St. Louis, MO) and homogenized in a stomacher blender. After 30 min of incubation at room temperature, 15 ml of the suspension was transferred to a new tube and incubated overnight for decontamination and sedimentation. Approximately, 200 $\mu$l of the suspension was taken and inoculated in duplicate Herrold's egg yolk (Becton Dickinson, Franklin Lakes, NJ, USA) and Lowenstein-Jensen media (Difco, Detroit, MI, USA), both supplemented with 2 mg/L of mycobactin J (Allied Monitor, Fayette, MO, USA). A second aliquot from the same tissue homogenates was used for DNA isolation and MAP IS900 amplification (Adiagene, Saint Brieuc, France). PCR amplifications were performed on an ABI Prism 7000 Sequence Detection System (Applied Biosystems, Foster City, CA, US). Samples with a threshold cycle (Ct) below 40.0 were considered positive. Antibody production against MAP in serum samples was tested using a two-step ELISA PTB antibody screening and verification kit (IDEXX Laboratories, Inc., Westbrook, ME, USA) according to the manufacturer's instructions. The results were categorized as positive according to the sample-to-positive control ratio defined by the manufacturer.

**Example 1.5. Quantification of MAP load in macrophages**

Peripheral blood monocytes (PBMCs) were purified from blood samples, differentiated to monocyte-derived macrophages (MDMs), infected with MAP K10 isolate, and at 2 h and 7 d post-infection (p. i.) the intracellular load was quantified in the Bactec MGIT 960 system. Briefly, supplemented Mycobacteria Growth Indicator tubes (MGIT) (Becton, Dickinson, and Company, Sparks, MD) were inoculated with 0.5 ml of the cell lysates. The tubes were incubated at 37 $\pm$ 2°C for up to 42 days in a Bactec MGIT 960 instrument (Becton, Dickinson, and Company). The earliest instrumental indicator of positivity (i.e., time to detection [TTD]) for each tube was recorded. The predicted number of bacteria in each positive tube was calculated using standard curves that relate TTD (in days) to the estimated log CFUs. For the standard curves, a ten-fold dilution series of a MAP K10 strain cellular suspension (McFarland= 1) were prepared and the TTDs of 100 $\mu$l of each dilution in the BACTEC MGIT 960 system were detected. MAP (log CFUs) were plotted versus TTDs and the obtained mathematical equation was used to determine the estimated log CFUs for each sample. The log CFU ratios were calculated by dividing the estimated log CFUs at day 7 by that at 2 h p.i. MAP. Survival indexes were calculated according to Martinez et al., (2008) and Price et al., (2009) as the square root CFU ratio percentage at 7 days p.i. with respect to 2 h p.i. Lower values of the MAP survival index reflect higher resistance to the infection.

### Example 1.6. Interferon-gamma release assay (IGRA)

Blood stimulation was performed within the first eight hours after blood collection. Briefly, four 1.4 ml aliquots of lithium heparinized whole blood samples from each animal were added to four wells of a 24-well plate (Becton Dickinson, Franklin lakes, NJ, USA). The blood samples were then stimulated with 100 $\mu$l of phosphate-buffered saline (PBS), 100 $\mu$l of avian purified protein derivative (aPPD) (0.3 $\mu$g/$\mu$L) (CZ Veterinaria® SA, Porriño, Spain), 100 $\mu$l of bovine purified protein derivative (bPPD) (0.3 $\mu$g/$\mu$L) (CZ Veterinaria® SA, Porriño, Spain) and 100 $\mu$l of lectin (1 $\mu$g/ml) as positive control. After incubation for 16-24 hours at 37 °C in a 5% CO2 incubator, the plasmas were separated by centrifugation at 500 g for 10 minutes at room temperature (RT) and then were frozen at -20 °C until testing. Subsequently, IFNy levels were measured by triplicate in the plasma samples using a specific IFNy ELISA test according to the manufacturer's instructions (BovigamTM, Prionics, Schlieren, Switzerland). Briefly, 50 $\mu$l of plasma diluent and 50 $\mu$l of each stimulated sample were pipetted into a 96-well plate and the plate was incubated at RT for 60 minutes. The plate was then washed 6 times, 100 $\mu$l of the conjugate antibody was added and the plate was incubated at RT for 60 minutes. After 6 more washes, 100 $\mu$l of the chromogen solution was added and the plate was incubated for 30 minutes at RT. Finally, 50 $\mu$l of STOP solution were added, and the plate was read at 650 and 450 nm (final OD value = OD450 - OD650). IFNy levels were expressed as the OD of the aPPD or bPPD-stimulated plasmas minus the OD of the PBS-stimulated samples. The 152 cows from the 343 animals with an IGRA record were selected because they had an OD (aPPD-PBS) higher than the OD (bPPD-PBS) and the OD (aPPD) was higher than the OD (PBS) as well. This selection avoids false positive reactions with *Mycobacterium bovis* infection and negative OD values, respectively.

### Example 1.7. Genotyping and imputation

Peripheral blood samples were collected in EDTA tubes and DNA was extracted using the QIAmp DNA Blood Mini Kit according to the manufacturer's instructions (Qiagen, Hilden, Germany). Purified genomic DNA was genotyped with the EuroG MD Bead Chip at the molecular genetic laboratory service of the Spanish Federation of Holstein Cattle (CONAFE). The InfiniumTM iScan software (Illumina, San Diego, CA) was used for allele assignation. Individual genotypes were phased using Eagle 2.4 and imputed with minimac4 1.0.2 to the Bovine HD Bead Chip using a reference panel of 1,278 Holstein bulls from Run7.0 of the 1,000 Bull Genomes project and 581,712 SNPs (ASR-UCD1.2). Imputation to WGS level was then undertaken using the same phasing and imputation procedure and a reference population of 2,333 *Bos taurus* from Run7.0 of the 1,000 Bull Genomes project. Finally, the following filters were applied: call rate > 0.80, minimum allele frequency (MAF) > 0.01, and imputation score ($r^2$) > 0.7. The final number of SNPs per animal was 12,856,161.

### Example 1.8. Variance components, h2 estimation and GWAS

The phenotypes analyzed were:

- Susceptibility

    ○ Infection confirmed by ELISA, PCR and bacteriological culture for MAP

        ▪ Cases (n = 55): ELISA, PCR, culture (+)
        ▪ Controls (n = 658): ELISA, PCR, culture (-)

    ○ Presence of diffuse lesions in gut tissues

        ▪ Cases (n = 36): Diffuse lesions (+)
        ▪ Controls (n = 373): No lesions

- Resistance

    ○ MAP load within macrophages
    ▪ The CFU data from macrophages infected ex vivo from 61 Holstein cattle was the quantitative phenotype in the GWAS analysis
    ○ IFN-gamma production in avian tuberculin stimulated blood
    ▪ The OD data from stimulated blood from 152 bovine animals was the quantitative phenotype in the GWAS

- Tolerance

◦ Presence of multifocal, but not diffuse lesions in gut tissues

- Cases (n = 33): Multifocal lesions (+)
- Controls (n = 373): No lesions

◦ Infection confirmed by tissue PCR and culture but without lesions in gut tissues

- Cases (n = 24): tissue PCR and culture (+) and no lesions
- Controls (n = 245): tissue PCR and culture (-) with focal lesions

The variance components and h2 explained by all the SNPs were calculated using the genome-wide complex trait analysis (GCTA) software 1.93.2 according to the following formula:

$$h^2 = \frac{\sigma_G^2}{\sigma_G^2 + \sigma_e^2}$$

where $\sigma_G^2$ would represent the variance explained by all the SNPs and $\sigma_e^2$ the residual variance. The WGS data and the phenotypes were analyzed using the mixed linear model association analysis of the GCTA 1.93.2 software, expressed as y = a + bx + g + e. In the model, y is the phenotype, a the mean term, b the allele effect, x the genotype of the SNP coded as 0, 1 or 2 depending on how many copies of the minor allele has the animal, g the polygenic effect as a random effect (assumed to be distributed as $N\sim(0, \sigma_e^2)$ and e the residual effect (also assumed to be distributed as $N\sim(0, \sigma_e^2))$. Age was included as a covariate in the analysis. To account for multiple testing, a 5 % genome-wide false discovery rate (FDR) was used. A threshold of P-value $\leq 5 \times 10^{-7}$ was used as suggested by the Wellcome Trust Case Control Consortium. The SNP effects (b-values) were also calculated with the GCTA 1.93.2 software. If the sign of the b-value is positive, it implies that there is a positive relationship between the SNP and the trait.

The EuroG MD Bead chip is based on the University of Maryland UMD 3.1 *Bos taurus* reference genome; hence, the coordinates of the GWAS-identified SNPs were converted to the ARS-UCD1.2 genome by using *liftOver* software (https://genome.ucsc.edu). The location of the identified SNPs in the ARS-UCD1.2 genome was determined using the Ensembl Variant Effect predictor (VEP).

**Example 2. Results**

The inventors of the present invention have identified 1140 SNPs that are significantly associated to susceptibility to MAP infection **(Table 1)**. More specifically, 398 of these SNPs **(Table 1A)** are associated to a positive ELISA, PCR and bacteriological culture, and 752 of these SNPs **(Table 1B)** are associated to the presence of diffuse lesions in gut tissues. Ten of these SNPs (represented with a grey shade in **Table 1**) were associated to both susceptibility traits.

In addition, 316 SNPs that are significantly associated to resistance to MAP infection were identified **(Table 2)**. More specifically, 6 of these SNPs are negatively associated to intracellular MAP load in macrophages **(Table 2A)**, meaning that the SNPs are associated to low MAP load, which is indicative of a high clearing efficiency, and 310 of these SNPs are positively associated to IFN-gamma production in response to avian tuberculin **(Table 2B)**, meaning that the SNPs are associated to a high IFN-gamma production, which is indicative of a strong response to MAP. None of these SNPs were associated to both resistance traits.

Finally, they have identified 591 SNPs that are significantly associated to tolerance to PTB **(Table 3)**. More specifically, 142 of these SNPs are associated to infection confirmed by tissue PCR and bacteriological culture but without lesions in gut tissues **(Table 3A)**, and 449 of these SNPs are associated the presence of multifocal, but not diffuse, lesions in gut tissues **(Table 3B)**. None of these SNPs were associated to both tolerance traits.

Importantly, the specific alleles associated with the above-mentioned traits are indicated in **Tables 1, 2** and **3.**

**Table 1.** SNPs associated to susceptibility traits, including (A) infection confirmed by ELISA, PCR and bacteriological culture for MAP and (B) Presence of diffuse lesions in gut tissues. SNP IDs are expressed as follows: *chromosome: position: reference allele: alternative allele*, wherein the alternative allele is the allele associated with the indicated trait. SNPs that common to A and B (10 SNPs) are represented with a grey shade.

| SNPs associated to susceptibility | | | | | |
|---|---|---|---|---|---|
| A) Infection confirmed by ELISA, PCR and bacteriological culture for MAP | | | B) Presence of diffuse lesions in gut tissues | | |
| ID | p value | FDR value | ID | p value | FDR value |
| 23:15594386:CCATGGGGT:C | 1.06E-09 | 0.004 | 13:67404014:A:C | 4.70E-10 | 0.006 |
| 23:19329213:C:A | 1.26E-09 | 0.004 | 13:67329928:T:C | 9.47E-10 | 0.006 |
| 23:19331156:C:T | 1.59E-09 | 0.004 | 7:16113384:G:C | 2.91E-09 | 0.012 |
| 23:19326305:C:T | 2.16E-09 | 0.004 | 13:67403427:T:G | 4.68E-09 | 0.015 |
| 23:22401117:G:C | 3.19E-09 | 0.004 | 8:78394983:C:T | 9.77E-09 | 0.021 |
| 23:22398408:A:G | 3.19E-09 | 0.004 | 8:78394984:T:G | 9.77E-09 | 0.021 |
| 23:22401993:A:G | 3.19E-09 | 0.004 | 7:16775615:G:A | 2.75E-08 | 0.027 |
| 23:22401991:TA:T | 3.19E-09 | 0.004 | 13:68298816:T:C | 3.57E-08 | 0.027 |

| | | | | | |
|---|---|---|---|---|---|
| 23:22399252:T:G | 3.19E-09 | 0.004 | 13:68299164:T:C | 3.57E-08 | 0.027 |
| 23:15753782:AATGCAC:A | 3.71E-09 | 0.004 | 13:68299183:A:C | 3.57E-08 | 0.027 |
| 23:15750163:A:G | 3.71E-09 | 0.004 | 7:16333421:C:T | 4.21E-08 | 0.027 |
| 23:19332117:GATCCCTGGGGC:G | 5.71E-09 | 0.004 | 7:16419302:G:A | 4.21E-08 | 0.027 |
| 23:19332281:G:A | 5.71E-09 | 0.004 | 7:16468390:C:A | 4.21E-08 | 0.027 |
| 23:19332271:G:T | 5.71E-09 | 0.004 | 7:16485206:G:A | 4.21E-08 | 0.027 |
| 23:19332328:T:C | 5.71E-09 | 0.004 | 7:16487526:G:A | 4.21E-08 | 0.027 |
| 23:19332257:A:G | 5.71E-09 | 0.004 | 7:16500822:C:T | 4.21E-08 | 0.027 |
| 23:19446723:G:A | 8.49E-09 | 0.004 | 28:41937984:A:T | 6.86E-08 | 0.027 |
| 23:19450840:T:C | 8.49E-09 | 0.004 | 28:41938126:C:T | 6.86E-08 | 0.027 |
| 23:19320649:T:C | 9.05E-09 | 0.004 | 7:16542919:A:T | 7.02E-08 | 0.027 |
| 23:19325462:T:G | 9.05E-09 | 0.004 | 7:16547785:G:A | 7.02E-08 | 0.027 |
| 23:19321362:G:GA | 9.05E-09 | 0.004 | 7:16550370:T:C | 7.02E-08 | 0.027 |
| 23:19318903:T:C | 9.05E-09 | 0.004 | 7:16577414:G:A | 7.02E-08 | 0.027 |
| 23:19321836:G:T | 9.05E-09 | 0.004 | 7:26536434:A:G | 1.12E-07 | 0.027 |
| 23:19322366:C:T | 9.05E-09 | 0.004 | 7:26536539:T:C | 1.12E-07 | 0.027 |
| 23:19321435:G:A | 9.05E-09 | 0.004 | 7:26537145:T:G | 1.12E-07 | 0.027 |
| 23:19322267:A:T | 9.05E-09 | 0.004 | 7:26539799:G:A | 1.12E-07 | 0.027 |
| 23:19319554:C:T | 9.05E-09 | 0.004 | 7:26540250:T:C | 1.12E-07 | 0.027 |
| 23:19325169:G:A | 9.05E-09 | 0.004 | 7:26540857:G:A | 1.12E-07 | 0.027 |
| 23:19325956:C:G | 9.05E-09 | 0.004 | 7:26541076:T:C | 1.12E-07 | 0.027 |
| 23:19324363:A:T | 9.05E-09 | 0.004 | 7:26541461:C:T | 1.12E-07 | 0.027 |
| 23:19319423:A:G | 9.05E-09 | 0.004 | 7:26541927:T:A | 1.12E-07 | 0.027 |

| | | | | | |
|---|---|---|---|---|---|
| 23:19319853:C:T | 9.05E-09 | 0.004 | 7:26547429:T:G | 1.12E-07 | 0.027 |
| 23:19329092:G:A | 1.54E-08 | 0.005 | 7:26547965:C:T | 1.12E-07 | 0.027 |
| 23:19315036:C:T | 1.67E-08 | 0.005 | 7:26558979:T:A | 1.12E-07 | 0.027 |
| 23:19313754:G:C | 1.67E-08 | 0.005 | 7:16426929:G:A | 1.26E-07 | 0.027 |
| 23:19315552:G:A | 1.67E-08 | 0.005 | 7:15525527:A:C | 1.67E-07 | 0.027 |
| 23:19312221:C:T | 1.67E-08 | 0.005 | 7:15525543:T:G | 1.67E-07 | 0.027 |
| 23:19312971:T:G | 1.67E-08 | 0.005 | 7:24296173:C:T | 1.73E-07 | 0.027 |
| 23:19317414:A:T | 1.67E-08 | 0.005 | 7:24367043:C:G | 1.73E-07 | 0.027 |
| 23:19316615:G:A | 1.67E-08 | 0.005 | 7:24586968:G:A | 1.73E-07 | 0.027 |
| 23:19316797:A:G | 1.67E-08 | 0.005 | 7:16527694:G:C | 1.79E-07 | 0.027 |
| 23:19315940:C:A | 1.67E-08 | 0.005 | 7:16532523:G:C | 1.79E-07 | 0.027 |
| 23:19317996:T:C | 1.67E-08 | 0.005 | 7:16532696:G:A | 1.79E-07 | 0.027 |
| 23:19313709:C:T | 1.67E-08 | 0.005 | 7:19326957:C:T | 1.97E-07 | 0.027 |
| 23:19316642:G:A | 1.67E-08 | 0.005 | 1:97523460:A:C | 2.78E-07 | 0.027 |
| 23:19317731:TA:T | 1.67E-08 | 0.005 | 1:97506475:A:G | 2.82E-07 | 0.027 |
| 23:19316034:A:AAAT | 1.67E-08 | 0.005 | 1:97506747:G:A | 2.82E-07 | 0.027 |
| 23:19327112:TA:T | 1.71E-08 | 0.005 | 1:97506756:A:G | 2.82E-07 | 0.027 |
| 23:19329007:A:G | 1.71E-08 | 0.005 | 1:97506819:G:C | 2.82E-07 | 0.027 |
| 23:19328635:A:G | 1.71E-08 | 0.005 | 1:97507455:A:G | 2.82E-07 | 0.027 |
| 23:19329026:A:G | 1.71E-08 | 0.005 | 1:97508006:G:A | 2.82E-07 | 0.027 |
| 23:19331429:G:A | 1.94E-08 | 0.005 | 1:97509200:T:C | 2.82E-07 | 0.027 |
| 23:19331002:G:C | 1.94E-08 | 0.005 | 1:97509624:T:A | 2.82E-07 | 0.027 |
| 23:19330011:C:T | 1.94E-08 | 0.005 | 1:97510594:C:T | 2.82E-07 | 0.027 |
| 23:19329934:T:C | 1.94E-08 | 0.005 | 1:97511821:T:C | 2.82E-07 | 0.027 |

| | | | | | |
|---|---|---|---|---|---|
| 23:19330418:G:T | 1.94E-08 | 0.005 | 1:97513662:G:A | 2.82E-07 | 0.027 |
| 23:19330961:C:T | 1.94E-08 | 0.005 | 1:97521005:T:C | 2.82E-07 | 0.027 |
| 23:11900592:A:G | 2.32E-08 | 0.005 | 1:97523969:C:A | 2.82E-07 | 0.027 |
| 23:11893154:T:C | 2.32E-08 | 0.005 | 1:97548872:T:A | 2.82E-07 | 0.027 |
| 23:11887787:T:C | 2.32E-08 | 0.005 | 1:97553066:C:T | 2.82E-07 | 0.027 |
| 23:11892301:G:A | 2.32E-08 | 0.005 | 1:97558645:T:A | 2.82E-07 | 0.027 |
| 23:19431532:C:T | 2.48E-08 | 0.006 | 1:97561108:A:G | 2.82E-07 | 0.027 |
| 23:20110605:G:A | 2.65E-08 | 0.006 | 1:97563364:C:T | 2.82E-07 | 0.027 |
| 23:20433699:G:T | 2.73E-08 | 0.006 | 1:97569456:C:T | 2.82E-07 | 0.027 |
| 23:19315297:A:G | 2.77E-08 | 0.006 | 1:97570894:T:TA | 2.82E-07 | 0.027 |
| 23:15809858:C:T | 3.45E-08 | 0.007 | 1:97571904:C:T | 2.82E-07 | 0.027 |
| 23:20152368:A:G | 3.45E-08 | 0.007 | 1:97572230:GAGT:G | 2.82E-07 | 0.027 |
| 23:20153275:T:G | 3.67E-08 | 0.007 | 1:97572515:G:A | 2.82E-07 | 0.027 |
| 23:11909706:T:C | 3.73E-08 | 0.007 | 1:97574729:C:T | 2.82E-07 | 0.027 |
| 23:11915353:A:G | 3.73E-08 | 0.007 | 1:97577453:C:A | 2.82E-07 | 0.027 |
| 23:15815828:ATCT:A | 4.32E-08 | 0.008 | 1:97580660:T:C | 2.82E-07 | 0.027 |
| 23:15797253:CCATT:C | 4.32E-08 | 0.008 | 1:97583575:G:A | 2.82E-07 | 0.027 |
| 23:19434620:G:T | 4.40E-08 | 0.008 | 1:97588523:T:TA | 2.82E-07 | 0.027 |
| 23:19459979:A:G | 6.03E-08 | 0.009 | 1:97589017:G:C | 2.82E-07 | 0.027 |
| 23:19460313:T:C | 6.03E-08 | 0.009 | 1:97590756:T:C | 2.82E-07 | 0.027 |
| 23:12802924:AAT:A | 6.52E-08 | 0.009 | 1:97591843:G:A | 2.82E-07 | 0.027 |
| 23:22458623:A:G | 8.11E-08 | 0.009 | 1:97596917:A:AC | 2.82E-07 | 0.027 |
| 23:11910053:G:A | 8.92E-08 | 0.009 | 1:97605301:T:C | 2.82E-07 | 0.027 |
| 23:11911419:G:C | 8.92E-08 | 0.009 | 1:97611140:T:C | 2.82E-07 | 0.027 |

| | | | | | |
|---|---|---|---|---|---|
| 23:12028117:G:A | 1.02E-07 | 0.009 | 3:63939078:C:T | 3.16E-07 | 0.027 |
| 23:11994113:T:C | 1.02E-07 | 0.009 | 7:26551289:C:T | 3.52E-07 | 0.027 |
| 23:11994077:A:C | 1.02E-07 | 0.009 | 7:24357372:C:A | 3.55E-07 | 0.027 |
| 23:11994062:A:G | 1.02E-07 | 0.009 | 7:26542159:A:G | 3.67E-07 | 0.027 |
| 23:20104374:A:G | 1.12E-07 | 0.010 | 7:26542205:G:A | 3.67E-07 | 0.027 |
| 5:35577195:T:C | 1.35E-07 | 0.010 | 7:26547514:G:A | 3.67E-07 | 0.027 |
| 5:35557506:A:G | 1.35E-07 | 0.010 | 7:26556858:G:A | 3.67E-07 | 0.027 |
| 5:35536371:T:C | 1.35E-07 | 0.010 | 7:26558169:G:A | 3.67E-07 | 0.027 |
| 5:35611179:T:G | 1.35E-07 | 0.010 | 7:26558874:TA:T | 3.67E-07 | 0.027 |
| 5:35611093:G:A | 1.35E-07 | 0.010 | 7:26559884:G:A | 3.67E-07 | 0.027 |
| 5:35611099:T:A | 1.35E-07 | 0.010 | 7:16417913:C:A | 3.71E-07 | 0.027 |
| 5:35580801:T:C | 1.35E-07 | 0.010 | 7:16422344:C:T | 3.71E-07 | 0.027 |
| 5:35566542:T:C | 1.35E-07 | 0.010 | 7:16431139:A:G | 3.71E-07 | 0.027 |
| 5:35567737:A:T | 1.35E-07 | 0.010 | 7:16436490:A:G | 3.71E-07 | 0.027 |
| 5:35575061:A:T | 1.35E-07 | 0.010 | 7:16498178:G:A | 3.71E-07 | 0.027 |
| 5:35607873:T:G | 1.35E-07 | 0.010 | 23:14069222:A:G | 3.91E-07 | 0.027 |
| 5:35561733:A:G | 1.35E-07 | 0.010 | 23:14069287:C:A | 4.21E-07 | 0.027 |
| 5:35540288:TTTATATTA:T | 1.35E-07 | 0.010 | 23:14069289:C:T | 4.21E-07 | 0.027 |
| 5:35602435:T:C | 1.35E-07 | 0.010 | 1:94425704:A:T | 4.30E-07 | 0.027 |
| 5:35540298:CTATAAATATCTATTATTAATT:C | 1.35E-07 | 0.010 | 1:94428765:AGAAATGTCAAGAACAAAAAAT:A | 4.30E-07 | 0.027 |
| 5:35574845:A:G | 1.35E-07 | 0.010 | 1:94429051:G:A | 4.30E-07 | 0.027 |
| 5:35553769:G:A | 1.35E-07 | 0.010 | 1:94429241:C:A | 4.30E-07 | 0.027 |
| 5:35604945:G:A | 1.35E-07 | 0.010 | 1:94440953:G:A | 4.30E-07 | 0.027 |

| | | | | | |
|---|---|---|---|---|---|
| 5:35552041:C:T | 1.35E-07 | 0.010 | 1:94448966:C:G | 4.30E-07 | 0.027 |
| 5:35573691:A:G | 1.35E-07 | 0.010 | 1:94450945:C:T | 4.30E-07 | 0.027 |
| 5:35555045:T:A | 1.35E-07 | 0.010 | 1:94455885:T:C | 4.30E-07 | 0.027 |
| 5:35549442:G:A | 1.35E-07 | 0.010 | 7:16422991:T:TC | 5.01E-07 | 0.027 |
| 5:35598258:C:T | 1.35E-07 | 0.010 | 7:15527677:A:G | 5.17E-07 | 0.027 |
| 5:35535666:T:C | 1.35E-07 | 0.010 | 3:63938489:C:A | 5.57E-07 | 0.027 |
| 5:35607095:T:A | 1.35E-07 | 0.010 | 3:63938595:T:G | 5.57E-07 | 0.027 |
| 5:35575142:GGAGTGATGAATGAAAGGGACATATAGAGTATCTGAT:G | 1.35E-07 | 0.010 | 1:97501471:T:C | 5.59E-07 | 0.027 |
| 5:35574712:G:A | 1.35E-07 | 0.010 | 1:97501473:AGATG:A | 5.59E-07 | 0.027 |
| 5:35541149:GT:G | 1.35E-07 | 0.010 | 1:97501497:AT:A | 5.59E-07 | 0.027 |
| 5:35599284:C:T | 1.35E-07 | 0.010 | 3:63863336:A:G | 5.99E-07 | 0.027 |
| 5:35599285:A:G | 1.35E-07 | 0.010 | 3:63875144:T:C | 5.99E-07 | 0.027 |
| 5:35535017:T:C | 1.35E-07 | 0.010 | 1:97501465:C:T | 6.02E-07 | 0.027 |
| 23:11912056:T:C | 1.36E-07 | 0.010 | 1:97501466:T:C | 6.02E-07 | 0.027 |
| 23:13696234:G:A | 1.44E-07 | 0.011 | 1:97495903:G:A | 6.46E-07 | 0.027 |
| 23:20104359:T:C | 1.50E-07 | 0.011 | 1:97495906:A:G | 6.46E-07 | 0.027 |
| 23:15977704:C:T | 1.66E-07 | 0.012 | 1:97495951:G:C | 6.46E-07 | 0.027 |
| 23:15927592:T:C | 1.72E-07 | 0.012 | 1:97496029:G:A | 6.46E-07 | 0.027 |
| 5:35551340:T:C | 1.78E-07 | 0.013 | 1:97496252:T:C | 6.46E-07 | 0.027 |
| 23:12050472:C:T | 1.78E-07 | 0.013 | 1:97496291:G:GC | 6.46E-07 | 0.027 |
| 23:15959263:T:C | 1.98E-07 | 0.014 | 1:97496383:A:G | 6.46E-07 | 0.027 |
| 23:11849049:C:A | 2.22E-07 | 0.015 | 1:97496502:A:T | 6.46E-07 | 0.027 |

| | | | | | |
|---|---|---|---|---|---|
| 23:11849876: G:T | 2.22E-07 | 0.015 | 1:97496658:C :T | 6.46E-07 | 0.027 |
| 23:11836620: T:C | 2.22E-07 | 0.015 | 1:97496708:G :A | 6.46E-07 | 0.027 |
| 23:11839925: T:G | 2.22E-07 | 0.015 | 1:97496771:C A:C | 6.46E-07 | 0.027 |
| 23:11841147: A:G | 2.22E-07 | 0.015 | 1:97496836:C :G | 6.46E-07 | 0.027 |
| 23:11861439: G:A | 2.22E-07 | 0.015 | 1:97496844:T :C | 6.46E-07 | 0.027 |
| 23:11832059: G:A | 2.22E-07 | 0.015 | 1:97496904:G :A | 6.46E-07 | 0.027 |
| 23:11885244: C:T | 2.22E-07 | 0.015 | 1:97496958:G :GTACTTAT C | 6.46E-07 | 0.027 |
| 23:11838911: GT:G | 2.22E-07 | 0.015 | 1:97497072:T :A | 6.46E-07 | 0.027 |
| 23:11878374: G:A | 2.22E-07 | 0.015 | 1:97497516:C :T | 6.46E-07 | 0.027 |
| 23:11843887: A:T | 2.22E-07 | 0.015 | 1:97498169:A :G | 6.46E-07 | 0.027 |
| 23:11926768: C:T | 2.22E-07 | 0.015 | 1:97498172:A :C | 6.46E-07 | 0.027 |
| 23:11944747: C:T | 2.32E-07 | 0.015 | 1:97498419:T :C | 6.46E-07 | 0.027 |
| 23:11937597: T:C | 2.32E-07 | 0.015 | 1:97499338:A :G | 6.46E-07 | 0.027 |
| 5:35554885:T :C | 2.36E-07 | 0.015 | 1:97499581:C :CA | 6.46E-07 | 0.027 |
| 23:16364597: C:T | 2.45E-07 | 0.016 | 1:97500331:C :T | 6.46E-07 | 0.027 |
| 23:15560376: T:C | 2.67E-07 | 0.017 | 1:97500354:T :C | 6.46E-07 | 0.027 |
| 23:13037989: G:A | 2.80E-07 | 0.018 | 1:97500371:C :T | 6.46E-07 | 0.027 |
| 23:13037906: G:T | 2.80E-07 | 0.018 | 1:97500443:C :T | 6.46E-07 | 0.027 |
| 23:11934117: A:AT | 3.07E-07 | 0.019 | 1:97500478:A :G | 6.46E-07 | 0.027 |
| 23:11933264: T:A | 3.07E-07 | 0.019 | 1:97500880:T :C | 6.46E-07 | 0.027 |
| 23:11940111: G:A | 3.07E-07 | 0.019 | 1:97501058:C :T | 6.46E-07 | 0.027 |
| 23:11933806: T:C | 3.07E-07 | 0.019 | 1:97501240:A :G | 6.46E-07 | 0.027 |
| 23:11941843: G:A | 3.07E-07 | 0.019 | 1:97501313:G :A | 6.46E-07 | 0.027 |

| | | | | | |
|---|---|---|---|---|---|
| 23:11940883:T:C | 3.07E-07 | 0.019 | 1:97501374:C:T | 6.46E-07 | 0.027 |
| 23:11925495:T:G | 3.42E-07 | 0.021 | 1:97501637:C:T | 6.46E-07 | 0.027 |
| 23:11925941:T:G | 3.42E-07 | 0.021 | 1:97501907:G:A | 6.46E-07 | 0.027 |
| 23:11921244:A:G | 3.42E-07 | 0.021 | 1:97501920:G:A | 6.46E-07 | 0.027 |
| 23:14872727:T:C | 3.75E-07 | 0.022 | 1:97501976:G:C | 6.46E-07 | 0.027 |
| 23:14872729:T:C | 3.75E-07 | 0.022 | 1:97501977:G:A | 6.46E-07 | 0.027 |
| 23:20198776:T:C | 3.75E-07 | 0.022 | 1:97502006:T:C | 6.46E-07 | 0.027 |
| 23:19624323:C:T | 3.77E-07 | 0.022 | 1:97502012:C:T | 6.46E-07 | 0.027 |
| 23:11890483:G:A | 4.03E-07 | 0.024 | 1:97502106:T:C | 6.46E-07 | 0.027 |
| 23:12752820:C:T | 4.09E-07 | 0.024 | 1:97502260:G:T | 6.46E-07 | 0.027 |
| 23:20145090:T:C | 4.11E-07 | 0.024 | 1:97502527:G:T | 6.46E-07 | 0.027 |
| 23:14004617:ACT:A | 4.96E-07 | 0.029 | 1:97503459:G:T | 6.46E-07 | 0.027 |
| 23:15540722:A:AC | 5.78E-07 | 0.032 | 1:97503478:G:A | 6.46E-07 | 0.027 |
| 23:15556822:G:A | 6.44E-07 | 0.032 | 1:97503678:G:A | 6.46E-07 | 0.027 |
| 23:15558455:T:C | 6.44E-07 | 0.032 | 1:97503756:C:T | 6.46E-07 | 0.027 |
| 1:108770299:G:GA | 6.45E-07 | 0.032 | 1:97503770:G:A | 6.46E-07 | 0.027 |
| 1:108806248:CTCCT:C | 6.45E-07 | 0.032 | 1:97503785:A:G | 6.46E-07 | 0.027 |
| 1:108802364:C:T | 6.45E-07 | 0.032 | 1:97503924:A:C | 6.46E-07 | 0.027 |
| 11:92308149:C:G | 6.54E-07 | 0.032 | 1:97503942:C:T | 6.46E-07 | 0.027 |
| 11:92285241:C:G | 6.54E-07 | 0.032 | 1:97503973:C:T | 6.46E-07 | 0.027 |
| 5:35831608:C:T | 6.71E-07 | 0.032 | 1:97504020:C:T | 6.46E-07 | 0.027 |
| 23:13715177:G:A | 6.87E-07 | 0.032 | 1:97504136:C:T | 6.46E-07 | 0.027 |
| 23:20075242:C:T | 7.09E-07 | 0.032 | 1:97504230:T:C | 6.46E-07 | 0.027 |
| 4:106491616:T:C | 7.42E-07 | 0.032 | 1:97504401:A:G | 6.46E-07 | 0.027 |

| | | | | | |
|---|---|---|---|---|---|
| 4:106489634: T:C | 7.42E-07 | 0.032 | 1:97504419:G :T | 6.46E-07 | 0.027 |
| 4:106490665: T:C | 7.42E-07 | 0.032 | 1:97504464:G :A | 6.46E-07 | 0.027 |
| 4:106495942: A:G | 7.42E-07 | 0.032 | 1:97504465:G :A | 6.46E-07 | 0.027 |
| 4:106490668: T:C | 7.42E-07 | 0.032 | 1:97504529:A :G | 6.46E-07 | 0.027 |
| 4:106495945: G:C | 7.42E-07 | 0.032 | 1:97505068:G :C | 6.46E-07 | 0.027 |
| 4:106492912: C:T | 7.42E-07 | 0.032 | 1:97505101:C :T | 6.46E-07 | 0.027 |
| 4:106493015: G:A | 7.42E-07 | 0.032 | 1:97505176:C :G | 6.46E-07 | 0.027 |
| 4:106494874: G:A | 7.42E-07 | 0.032 | 1:97505271:T :G | 6.46E-07 | 0.027 |
| 4:106492900: G:C | 7.42E-07 | 0.032 | 1:97505406:T :TG | 6.46E-07 | 0.027 |
| 4:106489307: C:T | 7.42E-07 | 0.032 | 1:97505587:C :T | 6.46E-07 | 0.027 |
| 4:106492525: C:A | 7.42E-07 | 0.032 | 1:97505698:G :A | 6.46E-07 | 0.027 |
| 4:106491704: A:G | 7.42E-07 | 0.032 | 1:97505762:T :G | 6.46E-07 | 0.027 |
| 4:106488210: G:A | 7.42E-07 | 0.032 | 4:112133668: G:A | 6.56E-07 | 0.027 |
| 4:106494388: G:A | 7.42E-07 | 0.032 | 4:112134309: C:T | 6.56E-07 | 0.027 |
| 4:106494673: C:G | 7.42E-07 | 0.032 | 7:19324245:A :AT | 6.64E-07 | 0.027 |
| 4:106494666: C:T | 7.42E-07 | 0.032 | 7:19324274:A :G | 6.64E-07 | 0.027 |
| 4:106494909: A:G | 7.42E-07 | 0.032 | 7:19324281:C :T | 6.64E-07 | 0.027 |
| 4:106491150: T:C | 7.42E-07 | 0.032 | 7:19324612:G :A | 6.64E-07 | 0.027 |
| 4:106494975: G:C | 7.42E-07 | 0.032 | 7:19324741:T TG:T | 6.64E-07 | 0.027 |
| 4:106490005: G:A | 7.42E-07 | 0.032 | 7:19324791:G :A | 6.64E-07 | 0.027 |
| 4:106494363: A:G | 7.42E-07 | 0.032 | 7:19326416:A :G | 6.64E-07 | 0.027 |
| 4:106490808: T:C | 7.42E-07 | 0.032 | 7:19326431:A :C | 6.64E-07 | 0.027 |
| 4:106492954: C:T | 7.42E-07 | 0.032 | 7:19326784:T :C | 6.64E-07 | 0.027 |
| 4:106493256: T:A | 7.42E-07 | 0.032 | 7:19326822:A :G | 6.64E-07 | 0.027 |

| | | | | | |
|---|---|---|---|---|---|
| 4:106493506: T:C | 7.42E-07 | 0.032 | 7:19326856:G :A | 6.64E-07 | 0.027 |
| 4:106490699: T:C | 7.42E-07 | 0.032 | 7:19327198:C :T | 6.64E-07 | 0.027 |
| 4:106490386: C:T | 7.42E-07 | 0.032 | 7:19327325:G :T | 6.64E-07 | 0.027 |
| 4:106494216: T:TAGG | 7.42E-07 | 0.032 | 7:19327869:T :G | 6.64E-07 | 0.027 |
| 4:106491830: G:T | 7.42E-07 | 0.032 | 7:19328033:C :G | 6.64E-07 | 0.027 |
| 4:106491831: A:T | 7.42E-07 | 0.032 | 7:19328077:C :T | 6.64E-07 | 0.027 |
| 4:106493195: A:G | 7.42E-07 | 0.032 | 7:19328479:C :T | 6.64E-07 | 0.027 |
| 4:106493198: T:C | 7.42E-07 | 0.032 | 7:19329014:G :A | 6.64E-07 | 0.027 |
| 4:106495683: C:CA | 7.42E-07 | 0.032 | 7:19329335:G :A | 6.64E-07 | 0.027 |
| 4:106495685: G:A | 7.42E-07 | 0.032 | 7:19329437:C :T | 6.64E-07 | 0.027 |
| 4:106494032: T:G | 7.42E-07 | 0.032 | 7:19329848:A :G | 6.64E-07 | 0.027 |
| 4:106490857: G:A | 7.42E-07 | 0.032 | 7:19330001:G :GTA | 6.64E-07 | 0.027 |
| 4:106490989: G:A | 7.42E-07 | 0.032 | 7:19330166:T :C | 6.64E-07 | 0.027 |
| 4:106491030: C:T | 7.42E-07 | 0.032 | 7:19330237:G :GTTGCCAT ATA | 6.64E-07 | 0.027 |
| 4:106492419: A:G | 7.42E-07 | 0.032 | 7:19330289:A :G | 6.64E-07 | 0.027 |
| 4:106492020: C:A | 7.42E-07 | 0.032 | 7:19331201:C :T | 6.64E-07 | 0.027 |
| 4:106490751: C:T | 7.42E-07 | 0.032 | 7:19331741:A :G | 6.64E-07 | 0.027 |
| 4:106493998: T:C | 7.42E-07 | 0.032 | 7:19331812:C :T | 6.64E-07 | 0.027 |
| 4:106492472: T:C | 7.42E-07 | 0.032 | 7:19331887:C :T | 6.64E-07 | 0.027 |
| 4:106492004: A:C | 7.42E-07 | 0.032 | 7:19332522:C :T | 6.64E-07 | 0.027 |
| 4:106490543: C:A | 7.42E-07 | 0.032 | 7:19332735:C :T | 6.64E-07 | 0.027 |
| 4:106492080: C:T | 7.42E-07 | 0.032 | 7:19333828:T :C | 6.64E-07 | 0.027 |
| 4:106492081: A:G | 7.42E-07 | 0.032 | 7:19334076:G :A | 6.64E-07 | 0.027 |

| | | | | | |
|---|---|---|---|---|---|
| 4:106491950:G:A | 7.42E-07 | 0.032 | 7:19334096:C:T | 6.64E-07 | 0.027 |
| 4:106493826:C:G | 7.42E-07 | 0.032 | 7:19336259:G:A | 6.64E-07 | 0.027 |
| 4:106493610:T:C | 7.42E-07 | 0.032 | 7:19336580:T:C | 6.64E-07 | 0.027 |
| 4:106495555:G:A | 7.42E-07 | 0.032 | 7:19336582:A:C | 6.64E-07 | 0.027 |
| 4:106493414:C:T | 7.42E-07 | 0.032 | 7:19337479:G:A | 6.64E-07 | 0.027 |
| 4:106485196:G:A | 7.42E-07 | 0.032 | 7:19337694:C:A | 6.64E-07 | 0.027 |
| 4:106494407:T:A | 7.42E-07 | 0.032 | 7:19339609:C:T | 6.64E-07 | 0.027 |
| 4:106498935:A:G | 7.42E-07 | 0.032 | 7:19340072:G:A | 6.64E-07 | 0.027 |
| 4:106492268:C:CCTTCTCT | 7.42E-07 | 0.032 | 7:19341386:C:T | 6.64E-07 | 0.027 |
| 4:106492270:A:ATATTGTGTCTCGAATAT | 7.42E-07 | 0.032 | 7:19342517:G:C | 6.64E-07 | 0.027 |
| 4:106493771:G:A | 7.42E-07 | 0.032 | 7:19342886:G:T | 6.64E-07 | 0.027 |
| 4:106493701:G:A | 7.42E-07 | 0.032 | 7:19342946:A:G | 6.64E-07 | 0.027 |
| 4:106493742:T:C | 7.42E-07 | 0.032 | 7:19343185:T:C | 6.64E-07 | 0.027 |
| 4:106493745:G:A | 7.42E-07 | 0.032 | 7:19343303:G:A | 6.64E-07 | 0.027 |
| 4:106493735:T:C | 7.42E-07 | 0.032 | 7:19343614:G:C | 6.64E-07 | 0.027 |
| 5:35812520:A:T | 7.78E-07 | 0.032 | 7:19343962:G:A | 6.64E-07 | 0.027 |
| 1:108732051:T:C | 7.80E-07 | 0.032 | 7:19344471:G:A | 6.64E-07 | 0.027 |
| 1:108787775:C:A | 7.80E-07 | 0.032 | 7:19344572:T:G | 6.64E-07 | 0.027 |
| 1:108769027:C:T | 7.80E-07 | 0.032 | 7:19344720:G:T | 6.64E-07 | 0.027 |
| 1:108777466:A:T | 7.80E-07 | 0.032 | 7:19345404:G:A | 6.64E-07 | 0.027 |
| 1:108777463:A:G | 7.80E-07 | 0.032 | 7:19347546:G:GGC | 6.64E-07 | 0.027 |
| 1:108778829:G:A | 7.80E-07 | 0.032 | 7:19351928:C:T | 6.64E-07 | 0.027 |
| 1:108777648:G:A | 7.80E-07 | 0.032 | 7:19353673:A:G | 6.64E-07 | 0.027 |

| | | | | | |
|---|---|---|---|---|---|
| 1:108777419:T:C | 7.80E-07 | 0.032 | 7:19354601:G:A | 6.64E-07 | 0.027 |
| 1:108737906:G:A | 7.80E-07 | 0.032 | 7:19354903:G:A | 6.64E-07 | 0.027 |
| 1:108737907:T:G | 7.80E-07 | 0.032 | 7:19364489:C:T | 6.64E-07 | 0.027 |
| 1:108782055:G:T | 7.80E-07 | 0.032 | 7:19365682:T:C | 6.64E-07 | 0.027 |
| 1:108729537:CATTTCT:C | 7.80E-07 | 0.032 | 7:19367158:C:T | 6.64E-07 | 0.027 |
| 1:108729872:T:C | 7.80E-07 | 0.032 | 7:19369437:T:C | 6.64E-07 | 0.027 |
| 1:108777612:C:T | 7.80E-07 | 0.032 | 7:19372959:T:G | 6.64E-07 | 0.027 |
| 23:11899044:A:C | 7.87E-07 | 0.032 | 7:19381078:C:A | 6.64E-07 | 0.027 |
| 23:14005079:C:T | 7.94E-07 | 0.032 | 7:19395796:G:A | 6.64E-07 | 0.027 |
| 23:14004771:C:T | 7.94E-07 | 0.032 | 7:19397752:G:A | 6.64E-07 | 0.027 |
| 24:46228397:G:A | 8.60E-07 | 0.034 | 7:19398965:G:C | 6.64E-07 | 0.027 |
| 23:15538980:G:C | 8.79E-07 | 0.034 | 7:19399807:C:T | 6.64E-07 | 0.027 |
| 23:15541112:G:T | 8.79E-07 | 0.034 | 7:19401651:G:T | 6.64E-07 | 0.027 |
| 23:15542994:A:AC | 8.79E-07 | 0.034 | 7:19405576:C:T | 6.64E-07 | 0.027 |
| 23:15545441:C:G | 8.79E-07 | 0.034 | 7:19405630:C:G | 6.64E-07 | 0.027 |
| 4:108688427:A:T | 9.00E-07 | 0.034 | 7:19405902:G:A | 6.64E-07 | 0.027 |
| 4:108692431:T:C | 9.00E-07 | 0.034 | 7:19406587:CTG:C | 6.64E-07 | 0.027 |
| 4:108693865:C:A | 9.00E-07 | 0.034 | 7:19407990:C:T | 6.64E-07 | 0.027 |
| 4:108683737:A:G | 9.00E-07 | 0.034 | 7:19409677:G:A | 6.64E-07 | 0.027 |
| 4:108689395:C:T | 9.00E-07 | 0.034 | 7:19410284:C:T | 6.64E-07 | 0.027 |
| 4:108688422:AT:A | 9.00E-07 | 0.034 | 7:19410661:G:A | 6.64E-07 | 0.027 |
| 4:108697752:CTA:C | 9.00E-07 | 0.034 | 7:19411295:C:T | 6.64E-07 | 0.027 |
| 4:108702703:TAA:T | 9.00E-07 | 0.034 | 7:19416230:G:A | 6.64E-07 | 0.027 |
| 4:108719627:C:T | 9.00E-07 | 0.034 | 7:19417182:G:GC | 6.64E-07 | 0.027 |

| | | | | | |
|---|---|---|---|---|---|
| 23:11060183: G:T | 9.44E-07 | 0.034 | 7:19420001:T :C | 6.64E-07 | 0.027 |
| 23:11070369: C:G | 9.44E-07 | 0.034 | 7:19420660:C :A | 6.64E-07 | 0.027 |
| 23:11071398: T:C | 9.44E-07 | 0.034 | 7:19421717:T :C | 6.64E-07 | 0.027 |
| 23:11060398: G:A | 9.44E-07 | 0.034 | 7:19422051:G :T | 6.64E-07 | 0.027 |
| 23:11050768: C:T | 9.44E-07 | 0.034 | 7:19423997:A :G | 6.64E-07 | 0.027 |
| 23:11050806: C:G | 9.44E-07 | 0.034 | 7:19424643:G :A | 6.64E-07 | 0.027 |
| 23:11074092: G:A | 9.44E-07 | 0.034 | 7:19425892:C :T | 6.64E-07 | 0.027 |
| 23:11035257: G:A | 9.44E-07 | 0.034 | 7:19428960:A :T | 6.64E-07 | 0.027 |
| 23:11032600: G:C | 9.44E-07 | 0.034 | 7:19429093:T :C | 6.64E-07 | 0.027 |
| 23:11024569: T:C | 9.44E-07 | 0.034 | 7:19433418:C :T | 6.64E-07 | 0.027 |
| 23:13305703: G:A | 9.54E-07 | 0.034 | 7:19434645:G :A | 6.64E-07 | 0.027 |
| 23:13305314: A:C | 9.54E-07 | 0.034 | 7:19436462:G :A | 6.64E-07 | 0.027 |
| 23:13153219: A:AC | 9.58E-07 | 0.034 | 7:19436583:G :A | 6.64E-07 | 0.027 |
| 23:13399547: C:T | 9.77E-07 | 0.035 | 7:19438257:C :T | 6.64E-07 | 0.027 |
| 23:19240264: A:T | 9.83E-07 | 0.035 | 7:19438475:A :G | 6.64E-07 | 0.027 |
| 23:14004062: A:G | 9.94E-07 | 0.035 | 7:19438634:A :G | 6.64E-07 | 0.027 |
| 23:14003236: A:G | 9.94E-07 | 0.035 | 7:19438992:C T:C | 6.64E-07 | 0.027 |
| 23:14000146: G:A | 9.94E-07 | 0.035 | 7:19440150:T :C | 6.64E-07 | 0.027 |
| 18:509878:C: A | 1.04E-06 | 0.036 | 7:19442527:G :A | 6.64E-07 | 0.027 |
| 18:519757:A: G | 1.04E-06 | 0.036 | 7:19443761:A :G | 6.64E-07 | 0.027 |
| 18:515358:G: T | 1.04E-06 | 0.036 | 7:19445182:C :T | 6.64E-07 | 0.027 |
| 18:520477:G: A | 1.04E-06 | 0.036 | 7:19445940:C :T | 6.64E-07 | 0.027 |
| 18:521052:G: A | 1.04E-06 | 0.036 | 7:19446678:T :C | 6.64E-07 | 0.027 |
| 23:13667206: G:T | 1.06E-06 | 0.037 | 7:19448576:G :A | 6.64E-07 | 0.027 |

| | | | | | |
|---|---|---|---|---|---|
| 4:108687528: A:AC | 1.09E-06 | 0.037 | 7:19450053:T :C | 6.64E-07 | 0.027 |
| 23:12203054: C:A | 1.09E-06 | 0.037 | 7:19450823:C A:C | 6.64E-07 | 0.027 |
| 23:14074320: A:G | 1.13E-06 | 0.039 | 7:19453467:A :G | 6.64E-07 | 0.027 |
| 4:108697756: T:G | 1.17E-06 | 0.040 | 7:19455428:C :T | 6.64E-07 | 0.027 |
| 23:12079322: C:T | 1.27E-06 | 0.043 | 7:19456784:T :C | 6.64E-07 | 0.027 |
| 23:12050262: T:C | 1.27E-06 | 0.043 | 7:19458427:C :T | 6.64E-07 | 0.027 |
| 23:12086053: A:G | 1.27E-06 | 0.043 | 7:19462364:A :G | 6.64E-07 | 0.027 |
| 23:12094718: C:T | 1.27E-06 | 0.043 | 7:19463490:G :A | 6.64E-07 | 0.027 |
| 23:12039559: A:G | 1.29E-06 | 0.043 | 7:19464665:C :T | 6.64E-07 | 0.027 |
| 23:12040571: C:T | 1.29E-06 | 0.043 | 7:19465210:T :C | 6.64E-07 | 0.027 |
| 18:508316:G: A | 1.33E-06 | 0.043 | 7:19467585:C :T | 6.64E-07 | 0.027 |
| 18:511155:T: C | 1.33E-06 | 0.043 | 7:19468156:T :A | 6.64E-07 | 0.027 |
| 18:505992:C: T | 1.33E-06 | 0.043 | 7:19472520:T :G | 6.64E-07 | 0.027 |
| 18:505810:A: G | 1.33E-06 | 0.043 | 7:19475967:T :C | 6.64E-07 | 0.027 |
| 18:519104:G: A | 1.33E-06 | 0.043 | 7:19477816:C :T | 6.64E-07 | 0.027 |
| 18:507033:A: C | 1.33E-06 | 0.043 | 7:19478958:A :AG | 6.64E-07 | 0.027 |
| 18:508820:T: C | 1.33E-06 | 0.043 | 7:19481203:G :A | 6.64E-07 | 0.027 |
| 18:504524:A: C | 1.33E-06 | 0.043 | 7:19483083:C :G | 6.64E-07 | 0.027 |
| 18:528300:T: C | 1.33E-06 | 0.043 | 7:19483756:C :T | 6.64E-07 | 0.027 |
| 18:526486:C: A | 1.33E-06 | 0.043 | 7:19483757:C :A | 6.64E-07 | 0.027 |
| 18:505939:G: A | 1.33E-06 | 0.043 | 7:19484706:A :G | 6.64E-07 | 0.027 |
| 18:527220:T: C | 1.33E-06 | 0.043 | 7:19485775:G :C | 6.64E-07 | 0.027 |
| 18:527142:C: CT | 1.33E-06 | 0.043 | 7:19486522:A :G | 6.64E-07 | 0.027 |
| 18:528787:T: A | 1.33E-06 | 0.043 | 14:27485021: T:C | 7.04E-07 | 0.029 |

| | | | | | |
|---|---|---|---|---|---|
| 18:523850:C:A | 1.33E-06 | 0.043 | 23:14069397:A:G | 7.04E-07 | 0.029 |
| 23:22640856:T:G | 1.34E-06 | 0.043 | 7:16549444:T:G | 7.22E-07 | 0.029 |
| 23:12759569:C:T | 1.34E-06 | 0.043 | 3:63880114:C:T | 7.88E-07 | 0.031 |
| 23:12760939:C:T | 1.34E-06 | 0.043 | 3:63889101:C:T | 7.88E-07 | 0.031 |
| 23:12760317:C:A | 1.34E-06 | 0.043 | 3:63904596:A:T | 7.88E-07 | 0.031 |
| 23:12760324:A:C | 1.34E-06 | 0.043 | 3:63905111:A:C | 7.88E-07 | 0.031 |
| 23:19313458:G:C | 1.39E-06 | 0.044 | 3:63905427:A:T | 7.88E-07 | 0.031 |
| 23:19318225:TA:T | 1.39E-06 | 0.044 | 4:111753318:AG:A | 7.90E-07 | 0.031 |
| 23:19312988:CA:C | 1.39E-06 | 0.044 | 4:112125486:T:C | 8.09E-07 | 0.032 |
| 4:105373682:T:C | 1.41E-06 | 0.044 | 3:63876996:C:T | 8.41E-07 | 0.033 |
| 4:105373681:G:A | 1.41E-06 | 0.044 | 7:16587288:G:T | 8.53E-07 | 0.034 |
| 4:105373613:G:T | 1.41E-06 | 0.044 | 23:19446723:G:A | 9.01E-07 | 0.035 |
| 4:105373631:C:T | 1.41E-06 | 0.044 | 23:19450840:T:C | 9.01E-07 | 0.035 |
| 5:33224344:A:G | 1.46E-06 | 0.046 | 7:16789172:C:T | 9.31E-07 | 0.036 |
| 23:21194779:G:C | 1.48E-06 | 0.047 | 3:63942066:G:A | 9.36E-07 | 0.036 |
| 23:19791252:A:G | 1.53E-06 | 0.047 | 28:41922807:T:C | 9.47E-07 | 0.036 |
| 23:15565481:T:C | 1.54E-06 | 0.047 | 3:63884480:C:T | 9.53E-07 | 0.036 |
| 23:12061234:A:C | 1.54E-06 | 0.047 | 7:24555957:G:A | 9.58E-07 | 0.036 |
| 23:12055131:C:T | 1.54E-06 | 0.047 | 7:24570491:T:C | 9.58E-07 | 0.036 |
| 23:12073063:G:C | 1.54E-06 | 0.047 | 23:12802924:AAT:A | 9.61E-07 | 0.036 |
| 23:12053250:TTTGAC:T | 1.54E-06 | 0.047 | 3:63861307:G:A | 9.64E-07 | 0.036 |
| 23:12053248:C:A | 1.54E-06 | 0.047 | 3:63866855:TG:T | 9.64E-07 | 0.036 |
| 23:12053235:C:T | 1.54E-06 | 0.047 | 3:63893871:G:A | 9.65E-07 | 0.036 |
| 23:12076397:A:T | 1.54E-06 | 0.047 | 3:63900766:G:A | 9.65E-07 | 0.036 |

| | | | | | |
|---|---|---|---|---|---|
| 23:12053194: G:A | 1.54E-06 | 0.047 | 3:63902793:G :A | 9.65E-07 | 0.036 |
| 23:12051531: G:C | 1.54E-06 | 0.047 | 3:63908465:G :T | 9.65E-07 | 0.036 |
| 23:12043819: C:G | 1.54E-06 | 0.047 | 3:63912482:T :C | 9.65E-07 | 0.036 |
| 23:12074164: G:C | 1.54E-06 | 0.047 | 8:78442051:G :A | 9.89E-07 | 0.036 |
| 23:12058950: C:T | 1.54E-06 | 0.047 | 3:63929874:A :G | 1.07E-06 | 0.036 |
| 23:12083058: G:A | 1.54E-06 | 0.047 | 3:63929916:A :C | 1.07E-06 | 0.036 |
| 23:12086668: A:AT | 1.54E-06 | 0.047 | 3:63930410:T :G | 1.07E-06 | 0.036 |
| 23:12093094: G:A | 1.54E-06 | 0.047 | 3:63931422:T :G | 1.07E-06 | 0.036 |
| 23:12042167: G:A | 1.54E-06 | 0.047 | 3:63932169:C :A | 1.07E-06 | 0.036 |
| 23:12102787: C:G | 1.56E-06 | 0.047 | 23:19431532: C:T | 1.14E-06 | 0.036 |
| 23:15524380: A:T | 1.60E-06 | 0.048 | 3:63935067:T :C | 1.14E-06 | 0.036 |
| 23:15541010: A:G | 1.67E-06 | 0.048 | 3:63935070:A :G | 1.14E-06 | 0.036 |
| 23:16029663: G:A | 1.69E-06 | 0.048 | 3:63935861:T :C | 1.14E-06 | 0.036 |
| 4:106497158: A:G | 1.70E-06 | 0.048 | 3:63936844:A :G | 1.14E-06 | 0.036 |
| 4:106498917: A:G | 1.70E-06 | 0.048 | 3:63939614:T :C | 1.14E-06 | 0.036 |
| 4:106497972: G:T | 1.70E-06 | 0.048 | 3:63939687:T C:T | 1.14E-06 | 0.036 |
| 4:106497903: C:T | 1.70E-06 | 0.048 | 3:63939782:A :C | 1.14E-06 | 0.036 |
| 4:106497770: A:G | 1.70E-06 | 0.048 | 3:63939859:G :A | 1.14E-06 | 0.036 |
| 4:106497055: A:C | 1.70E-06 | 0.048 | 3:63940484:A :G | 1.14E-06 | 0.036 |
| 4:106496773: G:A | 1.70E-06 | 0.048 | 3:63940703:G :A | 1.14E-06 | 0.036 |
| 4:106497402: T:C | 1.70E-06 | 0.048 | 3:63940792:G A:G | 1.14E-06 | 0.036 |
| 4:106497572: A:G | 1.70E-06 | 0.048 | 3:63941030:A :G | 1.14E-06 | 0.036 |
| 4:106496642: T:A | 1.70E-06 | 0.048 | 3:63941100:T :C | 1.14E-06 | 0.036 |
| 4:106497641: G:A | 1.70E-06 | 0.048 | 3:63941117:A :G | 1.14E-06 | 0.036 |

| | | | | | |
|---|---|---|---|---|---|
| 4:106496630:G:T | 1.70E-06 | 0.048 | 3:63941665:C:G | 1.14E-06 | 0.036 |
| 4:106497665:C:T | 1.70E-06 | 0.048 | 3:63942238:G:T | 1.14E-06 | 0.036 |
| 4:106497679:G:A | 1.70E-06 | 0.048 | 3:63942486:T:C | 1.14E-06 | 0.036 |
| 4:106498919:G:A | 1.70E-06 | 0.048 | 3:63942499:C:G | 1.14E-06 | 0.036 |
| 4:106499047:C:CTT | 1.70E-06 | 0.048 | 3:63942729:C:T | 1.14E-06 | 0.036 |
| 4:106498072:A:G | 1.70E-06 | 0.048 | 3:63942782:C:T | 1.14E-06 | 0.036 |
| 4:106498624:T:TAA | 1.70E-06 | 0.048 | 3:63944489:G:GA | 1.14E-06 | 0.036 |
| 4:106498029:T:C | 1.70E-06 | 0.048 | 3:63944548:T:C | 1.14E-06 | 0.036 |
| 4:106498280:T:C | 1.70E-06 | 0.048 | 3:63944697:T:C | 1.14E-06 | 0.036 |
| 4:106498685:T:C | 1.70E-06 | 0.048 | 3:63944708:G:GT | 1.14E-06 | 0.036 |
| 4:106498613:A:T | 1.70E-06 | 0.048 | 3:63944711:T:TC | 1.14E-06 | 0.036 |
| 4:106498119:A:G | 1.70E-06 | 0.048 | 3:63945096:A:AT | 1.14E-06 | 0.036 |
| 4:106498806:T:A | 1.70E-06 | 0.048 | 3:63945265:T:C | 1.14E-06 | 0.036 |
| 4:106498410:T:TC | 1.70E-06 | 0.048 | 3:63945297:A:G | 1.14E-06 | 0.036 |
| 4:106498406:CT:C | 1.70E-06 | 0.048 | 3:63945310:G:A | 1.14E-06 | 0.036 |
| 23:19314420:T:C | 1.74E-06 | 0.048 | 3:63945472:G:A | 1.14E-06 | 0.036 |
| 23:19318056:ATGGCATAGC:A | 1.74E-06 | 0.048 | 3:63946157:C:T | 1.14E-06 | 0.036 |
| 23:19315964:T:C | 1.74E-06 | 0.048 | 3:63946541:G:A | 1.14E-06 | 0.036 |
| 18:531345:T:C | 1.74E-06 | 0.048 | 3:63946989:A:AT | 1.14E-06 | 0.036 |
| 18:532174:C:T | 1.74E-06 | 0.048 | 3:63947051:T:G | 1.14E-06 | 0.036 |
| 18:545817:G:A | 1.74E-06 | 0.048 | 7:23842477:G:A | 1.21E-06 | 0.036 |
| 18:536045:T:G | 1.76E-06 | 0.048 | 7:23905811:C:A | 1.21E-06 | 0.036 |
| 18:531557:G:A | 1.76E-06 | 0.048 | 7:23906204:A:G | 1.21E-06 | 0.036 |

| 18:529644:A:T | 1.76E-06 | 0.048 | 7:23915531:C:G | 1.21E-06 | 0.036 |
|---|---|---|---|---|---|
| 18:539230:A:G | 1.76E-06 | 0.048 | 7:23916791:G:A | 1.21E-06 | 0.036 |
| 18:537857:T:A | 1.76E-06 | 0.048 | 7:23922916:T:C | 1.21E-06 | 0.036 |
| 18:533146:G:C | 1.76E-06 | 0.048 | 7:23925346:T:C | 1.21E-06 | 0.036 |
| 18:534696:G:A | 1.76E-06 | 0.048 | 7:23933830:C:G | 1.21E-06 | 0.036 |
| 18:547967:C:T | 1.76E-06 | 0.048 | 7:23939559:A:ATGTT | 1.21E-06 | 0.036 |
| 4:106343439:T:C | 1.79E-06 | 0.048 | 7:23944245:TA:T | 1.21E-06 | 0.036 |
| 4:106344609:C:G | 1.79E-06 | 0.048 | 7:23945135:A:G | 1.21E-06 | 0.036 |
| 4:106345149:C:G | 1.79E-06 | 0.048 | 7:23950960:G:A | 1.21E-06 | 0.036 |
| 4:106343551:G:C | 1.79E-06 | 0.048 | 7:23955404:A:G | 1.21E-06 | 0.036 |
| 4:106344750:A:G | 1.79E-06 | 0.048 | 7:23961782:A:G | 1.21E-06 | 0.036 |
| 4:106344964:C:T | 1.79E-06 | 0.048 | 7:23971123:C:T | 1.21E-06 | 0.036 |
| 4:106342153:T:C | 1.79E-06 | 0.048 | 7:23972934:G:A | 1.21E-06 | 0.036 |
| 4:106343830:G:A | 1.79E-06 | 0.048 | 7:23998736:G:A | 1.21E-06 | 0.036 |
| | | | 7:23999174:C:T | 1.21E-06 | 0.036 |
| | | | 7:23999744:C:A | 1.21E-06 | 0.036 |
| | | | 7:24000251:C:T | 1.21E-06 | 0.036 |
| | | | 7:24001468:A:G | 1.21E-06 | 0.036 |
| | | | 7:24003268:T:TA | 1.21E-06 | 0.036 |
| | | | 7:24005095:A:T | 1.21E-06 | 0.036 |
| | | | 7:24005247:T:A | 1.21E-06 | 0.036 |
| | | | 7:24007182:T:A | 1.21E-06 | 0.036 |
| | | | 7:24010014:G:T | 1.21E-06 | 0.036 |
| | | | 7:24018715:T:C | 1.21E-06 | 0.036 |

| | | | 7:24018916:T:C | 1.21E-06 | 0.036 |
|---|---|---|---|---|---|
| | | | 7:24019155:A:G | 1.21E-06 | 0.036 |
| | | | 7:24021667:A:G | 1.21E-06 | 0.036 |
| | | | 7:24024675:T:A | 1.21E-06 | 0.036 |
| | | | 7:24085077:T:G | 1.21E-06 | 0.036 |
| | | | 7:24185342:T:C | 1.21E-06 | 0.036 |
| | | | 7:24229880:G:T | 1.21E-06 | 0.036 |
| | | | 7:24242121:C:G | 1.21E-06 | 0.036 |
| | | | 7:24250975:C:T | 1.21E-06 | 0.036 |
| | | | 7:24263769:T:C | 1.21E-06 | 0.036 |
| | | | 7:24263770:G:T | 1.21E-06 | 0.036 |
| | | | 7:24265940:G:T | 1.21E-06 | 0.036 |
| | | | 7:24268338:A:G | 1.21E-06 | 0.036 |
| | | | 7:24269014:A:T | 1.21E-06 | 0.036 |
| | | | 7:24274231:C:T | 1.21E-06 | 0.036 |
| | | | 7:24274613:T:C | 1.21E-06 | 0.036 |
| | | | 7:24278343:A:AG | 1.21E-06 | 0.036 |
| | | | 7:24279856:A:G | 1.21E-06 | 0.036 |
| | | | 7:24281553:G:A | 1.21E-06 | 0.036 |
| | | | 7:25235052:T:G | 1.21E-06 | 0.036 |
| | | | 7:25241306:C:G | 1.21E-06 | 0.036 |
| | | | 7:25243475:G:A | 1.21E-06 | 0.036 |
| | | | 23:14068222:T:C | 1.24E-06 | 0.037 |
| | | | 3:79313378:A:C | 1.33E-06 | 0.039 |

| | | | | | |
|---|---|---|---|---|---|
| | | | 7:26563195:C:T | 1.37E-06 | 0.040 |
| | | | 7:26564231:C:T | 1.37E-06 | 0.040 |
| | | | 7:26567883:C:T | 1.37E-06 | 0.040 |
| | | | 7:26571930:C:A | 1.37E-06 | 0.040 |
| | | | 7:24266165:T:G | 1.40E-06 | 0.041 |
| | | | 3:63884650:C:T | 1.43E-06 | 0.041 |
| | | | 3:63886167:T:C | 1.43E-06 | 0.041 |
| | | | 3:63887594:A:G | 1.43E-06 | 0.041 |
| | | | 3:63887638:C:T | 1.43E-06 | 0.041 |
| | | | 7:24435625:A:T | 1.43E-06 | 0.041 |
| | | | 7:24389043:ACT:A | 1.46E-06 | 0.042 |
| | | | 7:24671714:C:G | 1.48E-06 | 0.042 |
| | | | 7:24524541:G:A | 1.61E-06 | 0.046 |
| | | | 7:24582199:C:T | 1.61E-06 | 0.046 |
| | | | 14:27504524:A:G | 1.63E-06 | 0.046 |
| | | | 3:63859869:C:G | 1.69E-06 | 0.047 |
| | | | 3:63860435:T:C | 1.69E-06 | 0.047 |
| | | | 23:19326305:C:T | 1.70E-06 | 0.047 |
| | | | 7:15545678:C:A | 1.86E-06 | 0.047 |
| | | | 7:15547932:A:G | 1.86E-06 | 0.047 |
| | | | 13:67162123:C:G | 1.88E-06 | 0.047 |
| | | | 4:111789791:T:C | 1.94E-06 | 0.047 |
| | | | 23:7189921:A:G | 1.95E-06 | 0.047 |
| | | | 23:7190185:AT:A | 1.95E-06 | 0.047 |

| | | | | | |
|---|---|---|---|---|---|
| | | | 23:7190440:GTA:G | 1.95E-06 | 0.047 |
| | | | 27:35373646:CA:C | 1.97E-06 | 0.047 |
| | | | 13:69094815:T:TAC | 2.02E-06 | 0.047 |
| | | | 7:16845824:A:G | 2.08E-06 | 0.047 |
| | | | 13:67341841:C:T | 2.11E-06 | 0.047 |
| | | | 1:97495707:C:T | 2.11E-06 | 0.047 |
| | | | 1:97495713:T:C | 2.11E-06 | 0.047 |
| | | | 1:97495725:G:T | 2.11E-06 | 0.047 |
| | | | 1:97498573:C:A | 2.11E-06 | 0.047 |
| | | | 1:97537099:A:C | 2.19E-06 | 0.047 |
| | | | 1:97555931:G:A | 2.19E-06 | 0.047 |
| | | | 10:27649735:C:T | 2.27E-06 | 0.047 |
| | | | 10:27650567:G:A | 2.27E-06 | 0.047 |
| | | | 10:27652300:G:A | 2.27E-06 | 0.047 |
| | | | 10:27656756:G:A | 2.27E-06 | 0.047 |
| | | | 10:27656837:T:C | 2.27E-06 | 0.047 |
| | | | 10:27657143:G:A | 2.27E-06 | 0.047 |
| | | | 1:95986222:A:T | 2.28E-06 | 0.047 |
| | | | 13:25611735:G:A | 2.31E-06 | 0.047 |
| | | | 23:11887787:T:C | 2.34E-06 | 0.047 |
| | | | 23:11892301:G:A | 2.34E-06 | 0.047 |
| | | | 23:11893154:T:C | 2.34E-06 | 0.047 |
| | | | 23:11900592:A:G | 2.34E-06 | 0.047 |
| | | | 3:79419871:G:A | 2.35E-06 | 0.047 |

| | | | 3:79422925:T :C | 2.35E-06 | 0.047 |
|---|---|---|---|---|---|
| | | | 3:79423885:T :C | 2.35E-06 | 0.047 |
| | | | 3:79430156:C :T | 2.35E-06 | 0.047 |
| | | | 3:79432477:A :T | 2.35E-06 | 0.047 |
| | | | 3:79441821:G :A | 2.35E-06 | 0.047 |
| | | | 3:79447972:C :T | 2.35E-06 | 0.047 |
| | | | 3:79448396:C :T | 2.35E-06 | 0.047 |
| | | | 1:97513427:C :T | 2.37E-06 | 0.047 |
| | | | 1:97514818:C :T | 2.37E-06 | 0.047 |
| | | | 1:97517273:C :T | 2.37E-06 | 0.047 |
| | | | 1:97531248:G :A | 2.37E-06 | 0.047 |
| | | | 1:97538915:G :A | 2.37E-06 | 0.047 |
| | | | 1:97548126:G :C | 2.37E-06 | 0.047 |
| | | | 1:97548532:C :T | 2.37E-06 | 0.047 |
| | | | 1:97555813:T :G | 2.37E-06 | 0.047 |
| | | | 1:97561066:C :G | 2.37E-06 | 0.047 |
| | | | 1:97569774:A :G | 2.37E-06 | 0.047 |
| | | | 1:97574282:C :T | 2.37E-06 | 0.047 |
| | | | 1:97593033:G :A | 2.37E-06 | 0.047 |
| | | | 1:97594324:T :A | 2.37E-06 | 0.047 |
| | | | 23:14075569: G:A | 2.38E-06 | 0.047 |
| | | | 23:14079397: A:G | 2.38E-06 | 0.047 |
| | | | 13:67432635: A:ATG | 2.40E-06 | 0.047 |
| | | | 1:97495764:A :T | 2.45E-06 | 0.047 |

| | | | 1:97496328:T:G | 2.45E-06 | 0.047 |
|---|---|---|---|---|---|
| | | | 1:97500267:T:C | 2.45E-06 | 0.047 |
| | | | 1:97503887:T:G | 2.45E-06 | 0.047 |
| | | | 13:67362683:A:G | 2.54E-06 | 0.047 |
| | | | 3:79245055:C:T | 2.56E-06 | 0.047 |
| | | | 3:79245059:A:G | 2.56E-06 | 0.047 |
| | | | 3:79258455:G:A | 2.56E-06 | 0.047 |
| | | | 3:79264854:C:A | 2.56E-06 | 0.047 |
| | | | 3:79264933:T:C | 2.56E-06 | 0.047 |
| | | | 3:79269426:G:A | 2.56E-06 | 0.047 |
| | | | 3:79283304:T:A | 2.56E-06 | 0.047 |
| | | | 3:79294625:C:A | 2.56E-06 | 0.047 |
| | | | 3:79302642:G:A | 2.56E-06 | 0.047 |
| | | | 3:79303145:AT:A | 2.56E-06 | 0.047 |
| | | | 26:15458536:A:G | 2.58E-06 | 0.047 |
| | | | 26:15466423:A:G | 2.58E-06 | 0.047 |
| | | | 1:97557361:C:T | 2.59E-06 | 0.047 |
| | | | 1:97585465:G:C | 2.59E-06 | 0.047 |
| | | | 1:97519009:G:T | 2.61E-06 | 0.047 |
| | | | 1:97527531:C:T | 2.61E-06 | 0.047 |
| | | | 1:97529220:G:A | 2.61E-06 | 0.047 |
| | | | 1:97529666:G:T | 2.61E-06 | 0.047 |
| | | | 1:97529805:T:C | 2.61E-06 | 0.047 |
| | | | 1:97529891:CAA:C | 2.61E-06 | 0.047 |

| | | | 1:97530425:C:T | 2.61E-06 | 0.047 |
|---|---|---|---|---|---|
| | | | 1:97530797:G:GCA | 2.61E-06 | 0.047 |
| | | | 1:97531012:T:C | 2.61E-06 | 0.047 |
| | | | 1:97531316:A:AT | 2.61E-06 | 0.047 |
| | | | 1:97531347:C:T | 2.61E-06 | 0.047 |
| | | | 1:97531479:C:G | 2.61E-06 | 0.047 |
| | | | 1:97531500:GGTCA:G | 2.61E-06 | 0.047 |
| | | | 1:97532014:A:G | 2.61E-06 | 0.047 |
| | | | 1:97532335:G:A | 2.61E-06 | 0.047 |
| | | | 1:97532373:C:T | 2.61E-06 | 0.047 |
| | | | 1:97532384:T:C | 2.61E-06 | 0.047 |
| | | | 1:97532385:G:C | 2.61E-06 | 0.047 |
| | | | 1:97532568:C:T | 2.61E-06 | 0.047 |
| | | | 1:97532570:A:AT | 2.61E-06 | 0.047 |
| | | | 1:97532642:C:T | 2.61E-06 | 0.047 |
| | | | 1:97532645:T:G | 2.61E-06 | 0.047 |
| | | | 1:97532668:C:A | 2.61E-06 | 0.047 |
| | | | 1:97532817:T:C | 2.61E-06 | 0.047 |
| | | | 1:97532841:T:C | 2.61E-06 | 0.047 |
| | | | 1:97533044:G:GCA | 2.61E-06 | 0.047 |
| | | | 1:97533286:A:G | 2.61E-06 | 0.047 |
| | | | 1:97533547:A:G | 2.61E-06 | 0.047 |
| | | | 1:97533620:C:A | 2.61E-06 | 0.047 |
| | | | 1:97533641:T:C | 2.61E-06 | 0.047 |

| | | | 1:97533642:G:A | 2.61E-06 | 0.047 |
|---|---|---|---|---|---|
| | | | 1:97533728:G:A | 2.61E-06 | 0.047 |
| | | | 1:97533766:G:A | 2.61E-06 | 0.047 |
| | | | 1:97533872:A:C | 2.61E-06 | 0.047 |
| | | | 1:97533998:T:C | 2.61E-06 | 0.047 |
| | | | 1:97534139:C:T | 2.61E-06 | 0.047 |
| | | | 1:97534216:G:T | 2.61E-06 | 0.047 |
| | | | 1:97534230:A:C | 2.61E-06 | 0.047 |
| | | | 1:97534333:C:T | 2.61E-06 | 0.047 |
| | | | 1:97534384:C:T | 2.61E-06 | 0.047 |
| | | | 1:97534525:A:T | 2.61E-06 | 0.047 |
| | | | 1:97534578:A:G | 2.61E-06 | 0.047 |
| | | | 1:97534628:A:G | 2.61E-06 | 0.047 |
| | | | 1:97534646:A:G | 2.61E-06 | 0.047 |
| | | | 1:97534671:T:G | 2.61E-06 | 0.047 |
| | | | 1:97534889:GC:G | 2.61E-06 | 0.047 |
| | | | 1:97534997:C:T | 2.61E-06 | 0.047 |
| | | | 1:97535268:A:G | 2.61E-06 | 0.047 |
| | | | 1:97535360:C:T | 2.61E-06 | 0.047 |
| | | | 1:97535532:T:C | 2.61E-06 | 0.047 |
| | | | 1:97535533:A:G | 2.61E-06 | 0.047 |
| | | | 1:97535654:T:C | 2.61E-06 | 0.047 |
| | | | 1:97535684:T:A | 2.61E-06 | 0.047 |
| | | | 1:97535726:G:A | 2.61E-06 | 0.047 |

| | | | 1:97536032:G:A | 2.61E-06 | 0.047 |
|---|---|---|---|---|---|
| | | | 1:97536243:A:G | 2.61E-06 | 0.047 |
| | | | 1:97536245:A:G | 2.61E-06 | 0.047 |
| | | | 1:97536261:T:C | 2.61E-06 | 0.047 |
| | | | 1:97536636:T:TCC | 2.61E-06 | 0.047 |
| | | | 1:97536814:T:C | 2.61E-06 | 0.047 |
| | | | 1:97537005:G:A | 2.61E-06 | 0.047 |
| | | | 1:97537010:A:G | 2.61E-06 | 0.047 |
| | | | 1:97537036:G:T | 2.61E-06 | 0.047 |
| | | | 1:97537306:CAGG:C | 2.61E-06 | 0.047 |
| | | | 1:97537514:C:A | 2.61E-06 | 0.047 |
| | | | 1:97537552:T:G | 2.61E-06 | 0.047 |
| | | | 1:97537811:C:G | 2.61E-06 | 0.047 |
| | | | 1:97537879:C:T | 2.61E-06 | 0.047 |
| | | | 1:97537881:A:C | 2.61E-06 | 0.047 |
| | | | 1:97538027:G:A | 2.61E-06 | 0.047 |
| | | | 1:97538040:T:C | 2.61E-06 | 0.047 |
| | | | 1:97538103:A:G | 2.61E-06 | 0.047 |
| | | | 1:97538168:G:T | 2.61E-06 | 0.047 |
| | | | 1:97538497:G:GA | 2.61E-06 | 0.047 |
| | | | 1:97538538:C:T | 2.61E-06 | 0.047 |
| | | | 1:97538742:A:C | 2.61E-06 | 0.047 |
| | | | 1:97539074:C:T | 2.61E-06 | 0.047 |
| | | | 1:97539081:G:A | 2.61E-06 | 0.047 |

| | | | 1:97539088:G :A | 2.61E-06 | 0.047 |
|---|---|---|---|---|---|
| | | | 1:97539183:C :T | 2.61E-06 | 0.047 |
| | | | 1:97539193:G :A | 2.61E-06 | 0.047 |
| | | | 1:97539209:A :G | 2.61E-06 | 0.047 |
| | | | 1:97539368:A :G | 2.61E-06 | 0.047 |
| | | | 1:97539383:G :C | 2.61E-06 | 0.047 |
| | | | 1:97539602:G :A | 2.61E-06 | 0.047 |
| | | | 1:97540001:A G:A | 2.61E-06 | 0.047 |
| | | | 1:97540084:A :C | 2.61E-06 | 0.047 |
| | | | 1:97540956:G :A | 2.61E-06 | 0.047 |
| | | | 1:97541152:T :C | 2.61E-06 | 0.047 |
| | | | 1:97541432:C :T | 2.61E-06 | 0.047 |
| | | | 1:97541496:G :A | 2.61E-06 | 0.047 |
| | | | 1:97541722:A :G | 2.61E-06 | 0.047 |
| | | | 1:97541814:T :G | 2.61E-06 | 0.047 |
| | | | 1:97541885:G :A | 2.61E-06 | 0.047 |
| | | | 1:97541889:G :A | 2.61E-06 | 0.047 |
| | | | 1:97542028:C :T | 2.61E-06 | 0.047 |
| | | | 1:97542214:A C:A | 2.61E-06 | 0.047 |
| | | | 1:97543211:C :T | 2.61E-06 | 0.047 |
| | | | 1:97543271:G :A | 2.61E-06 | 0.047 |
| | | | 1:97543516:G :A | 2.61E-06 | 0.047 |
| | | | 1:97543591:C :T | 2.61E-06 | 0.047 |
| | | | 1:97543843:C :T | 2.61E-06 | 0.047 |

| | | | 1:97543893:G:C | 2.61E-06 | 0.047 |
|---|---|---|---|---|---|
| | | | 1:97544233:G:C | 2.61E-06 | 0.047 |
| | | | 1:97544260:T:C | 2.61E-06 | 0.047 |
| | | | 1:97545058:C:A | 2.61E-06 | 0.047 |
| | | | 1:97545166:T:C | 2.61E-06 | 0.047 |
| | | | 1:97545455:C:A | 2.61E-06 | 0.047 |
| | | | 1:97545614:A:C | 2.61E-06 | 0.047 |
| | | | 1:97545672:C:T | 2.61E-06 | 0.047 |
| | | | 1:97545687:A:G | 2.61E-06 | 0.047 |
| | | | 1:97545952:G:T | 2.61E-06 | 0.047 |
| | | | 1:97546040:G:A | 2.61E-06 | 0.047 |
| | | | 1:97546049:C:G | 2.61E-06 | 0.047 |
| | | | 1:97546255:T:G | 2.61E-06 | 0.047 |
| | | | 1:97546585:T:C | 2.61E-06 | 0.047 |
| | | | 1:97546688:GC:G | 2.61E-06 | 0.047 |
| | | | 1:97546706:C:T | 2.61E-06 | 0.047 |
| | | | 1:97546883:C:A | 2.61E-06 | 0.047 |
| | | | 1:97546928:C:A | 2.61E-06 | 0.047 |
| | | | 1:97546998:G:A | 2.61E-06 | 0.047 |
| | | | 1:97547097:C:T | 2.61E-06 | 0.047 |
| | | | 1:97547112:A:ATGGT | 2.61E-06 | 0.047 |
| | | | 1:97547116:T:TA | 2.61E-06 | 0.047 |
| | | | 1:97547120:T:TAA | 2.61E-06 | 0.047 |
| | | | 1:97547352:G:A | 2.61E-06 | 0.047 |

| | | | 1:97547361:T:TAC | 2.61E-06 | 0.047 |
|---|---|---|---|---|---|
| | | | 1:97547408:A:G | 2.61E-06 | 0.047 |
| | | | 1:97547437:C:T | 2.61E-06 | 0.047 |
| | | | 1:97547696:C:T | 2.61E-06 | 0.047 |
| | | | 1:97548484:T:C | 2.61E-06 | 0.047 |
| | | | 1:97548768:T:A | 2.61E-06 | 0.047 |
| | | | 1:97548820:A:C | 2.61E-06 | 0.047 |
| | | | 1:97548891:G:C | 2.61E-06 | 0.047 |
| | | | 1:97549416:G:A | 2.61E-06 | 0.047 |
| | | | 1:97549509:T:C | 2.61E-06 | 0.047 |
| | | | 1:97549955:T:TG | 2.61E-06 | 0.047 |
| | | | 1:97550217:T:C | 2.61E-06 | 0.047 |
| | | | 1:97550355:G:A | 2.61E-06 | 0.047 |
| | | | 1:97550414:G:T | 2.61E-06 | 0.047 |
| | | | 1:97550566:C:CA | 2.61E-06 | 0.047 |
| | | | 1:97550615:G:A | 2.61E-06 | 0.047 |
| | | | 1:97550791:C:T | 2.61E-06 | 0.047 |
| | | | 1:97550875:TG:T | 2.61E-06 | 0.047 |
| | | | 1:97551591:G:A | 2.61E-06 | 0.047 |
| | | | 1:97551633:CCTT:C | 2.61E-06 | 0.047 |
| | | | 1:97551973:G:C | 2.61E-06 | 0.047 |
| | | | 1:97552257:A:G | 2.61E-06 | 0.047 |
| | | | 1:97552607:T:C | 2.61E-06 | 0.047 |
| | | | 1:97552636:T:C | 2.61E-06 | 0.047 |

| | | | 1:97552659:T:C | 2.61E-06 | 0.047 |
|---|---|---|---|---|---|
| | | | 1:97552691:C:T | 2.61E-06 | 0.047 |
| | | | 1:97552714:T:C | 2.61E-06 | 0.047 |
| | | | 1:97552851:C:T | 2.61E-06 | 0.047 |
| | | | 1:97552990:C:G | 2.61E-06 | 0.047 |
| | | | 1:97553187:G:C | 2.61E-06 | 0.047 |
| | | | 1:97553707:C:T | 2.61E-06 | 0.047 |
| | | | 1:97553794:C:T | 2.61E-06 | 0.047 |
| | | | 1:97553963:A:G | 2.61E-06 | 0.047 |
| | | | 1:97554133:C:T | 2.61E-06 | 0.047 |
| | | | 1:97554269:C:T | 2.61E-06 | 0.047 |
| | | | 1:97555442:C:T | 2.61E-06 | 0.047 |
| | | | 1:97555517:C:T | 2.61E-06 | 0.047 |
| | | | 1:97556005:G:A | 2.61E-06 | 0.047 |
| | | | 1:97557605:T:G | 2.61E-06 | 0.047 |
| | | | 1:97557973:A:G | 2.61E-06 | 0.047 |
| | | | 1:97557975:G:A | 2.61E-06 | 0.047 |
| | | | 1:97558429:T:A | 2.61E-06 | 0.047 |
| | | | 1:97559024:G:A | 2.61E-06 | 0.047 |
| | | | 1:97559551:G:A | 2.61E-06 | 0.047 |
| | | | 1:97559626:A:G | 2.61E-06 | 0.047 |
| | | | 1:97560047:G:A | 2.61E-06 | 0.047 |
| | | | 1:97560080:T:C | 2.61E-06 | 0.047 |
| | | | 1:97560404:T:G | 2.61E-06 | 0.047 |

| | | | | | |
|---|---|---|---|---|---|
| | | | 1:97560528:T:C | 2.61E-06 | 0.047 |
| | | | 1:97560929:A:G | 2.61E-06 | 0.047 |
| | | | 1:97561885:C:A | 2.61E-06 | 0.047 |
| | | | 1:97565617:C:T | 2.61E-06 | 0.047 |
| | | | 1:97571992:C:G | 2.61E-06 | 0.047 |
| | | | 1:97584737:C:T | 2.61E-06 | 0.047 |
| | | | 1:97588267:A:C | 2.61E-06 | 0.047 |
| | | | 1:97588512:T:G | 2.61E-06 | 0.047 |
| | | | 1:97595062:A:G | 2.61E-06 | 0.047 |
| | | | 1:97595064:T:A | 2.61E-06 | 0.047 |
| | | | 1:97595313:TAC:T | 2.61E-06 | 0.047 |
| | | | 1:97596657:T:C | 2.61E-06 | 0.047 |
| | | | 1:97601138:A:G | 2.61E-06 | 0.047 |
| | | | 1:97603298:C:T | 2.61E-06 | 0.047 |
| | | | 1:97611933:C:T | 2.61E-06 | 0.047 |
| | | | 14:25943007:G:GGC | 2.73E-06 | 0.048 |
| | | | 23:15594386:CCATGGGGT:C | 2.77E-06 | 0.048 |
| | | | 7:16426118:G:A | 2.81E-06 | 0.048 |
| | | | 7:16438701:G:A | 2.81E-06 | 0.048 |
| | | | 7:16688057:C:T | 2.84E-06 | 0.048 |
| | | | 7:25635770:A:C | 2.84E-06 | 0.048 |
| | | | 7:26362357:CAG:C | 2.84E-06 | 0.048 |
| | | | 7:26362426:G:C | 2.84E-06 | 0.048 |

|  |  |  | 7:26362433:A :AG | 2.84E-06 | 0.048 |
|---|---|---|---|---|---|
|  |  |  | 7:26362445:A :G | 2.84E-06 | 0.048 |
|  |  |  | 7:26364025:G :GGGT | 2.84E-06 | 0.048 |
|  |  |  | 7:26364403:T :C | 2.84E-06 | 0.048 |
|  |  |  | 7:26364434:A :C | 2.84E-06 | 0.048 |
|  |  |  | 7:26364448:C :T | 2.84E-06 | 0.048 |
|  |  |  | 7:26366228:C :T | 2.84E-06 | 0.048 |
|  |  |  | 7:26366449:A :G | 2.84E-06 | 0.048 |
|  |  |  | 7:26366818:C :A | 2.84E-06 | 0.048 |
|  |  |  | 7:26367445:G :A | 2.84E-06 | 0.048 |
|  |  |  | 7:26368067:A :G | 2.84E-06 | 0.048 |
|  |  |  | 7:26368459:T :C | 2.84E-06 | 0.048 |
|  |  |  | 7:26368663:A :G | 2.84E-06 | 0.048 |
|  |  |  | 7:26370831:C :T | 2.84E-06 | 0.048 |
|  |  |  | 7:26371126:T :C | 2.84E-06 | 0.048 |
|  |  |  | 7:26372532:T :G | 2.84E-06 | 0.048 |
|  |  |  | 7:26372975:A :G | 2.84E-06 | 0.048 |
|  |  |  | 7:26373684:C :T | 2.84E-06 | 0.048 |
|  |  |  | 7:26375068:G :A | 2.84E-06 | 0.048 |
|  |  |  | 7:26375396:G :A | 2.84E-06 | 0.048 |
|  |  |  | 7:26376550:C :T | 2.84E-06 | 0.048 |
|  |  |  | 7:26376684:G :A | 2.84E-06 | 0.048 |
|  |  |  | 7:26377413:G :A | 2.84E-06 | 0.048 |
|  |  |  | 7:26377802:G T:G | 2.84E-06 | 0.048 |

| | | | 7:26385438:T :C | 2.84E-06 | 0.048 |
|---|---|---|---|---|---|
| | | | 7:26395215:G :A | 2.84E-06 | 0.048 |
| | | | 7:26397356:G :A | 2.84E-06 | 0.048 |
| | | | 7:26406709:T :C | 2.84E-06 | 0.048 |
| | | | 7:26418025:C :T | 2.84E-06 | 0.048 |
| | | | 7:26445344:A :G | 2.84E-06 | 0.048 |
| | | | 7:26454891:A :G | 2.84E-06 | 0.048 |
| | | | 7:26467401:C :T | 2.84E-06 | 0.048 |
| | | | 7:26467451:C :T | 2.84E-06 | 0.048 |

**Table 2.** SNPs associated to resistance to infection traits, including (A) MAP load in macrophages, which is indicative of MAP clearing efficiency, and (B) IFN-gamma production in response to avian tuberculin, which is indicative of the immune response against MAP. SNP IDs are expressed as follows: *chromosome: position: reference allele: alternative allele*, wherein the alternative allele is the allele associated with the indicated trait.

| SNPs associated to resistance to infection | | | | | |
|---|---|---|---|---|---|
| **Low MAP load in macrophages** (indicative of MAP clearing efficiency) | | | **B) High IFN-gamma production in response to avian tuberculin** (indicative of a strong immune response against MAP) | | |
| **ID** | **p value** | **FDR value** | **ID** | **p value** | **FDR value** |
| 2:30652327:A :G | 3.28E-07 | 0.007 | 1:2523909:G: A | 9.26E-06 | 0.039 |
| 18:27466718: C:T | 3.24E-07 | 0.007 | 1:6126696:C: G | 3.81E-06 | 0.028 |
| 17:51720958: A:T | 2.94E-06 | 0.033 | 1:15673373:G :A | 1.25E-06 | 0.015 |
| 18:26736017: G:C | 2.94E-06 | 0.033 | 1:15882035:T :A | 1.25E-06 | 0.015 |
| 2:18552906:A :G | 5.57E-06 | 0.041 | 1:15967487:A :C | 1.25E-06 | 0.015 |
| 21:49387921: G:A | 5.57E-06 | 0.041 | 1:16229344:G :C | 1.25E-06 | 0.015 |
| | | | 1:26283242:C TTAT:C | 3.34E-07 | 0.007 |
| | | | 1:26311956:T A:T | 9.45E-06 | 0.039 |
| | | | 1:65360935:A :G | 4.32E-06 | 0.031 |
| | | | 1:67055505:A :G | 4.19E-06 | 0.030 |
| | | | 1:67329857:C :A | 4.19E-06 | 0.030 |
| | | | 1:85498699:T :C | 9.26E-06 | 0.039 |
| | | | 1:85768079:A :T | 9.26E-06 | 0.039 |
| | | | 1:85917516:A :T | 9.26E-06 | 0.039 |
| | | | 1:86359799:G :A | 9.26E-06 | 0.039 |
| | | | 1:86527331:A :G | 9.26E-06 | 0.039 |

(continued)

| SNPs associated to resistance to infection | | | | | |
|---|---|---|---|---|---|
| **Low MAP load in macrophages** (indicative of MAP clearing efficiency) | | | **B) High IFN-gamma production in response to avian tuberculin** (indicative of a strong immune response against MAP) | | |
| ID | p value | FDR value | ID | p value | FDR value |
| | | | 1:90034825:G :A | 4.42E-07 | 0.007 |
| | | | 1:90045608:A :G | 4.33E-06 | 0.031 |
| | | | 1:90066858:C :T | 4.33E-06 | 0.031 |
| | | | 1:90148533:G :A | 4.42E-07 | 0.007 |
| | | | 1:90360896:A :T | 4.42E-07 | 0.007 |
| | | | 1:99683124:T :C | 6.42E-06 | 0.039 |
| | | | 1:139407112: T:C | 9.26E-06 | 0.039 |
| | | | 1:141648602: T:C | 8.59E-06 | 0.039 |
| | | | 1:156151216: A:T | 9.26E-06 | 0.039 |
| | | | 1:156361868: C:T | 9.26E-06 | 0.039 |
| | | | 2:4896965:G: A | 1.09E-05 | 0.044 |
| | | | 2:24172272:C :T | 9.26E-06 | 0.039 |
| | | | 2:41226927:G :T | 9.26E-06 | 0.039 |
| | | | 2:41248487:A :T | 9.26E-06 | 0.039 |
| | | | 2:41281833:A :G | 9.26E-06 | 0.039 |
| | | | 2:41452506:G :A | 9.26E-06 | 0.039 |
| | | | 2:41453339:G :C | 4.42E-07 | 0.007 |
| | | | 2:41537050:C :G | 9.26E-06 | 0.039 |
| | | | 2:58125451:C :T | 9.26E-06 | 0.039 |
| | | | 2:89876997:C :T | 4.75E-06 | 0.033 |
| | | | 2:89914712:G :T | 1.23E-05 | 0.048 |
| | | | 2:90539674:G :A | 8.79E-06 | 0.039 |
| | | | 2:90850109:G :T | 1.51E-07 | 0.007 |
| | | | 2:102459609: A:G | 5.60E-06 | 0.038 |
| | | | 2:111599011: G:T | 3.76E-08 | 0.003 |
| | | | 2:113193787: G:T | 1.24E-05 | 0.049 |
| | | | 2:117642026: G:T | 3.10E-06 | 0.023 |
| | | | 2:121229720: T:C | 9.26E-06 | 0.039 |
| | | | 3:16604497:C A:C | 1.11E-05 | 0.044 |
| | | | 3:48535952:A :G | 7.60E-06 | 0.039 |
| | | | 3:81567807:T :C | 1.02E-05 | 0.041 |
| | | | 3:88275909:T :G | 2.12E-06 | 0.019 |
| | | | 3:88432545:G :A | 5.32E-08 | 0.003 |
| | | | 3:98456923:T :C | 9.26E-06 | 0.039 |
| | | | 3:105620612: A:G | 9.26E-06 | 0.039 |
| | | | 3:105628698: CA:C | 9.26E-06 | 0.039 |

(continued)

| SNPs associated to resistance to infection | | | | | |
|---|---|---|---|---|---|
| **Low MAP load in macrophages** (indicative of MAP clearing efficiency) | | | **B) High IFN-gamma production in response to avian tuberculin** (indicative of a strong immune response against MAP) | | |
| ID | p value | FDR value | ID | p value | FDR value |
|  |  |  | 4:22040768:A :G | 4.66E-06 | 0.033 |
|  |  |  | 4:31901616:G :A | 5.32E-08 | 0.003 |
|  |  |  | 4:49387332:A :T | 9.26E-06 | 0.039 |
|  |  |  | 4:53215664:C :T | 3.05E-06 | 0.023 |
|  |  |  | 4:53759320:G :C | 9.26E-06 | 0.039 |
|  |  |  | 4:104488949: C:T | 9.26E-06 | 0.039 |
|  |  |  | 4:111911267: G:A | 9.26E-06 | 0.039 |
|  |  |  | 4:111988447: C:A | 9.26E-06 | 0.039 |
|  |  |  | 4:115751543: G:A | 9.26E-06 | 0.039 |
|  |  |  | 5:2693061:G: C | 8.03E-06 | 0.039 |
|  |  |  | 5:6479736:T: G | 5.04E-06 | 0.035 |
|  |  |  | 5:41034230:C TTTGTG:C | 9.26E-06 | 0.039 |
|  |  |  | 5:61688429:T :C | 7.95E-06 | 0.039 |
|  |  |  | 5:85092012:G :C | 1.19E-05 | 0.047 |
|  |  |  | 5:97894629:T :A | 1.19E-05 | 0.047 |
|  |  |  | 5:111338979: G:C | 9.26E-06 | 0.039 |
|  |  |  | 5:115223532: T:G | 3.41E-06 | 0.025 |
|  |  |  | 6:4082302:A: G | 1.22E-05 | 0.048 |
|  |  |  | 6:40844607:C :T | 9.26E-06 | 0.039 |
|  |  |  | 6:49987842:G :A | 2.13E-06 | 0.019 |
|  |  |  | 6:100230212: T:G | 9.26E-06 | 0.039 |
|  |  |  | 7:512191:C:A | 1.36E-08 | 0.002 |
|  |  |  | 7:529224:G:A | 1.36E-08 | 0.002 |
|  |  |  | 7:530585:C:A | 1.36E-08 | 0.002 |
|  |  |  | 7:824684:G:A | 1.36E-08 | 0.002 |
|  |  |  | 7:941753:A:G | 1.36E-08 | 0.002 |
|  |  |  | 7:990108:A:T | 1.96E-06 | 0.018 |
|  |  |  | 7:1039149:T: A | 2.81E-06 | 0.021 |
|  |  |  | 7:1102759:G: A | 1.00E-06 | 0.014 |
|  |  |  | 7:1119144:C: T | 1.00E-06 | 0.014 |
|  |  |  | 7:1235068:C: CCACAGTT CAAAAGCA TCAATT | 4.37E-06 | 0.031 |
|  |  |  | 7:2173808:C: T | 9.35E-07 | 0.013 |
|  |  |  | 7:16113384:G :C | 1.96E-06 | 0.018 |
|  |  |  | 7:35081790:G :A | 6.07E-07 | 0.009 |

(continued)

| SNPs associated to resistance to infection | | | | | |
|---|---|---|---|---|---|
| Low MAP load in macrophages (indicative of MAP clearing efficiency) | | | B) High IFN-gamma production in response to avian tuberculin (indicative of a strong immune response against MAP) | | |
| ID | p value | FDR value | ID | p value | FDR value |
| | | | 7:62242617:A :G | 2.25E-06 | 0.020 |
| | | | 7:73286588:T :G | 9.26E-06 | 0.039 |
| | | | 8:11429376:C :T | 9.26E-06 | 0.039 |
| | | | 8:25421959:C :A | 1.19E-05 | 0.047 |
| | | | 8:27617274:T :TG | 9.26E-06 | 0.039 |
| | | | 8:60790644:G :A | 2.58E-06 | 0.020 |
| | | | 8:60877546:A :G | 9.76E-06 | 0.039 |
| | | | 8:66380459:C :T | 9.26E-06 | 0.039 |
| | | | 8:105610747: CATT:C | 9.26E-06 | 0.039 |
| | | | 9:9107205:A: C | 5.32E-08 | 0.003 |
| | | | 9:42469434:T :C | 5.32E-08 | 0.003 |
| | | | 9:64629386:T :C | 9.26E-06 | 0.039 |
| | | | 10:27643294: C:T | 1.13E-08 | 0.002 |
| | | | 10:27657626: A:G | 1.43E-06 | 0.015 |
| | | | 10:37469311: T:A | 3.07E-09 | 0.001 |
| | | | 10:37721448: T:G | 5.22E-06 | 0.036 |
| | | | 10:37770447: G:T | 1.17E-06 | 0.015 |
| | | | 10:37774809: C:T | 4.42E-07 | 0.007 |
| | | | 10:37833767: A:G | 1.94E-07 | 0.007 |
| | | | 10:41108685: C:T | 2.18E-07 | 0.007 |
| | | | 10:43278083: G:A | 8.32E-06 | 0.039 |
| | | | 10:44984198: A:G | 9.26E-06 | 0.039 |
| | | | 10:52041867: G:T | 1.04E-05 | 0.042 |
| | | | 10:54450209: A:C | 2.27E-09 | 0.001 |
| | | | 10:54461425: A:AC | 2.27E-09 | 0.001 |
| | | | 10:54702728: G:A | 1.15E-05 | 0.046 |
| | | | 10:57619635: G:C | 6.30E-06 | 0.039 |
| | | | 10:78308107: G:A | 2.18E-07 | 0.007 |
| | | | 10:79501139: T:G | 5.57E-08 | 0.003 |
| | | | 10:79542114: A:C | 5.57E-08 | 0.003 |
| | | | 10:79871701: C:T | 1.70E-07 | 0.007 |
| | | | 10:79873301: C:A | 1.70E-07 | 0.007 |
| | | | 10:79880645: G:A | 1.70E-07 | 0.007 |
| | | | 10:79902810: C:A | 1.70E-07 | 0.007 |
| | | | 10:79921123: T:G | 3.45E-06 | 0.025 |
| | | | 10:79922555: A:G | 8.97E-08 | 0.004 |

(continued)

| SNPs associated to resistance to infection | | | | | |
|---|---|---|---|---|---|
| **Low MAP load in macrophages** (indicative of MAP clearing efficiency) | | | **B) High IFN-gamma production in response to avian tuberculin** (indicative of a strong immune response against MAP) | | |
| ID | p value | FDR value | ID | p value | FDR value |
| | | | 10:79956249: A:G | 8.97E-08 | 0.004 |
| | | | 10:79957849: G:C | 3.55E-10 | 0.000 |
| | | | 10:80497047: G:A | 1.11E-05 | 0.044 |
| | | | 10:81843127: G:T | 2.70E-06 | 0.021 |
| | | | 10:90782892: C:G | 9.26E-06 | 0.039 |
| | | | 10:98658787: G:T | 4.54E-06 | 0.032 |
| | | | 10:98761003: C:T | 5.39E-07 | 0.008 |
| | | | 10:98816866: C:T | 4.54E-06 | 0.032 |
| | | | 10:98889984: T:C | 8.99E-06 | 0.039 |
| | | | 10:102172398 :T:C | 9.26E-06 | 0.039 |
| | | | 11:7139172:T :TA | 9.26E-06 | 0.039 |
| | | | 11:7148731:G :A | 6.81E-09 | 0.002 |
| | | | 11:8167092:G :A | 5.67E-06 | 0.038 |
| | | | 11:18791825: T:G | 2.70E-06 | 0.021 |
| | | | 11:23516987: TTGGC:T | 9.26E-06 | 0.039 |
| | | | 11:24677228: A:G | 9.26E-06 | 0.039 |
| | | | 11:26763300: T:C | 8.58E-10 | 0.000 |
| | | | 11:26794996: C:A | 3.45E-07 | 0.007 |
| | | | 11:26876367: G:A | 9.77E-06 | 0.039 |
| | | | 11:26883252: T:C | 9.77E-06 | 0.039 |
| | | | 11:29246062: T:C | 8.17E-06 | 0.039 |
| | | | 11:37624491: TG:T | 1.07E-05 | 0.043 |
| | | | 12:16386476: G:A | 9.62E-06 | 0.039 |
| | | | 12:19333393: T:TG | 9.26E-06 | 0.039 |
| | | | 12:77945756: G:A | 6.14E-06 | 0.039 |
| | | | 12:78134904: G:A | 3.16E-07 | 0.007 |
| | | | 12:78135232: A:G | 1.60E-06 | 0.015 |
| | | | 12:78215731: T:C | 1.23E-05 | 0.048 |
| | | | 12:78813208: A:T | 4.52E-07 | 0.007 |
| | | | 12:79310964: A:T | 9.26E-06 | 0.039 |
| | | | 13:16268793: T:C | 5.32E-08 | 0.003 |
| | | | 13:22002914: A:G | 4.66E-06 | 0.033 |
| | | | 13:55986937: A:T | 1.28E-05 | 0.050 |
| | | | 13:55992650: C:T | 1.35E-06 | 0.015 |
| | | | 13:62092010: G:A | 9.26E-06 | 0.039 |

(continued)

| SNPs associated to resistance to infection | | | | | |
| --- | --- | --- | --- | --- | --- |
| Low MAP load in macrophages (indicative of MAP clearing efficiency) | | | B) High IFN-gamma production in response to avian tuberculin (indicative of a strong immune response against MAP) | | |
| ID | p value | FDR value | ID | p value | FDR value |
|  |  |  | 13:66775257: G:T | 5.82E-06 | 0.039 |
|  |  |  | 13:67022965: C:A | 9.26E-06 | 0.039 |
|  |  |  | 14:2814304:G TGTGTGTTT GTGTACA:G | 1.52E-06 | 0.015 |
|  |  |  | 14:3109589:C :T | 9.26E-06 | 0.039 |
|  |  |  | 14:6165252:A :G | 5.67E-06 | 0.038 |
|  |  |  | 14:55418320: G:A | 9.26E-06 | 0.039 |
|  |  |  | 14:57311933: C:A | 9.26E-06 | 0.039 |
|  |  |  | 14:68015244: T:G | 5.26E-06 | 0.036 |
|  |  |  | 14:68039446: A:T | 9.26E-06 | 0.039 |
|  |  |  | 15:25831197: C:A | 9.26E-06 | 0.039 |
|  |  |  | 15:25839079: C:T | 9.26E-06 | 0.039 |
|  |  |  | 15:40526908: C:T | 8.15E-06 | 0.039 |
|  |  |  | 15:40534664: G:A | 7.47E-06 | 0.039 |
|  |  |  | 15:40972207: C:A | 2.58E-06 | 0.020 |
|  |  |  | 15:40981282: C:T | 7.90E-06 | 0.039 |
|  |  |  | 15:40993197: G:A | 1.08E-06 | 0.015 |
|  |  |  | 15:40993772: G:A | 1.05E-05 | 0.042 |
|  |  |  | 15:42346975: T:C | 2.57E-06 | 0.020 |
|  |  |  | 15:53672462: A:G | 9.26E-06 | 0.039 |
|  |  |  | 15:61264694: G:C | 9.26E-06 | 0.039 |
|  |  |  | 15:64819707: T:C | 9.26E-06 | 0.039 |
|  |  |  | 15:65654411: C:T | 9.20E-08 | 0.005 |
|  |  |  | 15:66050831: C:T | 7.33E-07 | 0.011 |
|  |  |  | 16:10484473: G:A | 2.00E-06 | 0.018 |
|  |  |  | 16:19909205: A:G | 1.24E-05 | 0.048 |
|  |  |  | 16:43836240: GCAGATTC CATCCCTA GT:G | 9.55E-06 | 0.039 |
|  |  |  | 16:43837374: T:C | 9.55E-06 | 0.039 |
|  |  |  | 16:44888428: G:A | 4.69E-07 | 0.007 |
|  |  |  | 16:45019492: C:G | 7.68E-10 | 0.000 |
|  |  |  | 16:46077301: G:A | 3.98E-07 | 0.007 |
|  |  |  | 16:46079141: C:T | 2.77E-08 | 0.002 |
|  |  |  | 16:46136976: G:C | 1.38E-07 | 0.006 |
|  |  |  | 16:46196018: G:A | 4.09E-07 | 0.007 |

(continued)

| SNPs associated to resistance to infection | | | | | |
|---|---|---|---|---|---|
| Low MAP load in macrophages (indicative of MAP clearing efficiency) | | | B) High IFN-gamma production in response to avian tuberculin (indicative of a strong immune response against MAP) | | |
| ID | p value | FDR value | ID | p value | FDR value |
| | | | 16:46204505: T:C | 3.98E-07 | 0.007 |
| | | | 16:46485012: C:A | 8.71E-06 | 0.039 |
| | | | 16:46759147: A:G | 8.71E-06 | 0.039 |
| | | | 16:46781627: C:CG | 1.62E-06 | 0.015 |
| | | | 16:46998522: A:G | 8.71E-06 | 0.039 |
| | | | 16:47010960: C:T | 1.86E-07 | 0.007 |
| | | | 16:47047708: T:C | 1.86E-07 | 0.007 |
| | | | 16:48420908: G:A | 8.71E-06 | 0.039 |
| | | | 16:59769942: TA:T | 4.86E-06 | 0.034 |
| | | | 16:62608428: AATA | 9.26E-06 | 0.039 |
| | | | 16:68659612: T:C | 5.95E-06 | 0.039 |
| | | | 16:68771488: G:A | 9.26E-06 | 0.039 |
| | | | 16:72020440: T:C | 4.42E-07 | 0.007 |
| | | | 16:74047042: T:TAC | 7.98E-06 | 0.039 |
| | | | 16:74047630: G:A | 7.98E-06 | 0.039 |
| | | | 16:74087043: C:G | 4.22E-06 | 0.031 |
| | | | 16:74098459: C:T | 4.52E-07 | 0.007 |
| | | | 16:74104193: G:A | 5.50E-06 | 0.038 |
| | | | 16:74378706: G:T | 9.57E-06 | 0.039 |
| | | | 16:75527068: C:T | 4.44E-06 | 0.032 |
| | | | 16:75544315: G:A | 4.44E-06 | 0.032 |
| | | | 16:75549580: ATA | 4.44E-06 | 0.032 |
| | | | 17:6676788:G :A | 8.32E-06 | 0.039 |
| | | | 17:8929084:A :G | 2.27E-09 | 0.001 |
| | | | 17:42749086: C:T | 7.56E-06 | 0.039 |
| | | | 17:46352136: G:A | 1.74E-06 | 0.016 |
| | | | 17:57784713: G:A | 2.59E-06 | 0.020 |
| | | | 17:57844456: A:AG | 2.42E-06 | 0.020 |
| | | | 17:57930217: G:A | 3.13E-08 | 0.002 |
| | | | 17:58357669: C:A | 2.93E-06 | 0.022 |
| | | | 17:58357860: G:T | 3.05E-08 | 0.002 |
| | | | 17:58376306: A:G | 8.58E-07 | 0.012 |
| | | | 17:58393881: A:G | 3.05E-08 | 0.002 |
| | | | 17:58424060: T:C | 6.13E-06 | 0.039 |
| | | | 17:64242684: G:A | 9.26E-06 | 0.039 |
| | | | 17:65815619: C:T | 2.89E-06 | 0.022 |

(continued)

| SNPs associated to resistance to infection | | | | | |
|---|---|---|---|---|---|
| **Low MAP load in macrophages** (indicative of MAP clearing efficiency) | | | **B) High IFN-gamma production in response to avian tuberculin** (indicative of a strong immune response against MAP) | | |
| ID | p value | FDR value | ID | p value | FDR value |
| | | | 17:65816128: A:T | 9.83E-06 | 0.040 |
| | | | 17:65859651: C:T | 2.89E-06 | 0.022 |
| | | | 17:65982514: C:A | 2.37E-06 | 0.020 |
| | | | 17:66037966: G:A | 1.98E-06 | 0.018 |
| | | | 17:66595816: C:T | 2.73E-06 | 0.021 |
| | | | 17:69625753: T:TC | 1.23E-06 | 0.015 |
| | | | 18:12038129: C:T | 5.06E-06 | 0.035 |
| | | | 18:13153710: T:C | 3.31E-06 | 0.024 |
| | | | 18:26472119: T:G | 9.26E-06 | 0.039 |
| | | | 18:54313025: A:G | 8.79E-06 | 0.039 |
| | | | 19:3988061:G :A | 5.29E-06 | 0.036 |
| | | | 20:923897:G: A | 9.26E-06 | 0.039 |
| | | | 20:21039539: G:T | 9.26E-06 | 0.039 |
| | | | 20:35702098: G:A | 9.26E-06 | 0.039 |
| | | | 20:36327174: G:T | 1.12E-05 | 0.045 |
| | | | 20:36557296: C:G | 7.91E-07 | 0.011 |
| | | | 20:36567397: G:A | 5.03E-07 | 0.007 |
| | | | 20:37487725: C:A | 2.18E-07 | 0.007 |
| | | | 20:37691867: G:A | 9.26E-06 | 0.039 |
| | | | 20:38295510: T:C | 5.32E-08 | 0.003 |
| | | | 20:41827305: G:GAT | 9.26E-06 | 0.039 |
| | | | 20:45398272: G:A | 9.26E-06 | 0.039 |
| | | | 20:55228299: A:T | 4.57E-06 | 0.033 |
| | | | 20:58416613: T:G | 9.26E-06 | 0.039 |
| | | | 20:68236954: T:G | 1.64E-06 | 0.016 |
| | | | 21:13584472: G:A | 2.27E-09 | 0.001 |
| | | | 21:27661262: A:G | 7.55E-06 | 0.039 |
| | | | 21:29656191: C:T | 7.55E-06 | 0.039 |
| | | | 21:58429839: C:T | 1.03E-07 | 0.005 |
| | | | 21:61857487: G:A | 9.26E-06 | 0.039 |
| | | | 21:62912331: A:C | 3.02E-06 | 0.022 |
| | | | 21:62955802: T:A | 9.39E-07 | 0.013 |
| | | | 22:12625812: C:T | 6.32E-06 | 0.039 |
| | | | 22:12939505: C:T | 1.37E-07 | 0.006 |
| | | | 22:12984148: A:G | 3.85E-07 | 0.007 |
| | | | 22:13079290: C:T | 6.46E-07 | 0.009 |

(continued)

| SNPs associated to resistance to infection | | | | | |
| Low MAP load in macrophages (indicative of MAP clearing efficiency) | | | B) High IFN-gamma production in response to avian tuberculin (indicative of a strong immune response against MAP) | | |
| ID | p value | FDR value | ID | p value | FDR value |
| --- | --- | --- | --- | --- | --- |
| | | | 22:24907335: T:C | 9.26E-06 | 0.039 |
| | | | 22:28603524: T:G | 2.00E-07 | 0.007 |
| | | | 22:31380734: A:G | 9.26E-06 | 0.039 |
| | | | 22:31402760: C:A | 9.26E-06 | 0.039 |
| | | | 22:32872399: T:C | 2.86E-06 | 0.022 |
| | | | 22:33463642: T:C | 6.91E-06 | 0.039 |
| | | | 22:33464942: C:T | 5.60E-08 | 0.003 |
| | | | 22:34362907: G:A | 6.30E-06 | 0.039 |
| | | | 22:55472813: A:G | 9.26E-06 | 0.039 |
| | | | 22:56007271: GT:G | 9.26E-06 | 0.039 |
| | | | 23:17859235: G:A | 5.33E-06 | 0.036 |
| | | | 23:37433909: T:G | 9.26E-06 | 0.039 |
| | | | 24:4569168:C :CGT | 2.31E-06 | 0.020 |
| | | | 24:40641071: C:G | 1.13E-05 | 0.045 |
| | | | 24:40977031: G:T | 8.43E-06 | 0.039 |
| | | | 24:45066420: G:C | 1.60E-06 | 0.015 |
| | | | 24:58573939: T:C | 6.54E-06 | 0.039 |
| | | | 24:60984804: T:TG | 8.27E-06 | 0.039 |
| | | | 25:12366113: TTTG:T | 2.79E-06 | 0.021 |
| | | | 25:12368203: T:C | 5.32E-08 | 0.003 |
| | | | 25:12394597: G:A | 5.32E-08 | 0.003 |
| | | | 25:12394807: T:C | 5.32E-08 | 0.003 |
| | | | 26:6521619:A :G | 2.65E-06 | 0.021 |
| | | | 26:8076021:C :T | 1.36E-07 | 0.006 |
| | | | 26:8092859:T :C | 1.42E-06 | 0.015 |
| | | | 26:10915431: G:A | 1.58E-06 | 0.015 |
| | | | 26:12482172: G:A | 2.18E-07 | 0.007 |
| | | | 26:12499709: TG:T | 2.18E-07 | 0.007 |
| | | | 26:15176321: G:T | 8.37E-11 | 0.000 |
| | | | 26:17955612: C:CT | 9.26E-06 | 0.039 |
| | | | 26:17955725: GA:G | 9.26E-06 | 0.039 |
| | | | 26:36004039: A:T | 6.27E-06 | 0.039 |
| | | | 26:36772520: C:T | 4.48E-06 | 0.032 |
| | | | 26:36850374: CAG:C | 5.83E-06 | 0.039 |
| | | | 26:38850335: G:A | 1.15E-05 | 0.046 |
| | | | 26:38865206: C:T | 7.27E-06 | 0.039 |

(continued)

| SNPs associated to resistance to infection | | | | | |
|---|---|---|---|---|---|
| **Low MAP load in macrophages** (indicative of MAP clearing efficiency) | | | **B) High IFN-gamma production in response to avian tuberculin** (indicative of a strong immune response against MAP) | | |
| **ID** | **p value** | **FDR value** | **ID** | **p value** | **FDR value** |
| | | | 26:38867160: C:G | 1.61E-06 | 0.015 |
| | | | 26:38868147: C:A | 7.27E-06 | 0.039 |
| | | | 26:43807975: G:A | 4.85E-06 | 0.034 |
| | | | 27:32664141: C:T | 9.26E-06 | 0.039 |
| | | | 27:32671437: T:G | 9.26E-06 | 0.039 |
| | | | 27:32676576: G:A | 9.26E-06 | 0.039 |
| | | | 27:32685305: C:A | 9.26E-06 | 0.039 |
| | | | 27:43552273: C:G | 9.26E-06 | 0.039 |
| | | | 28:21093117: ATA | 2.13E-06 | 0.019 |
| | | | 28:38626680: C:A | 2.57E-07 | 0.007 |
| | | | 29:16060386: T:C | 4.02E-06 | 0.029 |
| | | | 29:32420890: C:T | 9.26E-06 | 0.039 |

**Table 3.** SNPs associated to tolerance, including (A) infection confirmed by tissue PCR and bacteriological culture but without lesions in gut tissues and (B) presence of multifocal, but not diffuse, lesions in gut tissues. SNP IDs are expressed as follows: *chromosome: position: reference allele: alternative allele,* wherein the alternative allele is the allele associated with the indicated trait.

| SNPs associated to tolerance | | | | | |
|---|---|---|---|---|---|
| **A) Infection confirmed by tissue PCR and bacteriological culture but without lesions in gut tissues** | | | **B) Presence of multifocal, but not diffuse, lesions in gut tissues** | | |
| **ID** | **p value** | **FDR value** | **ID** | **p value** | **FDR value** |
| 4:66714449:A :G | 5.53172E-07 | 0.045 | 11:5755689:G :A | 1.66E-11 | 3.51E-05 |
| 4:66733154:C :T | 5.53172E-07 | 0.045 | 11:5842637:C :T | 1.66E-11 | 3.51E-05 |
| 4:66765303:A :T | 2.55081E-07 | 0.044 | 11:5846146:C :A | 1.66E-11 | 3.51E-05 |
| 4:66795699:T :C | 2.55081E-07 | 0.044 | 11:5847439:G :A | 1.66E-11 | 3.51E-05 |
| 4:66796616:T :A | 2.55081E-07 | 0.044 | 11:5850492:G :A | 1.66E-11 | 3.51E-05 |
| 4:66799875:C :A | 2.55081E-07 | 0.044 | 11:5871235:A :G | 1.66E-11 | 3.51E-05 |
| 4:66808372:T :C | 2.55081E-07 | 0.044 | 22:2008232:G :A | 1.50E-10 | 0.0003 |
| 4:66808774:T :A | 2.55081E-07 | 0.044 | 22:2049268:C :A | 6.05E-10 | 0.0006 |
| 4:66808775:A :T | 2.55081E-07 | 0.044 | 11:6118872:C GA:C | 6.75E-10 | 0.0006 |
| 4:66809158:G :A | 2.55081E-07 | 0.044 | 22:2039626:C :T | 7.60E-10 | 0.0006 |
| 4:80573101:C :T | 4.02032E-07 | 0.044 | 22:3138700:C :T | 8.62E-10 | 0.0006 |
| 9:101497090: A:G | 1.59797E-07 | 0.044 | 22:3138706:T :G | 8.62E-10 | 0.0006 |
| 16:21807714: TCACACG:T | 1.57621E-07 | 0.044 | 22:3138710:C :T | 8.62E-10 | 0.0006 |
| 16:71997220: T:C | 5.69625E-07 | 0.046 | 22:3138717:C :T | 8.62E-10 | 0.0006 |

(continued)

| SNPs associated to tolerance | | | | | |
|---|---|---|---|---|---|
| A) Infection confirmed by tissue PCR and bacteriological culture but without lesions in gut tissues | | | B) Presence of multifocal, but not diffuse, lesions in gut tissues | | |
| ID | p value | FDR value | ID | p value | FDR value |
| 16:72010469: G:C | 3.61403E-07 | 0.044 | 22:3138734:C :T | 8.62E-10 | 0.0006 |
| 16:72010839: C:T | 5.69625E-07 | 0.046 | 22:3138779:A :G | 8.62E-10 | 0.0006 |
| 25:9408535:G :A | 3.93036E-07 | 0.044 | 22:3138824:G :C | 8.62E-10 | 0.0006 |
| 25:9409692:T :A | 3.93036E-07 | 0.044 | 22:3138851:T :C | 8.62E-10 | 0.0006 |
| 25:9414382:C :T | 3.93036E-07 | 0.044 | 22:2006006:C :A | 2.15E-09 | 0.0013 |
| 25:9419006:C :T | 3.93036E-07 | 0.044 | 22:2015354:T :C | 2.16E-09 | 0.0013 |
| 25:9421170:C :T | 5.34367E-07 | 0.044 | 22:2039018:T :A | 2.16E-09 | 0.0013 |
| 25:9422143:C :T | 5.34367E-07 | 0.044 | 22:2046649:T :C | 3.64E-09 | 0.0021 |
| 25:9422174:C :T | 5.34367E-07 | 0.044 | 22:2058184:T :C | 4.38E-09 | 0.0024 |
| 25:9422305:T :C | 5.34367E-07 | 0.044 | 22:2025942:G :A | 4.66E-09 | 0.0025 |
| 25:9422358:T :C | 5.34367E-07 | 0.044 | 22:2074848:C :T | 7.02E-09 | 0.0036 |
| 25:9422359:G :A | 5.34367E-07 | 0.044 | 11:6038331:C :T | 9.13E-09 | 0.0045 |
| 25:9422384:A :G | 5.34367E-07 | 0.044 | 5:25667192:G :A | 1.39E-08 | 0.0059 |
| 25:9422511:G :C | 5.34367E-07 | 0.044 | 5:25714700:C :T | 1.39E-08 | 0.0059 |
| 25:9422715:C :T | 5.34367E-07 | 0.044 | 5:25749126:G :A | 1.39E-08 | 0.0059 |
| 25:9423176:C :T | 3.93036E-07 | 0.044 | 5:25754968:C :T | 1.39E-08 | 0.0059 |
| 25:9423644:C :T | 5.34367E-07 | 0.044 | 5:27777338:C :T | 1.69E-08 | 0.0069 |
| 25:9424940:C :T | 5.34367E-07 | 0.044 | 22:3466903:A :G | 2.24E-08 | 0.0084 |
| 25:9424948:A :C | 5.34367E-07 | 0.044 | 22:3475211:T :C | 2.24E-08 | 0.0084 |
| 25:9425105:A :G | 5.34367E-07 | 0.044 | 22:3482728:C :G | 2.24E-08 | 0.0084 |
| 25:9425146:G :A | 5.34367E-07 | 0.044 | 22:4243946:C :G | 5.04E-08 | 0.0162 |
| 25:9425178:G :A | 5.34367E-07 | 0.044 | 5:27760957:A :G | 6.04E-08 | 0.0162 |
| 25:9425200:A :T | 5.34367E-07 | 0.044 | 22:2007764:A :C | 6.38E-08 | 0.0162 |
| 25:9425836:T :G | 5.34367E-07 | 0.044 | 22:1780377:A :G | 6.38E-08 | 0.0162 |
| 25:9425885:A :G | 5.34367E-07 | 0.044 | 22:1780802:G :T | 6.38E-08 | 0.0162 |
| 25:9425956:G :A | 5.34367E-07 | 0.044 | 22:1781731:C :T | 6.38E-08 | 0.0162 |
| 25:9425959:A :G | 5.34367E-07 | 0.044 | 22:1784099:C :G | 6.38E-08 | 0.0162 |
| 25:9425966:T :C | 5.34367E-07 | 0.044 | 5:25441743:C :T | 7.78E-08 | 0.0162 |
| 25:9426030:T :C | 5.34367E-07 | 0.044 | 5:25462288:C :T | 7.78E-08 | 0.0162 |
| 25:9426112:A :G | 5.34367E-07 | 0.044 | 5:25462749:C :G | 7.78E-08 | 0.0162 |
| 25:9426141:C :T | 5.34367E-07 | 0.044 | 5:25463038:A :G | 7.78E-08 | 0.0162 |
| 25:9426208:T :C | 5.34367E-07 | 0.044 | 5:25472579:A :G | 7.78E-08 | 0.0162 |
| 25:9426385:G :A | 5.34367E-07 | 0.044 | 5:25479747:A :G | 7.78E-08 | 0.0162 |
| 25:9426387:A :C | 5.34367E-07 | 0.044 | 5:25480333:T :A | 7.78E-08 | 0.0162 |
| 25:9426427:G :A | 5.34367E-07 | 0.044 | 5:25508746:G :A | 7.78E-08 | 0.0162 |

(continued)

| SNPs associated to tolerance | | | | | |
|---|---|---|---|---|---|
| A) Infection confirmed by tissue PCR and bacteriological culture but without lesions in gut tissues | | | B) Presence of multifocal, but not diffuse, lesions in gut tissues | | |
| ID | p value | FDR value | ID | p value | FDR value |
| 25:9426471:A GGAAG:A | 5.34367E-07 | 0.044 | 5:25524412:A :C | 7.78E-08 | 0.0162 |
| 25:9426546:T :C | 5.34367E-07 | 0.044 | 5:25527221:G :A | 7.78E-08 | 0.0162 |
| 25:9426879:G :A | 5.34367E-07 | 0.044 | 5:25539364:G :A | 7.78E-08 | 0.0162 |
| 25:9426981:C :T | 5.34367E-07 | 0.044 | 5:25547992:G :T | 7.78E-08 | 0.0162 |
| 25:9427078:C :T | 5.34367E-07 | 0.044 | 5:25551205:T :A | 7.78E-08 | 0.0162 |
| 25:9427103:A :T | 5.34367E-07 | 0.044 | 5:25555182:C :T | 7.78E-08 | 0.0162 |
| 25:9427131:C :A | 5.34367E-07 | 0.044 | 5:25560288:G :A | 7.78E-08 | 0.0162 |
| 25:9427564:G :C | 5.34367E-07 | 0.044 | 5:25562781:G :A | 7.78E-08 | 0.0162 |
| 25:9427614:C :A | 5.34367E-07 | 0.044 | 5:25568227:C :G | 7.78E-08 | 0.0162 |
| 25:9427777:C :G | 5.34367E-07 | 0.044 | 5:25587685:A :C | 7.78E-08 | 0.0162 |
| 25:9428669:C :T | 5.34367E-07 | 0.044 | 5:25605099:A :C | 7.78E-08 | 0.0162 |
| 25:9428713:T :C | 5.34367E-07 | 0.044 | 5:25617749:G :A | 7.78E-08 | 0.0162 |
| 25:9429103:C :T | 5.34367E-07 | 0.044 | 11:13611793A:G | 1.01E-07 | 0.0207 |
| 25:9429137:G :T | 5.34367E-07 | 0.044 | 22:2006763:T :C | 1.13E-07 | 0.0229 |
| 25:9429150:G :A | 5.34367E-07 | 0.044 | 11:5723771:A :AGCGATCC CATCGTAT TTGCTTT | 1.28E-07 | 0.0235 |
| 25:9429177:A :G | 5.34367E-07 | 0.044 | 22:3574032:C :T | 1.48E-07 | 0.0235 |
| 25:9429228:T :C | 5.34367E-07 | 0.044 | 22:3574691:T A:T | 1.48E-07 | 0.0235 |
| 25:9429281:A :T | 5.34367E-07 | 0.044 | 3:24733907:C :T | 1.50E-07 | 0.0235 |
| 25:9429371:A :G | 5.34367E-07 | 0.044 | 3:24736890:C :T | 1.50E-07 | 0.0235 |
| 25:9429507:G :A | 5.34367E-07 | 0.044 | 3:24741450:C :T | 1.50E-07 | 0.0235 |
| 25:9429770:G :T | 5.34367E-07 | 0.044 | 3:24758644:A :G | 1.50E-07 | 0.0235 |
| 25:9430151:G :A | 5.34367E-07 | 0.044 | 3:24770407:T :C | 1.50E-07 | 0.0235 |
| 25:9430176:T :C | 5.34367E-07 | 0.044 | 3:24771942:A :C | 1.50E-07 | 0.0235 |
| 25:9430183:A :G | 5.34367E-07 | 0.044 | 3:24778870:A ACAG:A | 1.50E-07 | 0.0235 |
| 25:9430392:A :G | 5.34367E-07 | 0.044 | 3:24781942:C :T | 1.50E-07 | 0.0235 |
| 25:9430467:A :G | 5.34367E-07 | 0.044 | 3:24791452:C :T | 1.50E-07 | 0.0235 |
| 25:9430631:C :T | 5.34367E-07 | 0.044 | 3:24792339:C :T | 1.50E-07 | 0.0235 |
| 25:9430694:A :C | 5.34367E-07 | 0.044 | 3:24792804:T C:T | 1.50E-07 | 0.0235 |
| 25:9430988:A :T | 5.34367E-07 | 0.044 | 3:24796775:G :C | 1.50E-07 | 0.0235 |
| 25:9431595:G :A | 5.34367E-07 | 0.044 | 3:24802202:C :A | 1.50E-07 | 0.0235 |
| 25:9431621:G :A | 5.34367E-07 | 0.044 | 3:24807389:A :G | 1.50E-07 | 0.0235 |
| 25:9431860:G :A | 5.34367E-07 | 0.044 | 3:24825344:G :C | 1.50E-07 | 0.0235 |
| 25:9431925:T :C | 5.34367E-07 | 0.044 | 22:1924672:G :A | 1.57E-07 | 0.0243 |
| 25:9431926:G :C | 5.34367E-07 | 0.044 | 18:1178266:C :T | 1.64E-07 | 0.0245 |

(continued)

| SNPs associated to tolerance | | | | | |
|---|---|---|---|---|---|
| A) Infection confirmed by tissue PCR and bacteriological culture but without lesions in gut tissues | | | B) Presence of multifocal, but not diffuse, lesions in gut tissues | | |
| ID | p value | FDR value | ID | p value | FDR value |
| 25:9432704:C :T | 3.93036E-07 | 0.044 | 23:16463421: T:A | 1.75E-07 | 0.0245 |
| 25:9435082:C :T | 5.34367E-07 | 0.044 | 22:2007879:G :C | 1.75E-07 | 0.0245 |
| 25:9435351:G :T | 5.34367E-07 | 0.044 | 11:9916584:T :A | 1.98E-07 | 0.0245 |
| 25:9440538:G :A | 5.34367E-07 | 0.044 | 11:9917737:C :T | 1.98E-07 | 0.0245 |
| 25:9444088:C :CAT | 5.34367E-07 | 0.044 | 11:9924553:G :C | 1.98E-07 | 0.0245 |
| 25:9444305:G :A | 5.34367E-07 | 0.044 | 5:39288039:C :T | 2.04E-07 | 0.0245 |
| 25:9446904:C :G | 5.34367E-07 | 0.044 | 5:39290008:C :T | 2.04E-07 | 0.0245 |
| 25:9448186:C :G | 5.34367E-07 | 0.044 | 24:37683179: G:A | 2.33E-07 | 0.0245 |
| 25:9449438:G :A | 5.34367E-07 | 0.044 | 24:37683666: G:T | 2.33E-07 | 0.0245 |
| 25:9449490:G :A | 5.34367E-07 | 0.044 | 24:37683680: C:T | 2.33E-07 | 0.0245 |
| 25:9449737:A :G | 5.34367E-07 | 0.044 | 24:37683689: T:G | 2.33E-07 | 0.0245 |
| 25:9451350:A :G | 5.34367E-07 | 0.044 | 24:37685737: C:T | 2.33E-07 | 0.0245 |
| 25:9451763:G :A | 5.34367E-07 | 0.044 | 24:37685877: C:T | 2.33E-07 | 0.0245 |
| 25:9451782:C :T | 5.34367E-07 | 0.044 | 24:37685940: G:GC | 2.33E-07 | 0.0245 |
| 25:9451797:G :C | 5.34367E-07 | 0.044 | 24:37686142: C:T | 2.33E-07 | 0.0245 |
| 25:9451799:G :A | 5.34367E-07 | 0.044 | 24:37686362: T:A | 2.33E-07 | 0.0245 |
| 25:9451819:A :G | 5.34367E-07 | 0.044 | 24:37686424: T:C | 2.33E-07 | 0.0245 |
| 25:9451905:T :A | 5.34367E-07 | 0.044 | 24:37687778: C:T | 2.33E-07 | 0.0245 |
| 25:9451907:T :C | 5.34367E-07 | 0.044 | 24:37687926: G:A | 2.33E-07 | 0.0245 |
| 25:9451938:G C:G | 5.34367E-07 | 0.044 | 24:37687951: C:CTA | 2.33E-07 | 0.0245 |
| 25:9451957:C :G | 5.34367E-07 | 0.044 | 24:37687964: C:T | 2.33E-07 | 0.0245 |
| 25:9451976:G :T | 5.34367E-07 | 0.044 | 24:37688104: T:C | 2.33E-07 | 0.0245 |
| 25:9451994:C :CCG | 5.34367E-07 | 0.044 | 24:37688379: C:T | 2.33E-07 | 0.0245 |
| 25:9451999:T CC:T | 5.34367E-07 | 0.044 | 24:37688529: A:G | 2.33E-07 | 0.0245 |
| 25:9452009:T :G | 5.34367E-07 | 0.044 | 24:37688572: T:G | 2.33E-07 | 0.0245 |
| 25:9452180:A :G | 5.34367E-07 | 0.044 | 24:37688621: T:C | 2.33E-07 | 0.0245 |
| 25:9452808:G :A | 5.34367E-07 | 0.044 | 24:37688671: G:C | 2.33E-07 | 0.0245 |
| 25:9454296:G :A | 3.93036E-07 | 0.044 | 24:37688829: C:T | 2.33E-07 | 0.0245 |
| 25:9455949:G :A | 3.93036E-07 | 0.044 | 24:37689307: T:C | 2.33E-07 | 0.0245 |
| 25:9460078:T :C | 5.34367E-07 | 0.044 | 24:37689460: A:G | 2.33E-07 | 0.0245 |
| 25:9464842:T :G | 3.93036E-07 | 0.044 | 24:37689500: T:C | 2.33E-07 | 0.0245 |
| 25:9465773:A :C | 5.34367E-07 | 0.044 | 24:37689618: T:C | 2.33E-07 | 0.0245 |
| 25:9465777:A :G | 5.34367E-07 | 0.044 | 24:37689860: T:C | 2.33E-07 | 0.0245 |
| 25:9467048:A :G | 5.34367E-07 | 0.044 | 24:37689929: C:T | 2.33E-07 | 0.0245 |
| 25:9470336:T :C | 5.34367E-07 | 0.044 | 24:37689945: G:C | 2.33E-07 | 0.0245 |

(continued)

| SNPs associated to tolerance | | | | | |
| A) Infection confirmed by tissue PCR and bacteriological culture but without lesions in gut tissues | | | B) Presence of multifocal, but not diffuse, lesions in gut tissues | | |
| ID | p value | FDR value | ID | p value | FDR value |
|---|---|---|---|---|---|
| 25:9470510:G :C | 5.34367E-07 | 0.044 | 24:37690274: T:C | 2.33E-07 | 0.0245 |
| 25:9470536:C :T | 5.34367E-07 | 0.044 | 24:37690802: G:C | 2.33E-07 | 0.0245 |
| 25:9470758:G :A | 5.34367E-07 | 0.044 | 24:37691028: A:C | 2.33E-07 | 0.0245 |
| 25:9470775:G :A | 5.34367E-07 | 0.044 | 22:4437859:C :T | 2.95E-07 | 0.0308 |
| 25:9471258:A :G | 3.93036E-07 | 0.044 | 11:13920933: C:T | 3.17E-07 | 0.0325 |
| 25:9473850:T :C | 5.34367E-07 | 0.044 | 11:13922761: G:A | 3.17E-07 | 0.0325 |
| 25:9487744:G :A | 3.93036E-07 | 0.044 | 11:10198629: A:C | 3.24E-07 | 0.0330 |
| 25:9489612:G :T | 3.93036E-07 | 0.044 | 11:13414642: C:G | 3.45E-07 | 0.0346 |
| 25:9491143:A :G | 3.93036E-07 | 0.044 | 11:13433802: T:TG | 3.45E-07 | 0.0346 |
| 25:12394597: G:A | 3.80512E-07 | 0.044 | 22:4418858:T :G | 3.63E-07 | 0.0351 |
| 25:12396223: C:T | 3.80512E-07 | 0.044 | 5:27811273:G :C | 3.82E-07 | 0.0351 |
| 25:12411628: C:T | 3.80512E-07 | 0.044 | 5:27818916:A :G | 3.82E-07 | 0.0351 |
| 25:12413134: C:A | 3.80512E-07 | 0.044 | 5:27833252:G :A | 3.82E-07 | 0.0351 |
| 25:12415339: G:A | 3.80512E-07 | 0.044 | 5:27854826:A :C | 3.82E-07 | 0.0351 |
| 25:12415928: G:C | 3.80512E-07 | 0.044 | 22:6365513:A :G | 4.85E-07 | 0.0351 |
| 25:12427653: T:C | 3.80512E-07 | 0.044 | 5:23774617:C :T | 4.92E-07 | 0.0351 |
| 25:12428267: T:C | 3.80512E-07 | 0.044 | 5:23774623:C :G | 4.92E-07 | 0.0351 |
| 25:12428660: T:C | 3.80512E-07 | 0.044 | 5:23775923:C :G | 4.92E-07 | 0.0351 |
| 25:12429377: T:C | 3.80512E-07 | 0.044 | 5:23776754:A :G | 4.92E-07 | 0.0351 |
| 25:12429409: A:C | 3.80512E-07 | 0.044 | 5:23792059:C :T | 4.92E-07 | 0.0351 |
| 25:12430197: T:C | 3.80512E-07 | 0.044 | 23:17826599: C:T | 4.92E-07 | 0.0351 |
| 26:20550339: G:C | 2.9187E-07 | 0.044 | 11:13754135: C:T | 5.72E-07 | 0.0351 |
| 26:20559555: C:A | 2.9187E-07 | 0.044 | 11:13775903: C:T | 5.72E-07 | 0.0351 |
| 26:34408564: C:G | 3.69227E-07 | 0.044 | 22:1943354:G :A | 6.26E-07 | 0.0351 |
| | | | 24:37693018: T:C | 6.46E-07 | 0.0351 |
| | | | 24:37693624: C:T | 6.46E-07 | 0.0351 |
| | | | 24:37695839: T:C | 6.46E-07 | 0.0351 |
| | | | 24:37696205: T:C | 6.46E-07 | 0.0351 |
| | | | 24:37696214: C:T | 6.46E-07 | 0.0351 |
| | | | 24:37696225: T:A | 6.46E-07 | 0.0351 |
| | | | 24:37696648: A:G | 6.46E-07 | 0.0351 |
| | | | 24:37696756: A:C | 6.46E-07 | 0.0351 |
| | | | 24:37699053: G:A | 6.46E-07 | 0.0351 |
| | | | 24:37699119: C:T | 6.46E-07 | 0.0351 |
| | | | 24:37699144: G:T | 6.46E-07 | 0.0351 |

(continued)

| SNPs associated to tolerance | | | | | |
|---|---|---|---|---|---|
| A) Infection confirmed by tissue PCR and bacteriological culture but without lesions in gut tissues | | | B) Presence of multifocal, but not diffuse, lesions in gut tissues | | |
| ID | p value | FDR value | ID | p value | FDR value |
| | | | 24:37699145: A:G | 6.46E-07 | 0.0351 |
| | | | 24:37699498: G:A | 6.46E-07 | 0.0351 |
| | | | 24:37699516: T:C | 6.46E-07 | 0.0351 |
| | | | 24:37699616: A:AG | 6.46E-07 | 0.0351 |
| | | | 24:37699738: C:T | 6.46E-07 | 0.0351 |
| | | | 24:37699749: A:G | 6.46E-07 | 0.0351 |
| | | | 24:37699788: T:C | 6.46E-07 | 0.0351 |
| | | | 24:37699901: T:C | 6.46E-07 | 0.0351 |
| | | | 24:37699966: T:C | 6.46E-07 | 0.0351 |
| | | | 24:37700064: T:C | 6.46E-07 | 0.0351 |
| | | | 24:37700406: G:A | 6.46E-07 | 0.0351 |
| | | | 24:37700431: C:A | 6.46E-07 | 0.0351 |
| | | | 24:37700468: A:C | 6.46E-07 | 0.0351 |
| | | | 24:37700560: A:G | 6.46E-07 | 0.0351 |
| | | | 24:37700715: G:A | 6.46E-07 | 0.0351 |
| | | | 24:37700734: G:C | 6.46E-07 | 0.0351 |
| | | | 24:37700744: A:G | 6.46E-07 | 0.0351 |
| | | | 24:37700957: C:T | 6.46E-07 | 0.0351 |
| | | | 24:37702688: C:T | 6.46E-07 | 0.0351 |
| | | | 24:37703330: G:A | 6.46E-07 | 0.0351 |
| | | | 24:37704108: C:T | 6.46E-07 | 0.0351 |
| | | | 24:37704200: C:T | 6.46E-07 | 0.0351 |
| | | | 24:37704745: T:C | 6.46E-07 | 0.0351 |
| | | | 24:37704802: T:C | 6.46E-07 | 0.0351 |
| | | | 24:37705115: C:T | 6.46E-07 | 0.0351 |
| | | | 24:37705143: C:T | 6.46E-07 | 0.0351 |
| | | | 24:37705554: T:C | 6.46E-07 | 0.0351 |
| | | | 24:37705612: T:C | 6.46E-07 | 0.0351 |
| | | | 24:37705881: C:G | 6.46E-07 | 0.0351 |
| | | | 24:37705952: CA:C | 6.46E-07 | 0.0351 |
| | | | 24:37706252: C:G | 6.46E-07 | 0.0351 |
| | | | 24:37706592: G:A | 6.46E-07 | 0.0351 |
| | | | 24:37706632: G:T | 6.46E-07 | 0.0351 |
| | | | 24:37707103: TTAAATTTT G:T | 6.46E-07 | 0.0351 |
| | | | 24:37707291: T:C | 6.46E-07 | 0.0351 |

(continued)

| SNPs associated to tolerance | | | | | |
| A) Infection confirmed by tissue PCR and bacteriological culture but without lesions in gut tissues | | | B) Presence of multifocal, but not diffuse, lesions in gut tissues | | |
| ID | p value | FDR value | ID | p value | FDR value |
| --- | --- | --- | --- | --- | --- |
|  |  |  | 24:37707831: G:A | 6.46E-07 | 0.0351 |
|  |  |  | 24:37711530: T:C | 6.46E-07 | 0.0351 |
|  |  |  | 24:37712545: G:T | 6.46E-07 | 0.0351 |
|  |  |  | 24:37714792: C:T | 6.46E-07 | 0.0351 |
|  |  |  | 24:37721457: A:T | 6.46E-07 | 0.0351 |
|  |  |  | 24:37724485: A:G | 6.46E-07 | 0.0351 |
|  |  |  | 24:37729703: A:G | 6.46E-07 | 0.0351 |
|  |  |  | 24:37738273: C:T | 6.46E-07 | 0.0351 |
|  |  |  | 24:37660165: G:A | 6.55E-07 | 0.0351 |
|  |  |  | 24:37680463: G:A | 6.55E-07 | 0.0351 |
|  |  |  | 22:2000785:A :T | 6.57E-07 | 0.0351 |
|  |  |  | 22:2001085:G :A | 6.57E-07 | 0.0351 |
|  |  |  | 22:5890769:G :A | 6.74E-07 | 0.0351 |
|  |  |  | 22:5891288:A :G | 6.74E-07 | 0.0351 |
|  |  |  | 22:5891775:T :C | 6.74E-07 | 0.0351 |
|  |  |  | 22:5891963:G :A | 6.74E-07 | 0.0351 |
|  |  |  | 22:5892019:C :T | 6.74E-07 | 0.0351 |
|  |  |  | 22:5892462:C :T | 6.74E-07 | 0.0351 |
|  |  |  | 22:5892479:C :T | 6.74E-07 | 0.0351 |
|  |  |  | 22:5894026:G :T | 6.74E-07 | 0.0351 |
|  |  |  | 11:10694387: A:C | 6.91E-07 | 0.0351 |
|  |  |  | 11:10694396: C:T | 6.91E-07 | 0.0351 |
|  |  |  | 11:10694672: C:T | 6.91E-07 | 0.0351 |
|  |  |  | 11:10694892: A:G | 6.91E-07 | 0.0351 |
|  |  |  | 11:10695132: A:AT | 6.91E-07 | 0.0351 |
|  |  |  | 11:10695844: G:C | 6.91E-07 | 0.0351 |
|  |  |  | 11:10697227: C:T | 6.91E-07 | 0.0351 |
|  |  |  | 11:10700107: G:A | 6.91E-07 | 0.0351 |
|  |  |  | 11:10707400: G:A | 6.91E-07 | 0.0351 |
|  |  |  | 11:10718349: C:T | 6.91E-07 | 0.0351 |
|  |  |  | 11:10821244: GAA:G | 6.91E-07 | 0.0351 |
|  |  |  | 11:10827966: A:T | 6.91E-07 | 0.0351 |
|  |  |  | 11:10832068: A:G | 6.91E-07 | 0.0351 |
|  |  |  | 11:10834253: G:A | 6.91E-07 | 0.0351 |
|  |  |  | 11:10834338: G:A | 6.91E-07 | 0.0351 |

(continued)

| SNPs associated to tolerance | | | | | |
| A) Infection confirmed by tissue PCR and bacteriological culture but without lesions in gut tissues | | | B) Presence of multifocal, but not diffuse, lesions in gut tissues | | |
| ID | p value | FDR value | ID | p value | FDR value |
|---|---|---|---|---|---|
| | | | 11:10844086: T:G | 6.91E-07 | 0.0351 |
| | | | 11:10848486: C:G | 6.91E-07 | 0.0351 |
| | | | 11:10855475: C:A | 6.91E-07 | 0.0351 |
| | | | 11:10864839: A:G | 6.91E-07 | 0.0351 |
| | | | 11:10899285: G:A | 6.91E-07 | 0.0351 |
| | | | 11:10911692: G:A | 6.91E-07 | 0.0351 |
| | | | 11:10950396: C:T | 6.91E-07 | 0.0351 |
| | | | 11:10955428: T:C | 6.91E-07 | 0.0351 |
| | | | 11:10959465: T:C | 6.91E-07 | 0.0351 |
| | | | 11:10965954: T:C | 6.91E-07 | 0.0351 |
| | | | 11:10966737: T:C | 6.91E-07 | 0.0351 |
| | | | 11:10967866: ATA | 6.91E-07 | 0.0351 |
| | | | 11:10968448: T:C | 6.91E-07 | 0.0351 |
| | | | 11:10969030: C:T | 6.91E-07 | 0.0351 |
| | | | 11:10973590: G:A | 6.91E-07 | 0.0351 |
| | | | 11:10977074: T:C | 6.91E-07 | 0.0351 |
| | | | 11:10978400: A:C | 6.91E-07 | 0.0351 |
| | | | 11:10978497: C:T | 6.91E-07 | 0.0351 |
| | | | 11:10983941: T:C | 6.91E-07 | 0.0351 |
| | | | 11:10991920: AGTCTCAT GG:A | 6.91E-07 | 0.0351 |
| | | | 11:10993684: T:C | 6.91E-07 | 0.0351 |
| | | | 11:11000189: T:C | 6.91E-07 | 0.0351 |
| | | | 21:14274619: G:A | 6.92E-07 | 0.0351 |
| | | | 21:14275234: C:T | 6.92E-07 | 0.0351 |
| | | | 21:14279491: C:T | 6.92E-07 | 0.0351 |
| | | | 21:14282606: G:C | 6.92E-07 | 0.0351 |
| | | | 21:14284061: G:A | 6.92E-07 | 0.0351 |
| | | | 21:14285802: G:A | 6.92E-07 | 0.0351 |
| | | | 23:16438283: T:C | 7.36E-07 | 0.0368 |
| | | | 23:16462523: T:G | 7.36E-07 | 0.0368 |
| | | | 23:16489599: G:A | 7.36E-07 | 0.0368 |
| | | | 11:9944399:G :A | 7.51E-07 | 0.0368 |
| | | | 11:9953514:G :A | 7.51E-07 | 0.0368 |
| | | | 11:9956952:T :C | 7.51E-07 | 0.0368 |
| | | | 22:3421269:T :G | 8.09E-07 | 0.0368 |

(continued)

| SNPs associated to tolerance | | | | | |
|---|---|---|---|---|---|
| A) Infection confirmed by tissue PCR and bacteriological culture but without lesions in gut tissues | | | B) Presence of multifocal, but not diffuse, lesions in gut tissues | | |
| ID | p value | FDR value | ID | p value | FDR value |
| | | | 5:27878206:C :T | 8.36E-07 | 0.0368 |
| | | | 23:17825116: C:A | 8.43E-07 | 0.0368 |
| | | | 23:17825281: G:A | 8.43E-07 | 0.0368 |
| | | | 23:17825391: A:G | 8.43E-07 | 0.0368 |
| | | | 23:17825421: C:A | 8.43E-07 | 0.0368 |
| | | | 23:17825423: C:T | 8.43E-07 | 0.0368 |
| | | | 23:17825476: A:G | 8.43E-07 | 0.0368 |
| | | | 23:17825664: C:T | 8.43E-07 | 0.0368 |
| | | | 23:17825677: C:T | 8.43E-07 | 0.0368 |
| | | | 23:17825686: C:T | 8.43E-07 | 0.0368 |
| | | | 23:17825908: A:G | 8.43E-07 | 0.0368 |
| | | | 23:17826142: T:C | 8.43E-07 | 0.0368 |
| | | | 23:17826155: A:G | 8.43E-07 | 0.0368 |
| | | | 23:17826186: T:C | 8.43E-07 | 0.0368 |
| | | | 23:17826381: G:A | 8.43E-07 | 0.0368 |
| | | | 23:17826741: C:T | 8.43E-07 | 0.0368 |
| | | | 23:17826807: G:A | 8.43E-07 | 0.0368 |
| | | | 23:17827033: C:T | 8.43E-07 | 0.0368 |
| | | | 23:17827039: A:G | 8.43E-07 | 0.0368 |
| | | | 23:17827139: G:T | 8.43E-07 | 0.0368 |
| | | | 23:17827574: G:T | 8.43E-07 | 0.0368 |
| | | | 23:17827681: G:A | 8.43E-07 | 0.0368 |
| | | | 23:17828035: T:C | 8.43E-07 | 0.0368 |
| | | | 23:17829714: C:T | 8.43E-07 | 0.0368 |
| | | | 23:17829793: C:CTT | 8.43E-07 | 0.0368 |
| | | | 23:17830062: G:C | 8.43E-07 | 0.0368 |
| | | | 23:17830541: A:G | 8.43E-07 | 0.0368 |
| | | | 23:17830832: A:G | 8.43E-07 | 0.0368 |
| | | | 23:17831527: G:A | 8.43E-07 | 0.0368 |
| | | | 23:17831640: G:A | 8.43E-07 | 0.0368 |
| | | | 23:17832330: T:C | 8.43E-07 | 0.0368 |
| | | | 23:17832789: T:C | 8.43E-07 | 0.0368 |
| | | | 5:27798253:G :A | 8.51E-07 | 0.0368 |
| | | | 5:27823195:G :A | 8.51E-07 | 0.0368 |
| | | | 5:27824589:C :T | 8.51E-07 | 0.0368 |

(continued)

| SNPs associated to tolerance | | | | | |
|---|---|---|---|---|---|
| A) Infection confirmed by tissue PCR and bacteriological culture but without lesions in gut tissues | | | B) Presence of multifocal, but not diffuse, lesions in gut tissues | | |
| ID | p value | FDR value | ID | p value | FDR value |
| | | | 11:9970522:G :A | 8.63E-07 | 0.0368 |
| | | | 11:9972022:A :G | 8.63E-07 | 0.0368 |
| | | | 11:9972957:C :T | 8.63E-07 | 0.0368 |
| | | | 11:9974616:C A:C | 8.63E-07 | 0.0368 |
| | | | 11:9977670:G :A | 8.63E-07 | 0.0368 |
| | | | 11:9995132:T :C | 8.63E-07 | 0.0368 |
| | | | 22:3193472:A :G | 8.71E-07 | 0.0370 |
| | | | 22:4402737:T :C | 9.93E-07 | 0.0413 |
| | | | 11:9883803:G :A | 1.03E-06 | 0.0413 |
| | | | 11:9885234:G :A | 1.03E-06 | 0.0413 |
| | | | 11:9896834:C :G | 1.03E-06 | 0.0413 |
| | | | 22:3429156:A :T | 1.04E-06 | 0.0413 |
| | | | 22:3429161:T :A | 1.04E-06 | 0.0413 |
| | | | 22:3443080:T :A | 1.04E-06 | 0.0413 |
| | | | 5:25368526:A :G | 1.12E-06 | 0.0413 |
| | | | 5:25397349:G :T | 1.12E-06 | 0.0413 |
| | | | 5:25397729:A :G | 1.12E-06 | 0.0413 |
| | | | 5:25399115:C :T | 1.12E-06 | 0.0413 |
| | | | 5:25768967:C :T | 1.12E-06 | 0.0413 |
| | | | 5:25775186:G :A | 1.12E-06 | 0.0413 |
| | | | 5:25789560:T :C | 1.12E-06 | 0.0413 |
| | | | 5:25925784:T TTTCTCTGG TCCAAGGT CCTTAGGC AGAGGTGG GTAGCTGC CACTGGGG CCCAGGGC: T | 1.12E-06 | 0.0413 |
| | | | 5:25947490:T :C | 1.12E-06 | 0.0413 |
| | | | 5:25997741:C :T | 1.12E-06 | 0.0413 |
| | | | 5:26231815:C :T | 1.12E-06 | 0.0413 |
| | | | 5:26268203:T :C | 1.12E-06 | 0.0413 |
| | | | 5:26285431:C :T | 1.12E-06 | 0.0413 |
| | | | 5:26322262:G :T | 1.12E-06 | 0.0413 |
| | | | 5:27438266:A :C | 1.12E-06 | 0.0413 |
| | | | 28:43578814: G:A | 1.14E-06 | 0.0413 |
| | | | 28:43579152: T:C | 1.14E-06 | 0.0413 |
| | | | 28:43582705: C:T | 1.14E-06 | 0.0413 |
| | | | 28:43582996: A:G | 1.14E-06 | 0.0413 |

(continued)

| SNPs associated to tolerance | | | | | |
| A) Infection confirmed by tissue PCR and bacteriological culture but without lesions in gut tissues | | | B) Presence of multifocal, but not diffuse, lesions in gut tissues | | |
| ID | p value | FDR value | ID | p value | FDR value |
|---|---|---|---|---|---|
| | | | 28:43583222: A:C | 1.14E-06 | 0.0413 |
| | | | 28:43589380: G:A | 1.14E-06 | 0.0413 |
| | | | 28:43589797: T:C | 1.14E-06 | 0.0413 |
| | | | 28:43590710: A:G | 1.14E-06 | 0.0413 |
| | | | 5:23624140:C :G | 1.19E-06 | 0.0413 |
| | | | 5:23648132:C :CAT | 1.19E-06 | 0.0413 |
| | | | 18:1555288:C :T | 1.19E-06 | 0.0413 |
| | | | 18:504524:A: C | 1.20E-06 | 0.0413 |
| | | | 18:505810:A: G | 1.20E-06 | 0.0413 |
| | | | 18:505939:G: A | 1.20E-06 | 0.0413 |
| | | | 18:505992:C: T | 1.20E-06 | 0.0413 |
| | | | 18:507033:A: C | 1.20E-06 | 0.0413 |
| | | | 18:508316:G: A | 1.20E-06 | 0.0413 |
| | | | 18:508820:T: C | 1.20E-06 | 0.0413 |
| | | | 18:509878:C: A | 1.20E-06 | 0.0413 |
| | | | 18:511155:T: C | 1.20E-06 | 0.0413 |
| | | | 18:515358:G: T | 1.20E-06 | 0.0413 |
| | | | 18:519104:G: A | 1.20E-06 | 0.0413 |
| | | | 18:519757:A: G | 1.20E-06 | 0.0413 |
| | | | 18:520477:G: A | 1.20E-06 | 0.0413 |
| | | | 18:521052:G: A | 1.20E-06 | 0.0413 |
| | | | 18:523850:C: A | 1.20E-06 | 0.0413 |
| | | | 18:526486:C: A | 1.20E-06 | 0.0413 |
| | | | 18:527142:C: CT | 1.20E-06 | 0.0413 |
| | | | 18:527220:T: C | 1.20E-06 | 0.0413 |
| | | | 18:528300:T: C | 1.20E-06 | 0.0413 |
| | | | 18:528787:T: A | 1.20E-06 | 0.0413 |
| | | | 11:10810645: C:T | 1.22E-06 | 0.0413 |
| | | | 23:14532683: C:A | 1.27E-06 | 0.0413 |
| | | | 23:14533572: G:T | 1.27E-06 | 0.0413 |
| | | | 23:14541185: C:T | 1.27E-06 | 0.0413 |
| | | | 23:14603647: C:T | 1.27E-06 | 0.0413 |
| | | | 22:4418515:C :G | 1.29E-06 | 0.0413 |
| | | | 11:10961685: T:C | 1.29E-06 | 0.0413 |
| | | | 22:1177011:A :C | 1.29E-06 | 0.0413 |

(continued)

| SNPs associated to tolerance | | | | | |
|---|---|---|---|---|---|
| A) Infection confirmed by tissue PCR and bacteriological culture but without lesions in gut tissues | | | B) Presence of multifocal, but not diffuse, lesions in gut tissues | | |
| ID | p value | FDR value | ID | p value | FDR value |
| | | | 22:1180143:C :T | 1.29E-06 | 0.0413 |
| | | | 22:1187242:C :G | 1.29E-06 | 0.0413 |
| | | | 28:32439041: G:T | 1.31E-06 | 0.0413 |
| | | | 28:32439057: C:T | 1.31E-06 | 0.0413 |
| | | | 28:32439092: A:T | 1.31E-06 | 0.0413 |
| | | | 11:10558098: C:T | 1.32E-06 | 0.0413 |
| | | | 11:10583979: G:A | 1.32E-06 | 0.0413 |
| | | | 11:10587544: G:A | 1.32E-06 | 0.0413 |
| | | | 11:10591481: C:T | 1.32E-06 | 0.0413 |
| | | | 11:10592316: G:A | 1.32E-06 | 0.0413 |
| | | | 11:10593381: G:A | 1.32E-06 | 0.0413 |
| | | | 11:10593885: T:C | 1.32E-06 | 0.0413 |
| | | | 11:10594890: A:G | 1.32E-06 | 0.0413 |
| | | | 11:10594967: G:A | 1.32E-06 | 0.0413 |
| | | | 11:10595262: C:G | 1.32E-06 | 0.0413 |
| | | | 11:10596554: C:T | 1.32E-06 | 0.0413 |
| | | | 11:10597193: CTTAG:C | 1.32E-06 | 0.0413 |
| | | | 11:10599176: GACT:G | 1.32E-06 | 0.0413 |
| | | | 11:10599224: G:A | 1.32E-06 | 0.0413 |
| | | | 11:10599378: A:AC | 1.32E-06 | 0.0413 |
| | | | 11:10600322: G:T | 1.32E-06 | 0.0413 |
| | | | 11:10603017: T:TC | 1.32E-06 | 0.0413 |
| | | | 11:10603936: A:C | 1.32E-06 | 0.0413 |
| | | | 11:10607896: G:C | 1.32E-06 | 0.0413 |
| | | | 11:10608312: G:A | 1.32E-06 | 0.0413 |
| | | | 11:10610947: T:C | 1.32E-06 | 0.0413 |
| | | | 11:10616205: G:A | 1.32E-06 | 0.0413 |
| | | | 11:10617113: C:T | 1.32E-06 | 0.0413 |
| | | | 11:10619542: G:A | 1.32E-06 | 0.0413 |
| | | | 11:10619901: C:T | 1.32E-06 | 0.0413 |
| | | | 11:10620060: C:T | 1.32E-06 | 0.0413 |
| | | | 11:10620514: GA:G | 1.32E-06 | 0.0413 |
| | | | 11:10621824: G:A | 1.32E-06 | 0.0413 |
| | | | 11:10625571: G:A | 1.32E-06 | 0.0413 |
| | | | 11:10627687: A:T | 1.32E-06 | 0.0413 |

(continued)

| SNPs associated to tolerance | | | | | |
| A) Infection confirmed by tissue PCR and bacteriological culture but without lesions in gut tissues | | | B) Presence of multifocal, but not diffuse, lesions in gut tissues | | |
| ID | p value | FDR value | ID | p value | FDR value |
| --- | --- | --- | --- | --- | --- |
| | | | 11:10630386: G:A | 1.32E-06 | 0.0413 |
| | | | 11:10656942: C:A | 1.32E-06 | 0.0413 |
| | | | 11:10666685: G:C | 1.32E-06 | 0.0413 |
| | | | 11:10672036: G:T | 1.32E-06 | 0.0413 |
| | | | 11:10673102: G:T | 1.32E-06 | 0.0413 |
| | | | 11:10680023: C:T | 1.32E-06 | 0.0413 |
| | | | 11:10680917: T:A | 1.32E-06 | 0.0413 |
| | | | 11:10685248: G:C | 1.32E-06 | 0.0413 |
| | | | 11:10687356: G:A | 1.32E-06 | 0.0413 |
| | | | 11:10689817: C:T | 1.32E-06 | 0.0413 |
| | | | 11:10690852: C:A | 1.32E-06 | 0.0413 |
| | | | 11:10692309: C:T | 1.32E-06 | 0.0413 |
| | | | 11:9854782:A :G | 1.33E-06 | 0.0415 |
| | | | 5:23777588:C TAGTTTA:C | 1.42E-06 | 0.0428 |
| | | | 11:10003963: T:G | 1.43E-06 | 0.0428 |
| | | | 11:10005762: T:G | 1.43E-06 | 0.0428 |
| | | | 11:10006956: CAG:C | 1.43E-06 | 0.0428 |
| | | | 11:10008370: T:A | 1.43E-06 | 0.0428 |
| | | | 11:10016588: A:G | 1.43E-06 | 0.0428 |
| | | | 11:10019900: A:G | 1.43E-06 | 0.0428 |
| | | | 11:10024611: G:A | 1.43E-06 | 0.0428 |
| | | | 11:10030174: G:A | 1.43E-06 | 0.0428 |
| | | | 11:10039585: T:A | 1.43E-06 | 0.0428 |
| | | | 11:10043441: A:G | 1.43E-06 | 0.0428 |
| | | | 11:10049607: T:C | 1.43E-06 | 0.0428 |
| | | | 11:9979194:T :TTC | 1.43E-06 | 0.0428 |
| | | | 11:9979340:G :A | 1.43E-06 | 0.0428 |
| | | | 11:9985688:T :C | 1.43E-06 | 0.0428 |
| | | | 11:9989852:G GCC:G | 1.43E-06 | 0.0428 |
| | | | 5:25547142:T :A | 1.50E-06 | 0.0443 |
| | | | 5:25550441:A :C | 1.50E-06 | 0.0443 |
| | | | 5:25552936:A :C | 1.50E-06 | 0.0443 |
| | | | 5:25556137:G :T | 1.50E-06 | 0.0443 |
| | | | 5:25583759:A :C | 1.50E-06 | 0.0443 |
| | | | 5:25594005:T :C | 1.50E-06 | 0.0443 |

(continued)

| SNPs associated to tolerance | | | | | |
|---|---|---|---|---|---|
| A) Infection confirmed by tissue PCR and bacteriological culture but without lesions in gut tissues | | | B) Presence of multifocal, but not diffuse, lesions in gut tissues | | |
| ID | p value | FDR value | ID | p value | FDR value |
| | | | 22:8115353:C :T | 1.53E-06 | 0.0445 |
| | | | 22:8115355:C :T | 1.53E-06 | 0.0445 |
| | | | 22:8115378:G :A | 1.53E-06 | 0.0445 |
| | | | 22:8115387:G :A | 1.53E-06 | 0.0445 |
| | | | 22:8115398:C :T | 1.53E-06 | 0.0445 |
| | | | 22:8115408:A :G | 1.53E-06 | 0.0445 |
| | | | 11:10329826: A:T | 1.55E-06 | 0.0447 |
| | | | 11:10416949: A:G | 1.55E-06 | 0.0447 |
| | | | 11:10484685: C:T | 1.55E-06 | 0.0447 |
| | | | 11:10491203: A:G | 1.55E-06 | 0.0447 |
| | | | 11:10517597: C:A | 1.55E-06 | 0.0447 |
| | | | 11:10521760: T:A | 1.55E-06 | 0.0447 |
| | | | 23:17832348: T:C | 1.56E-06 | 0.0447 |
| | | | 4:12139927:A :C | 1.58E-06 | 0.0451 |
| | | | 23:13571756: A:G | 1.62E-06 | 0.0463 |
| | | | 1:109792787: A:G | 1.68E-06 | 0.0478 |
| | | | 1:109792856: G:A | 1.68E-06 | 0.0478 |
| | | | 28:32438743: G:C | 1.71E-06 | 0.0485 |

**Table 4.** Ability to predict susceptibility, resistance and tolerance using genomic prediction models based on the SNPs represented on Tables 1, 2 and 3.

| Traits | SNPs | AUC |
|---|---|---|
| Susceptibility: infection confirmed by ELISA, PCR and bacteriological culture | All SNPs shown in **Table 1A** (398) | 0.81 |
| Susceptibility: presence of multifocal lesions in gut tissues | All SNPs shown in **Table 1B** (752) | 0.80 |
| Resistance: Low MAP load in macrophages | All SNPs shown in **Table 2A** (6) | 0.95 |
| Resistance: High IFNγ production in response to aviar tuberculin | All SNPs shown in **Table 2B** (310) | 0.98 |
| Tolerance: infection confirmed by tissue PCR and bacteriological culture but without lesions in gut tissues | All SNPs shown in **Table 3A** (142) | 0.85 |
| Tolerance: Presence of multifocal, but not diffuse, lesions in gut tissues | All SNPs shown in **Table 3B** (449) | 0.90 |

**Claims**

1. *In vitro* method for classifying and/or selecting cattle according to their susceptibility, tolerance and/or resistance to *Mycobacterium avium* subsp. paratuberculosis (MAP) infection, which comprises:

a. determining the identity of at least one single nucleotide polymorphism (SNP) selected from the list shown in Tables 1, 2 or 3 in a biological sample obtained from cattle, wherein determining the identity of the at least one SNP comprises determining the identity of the allele present at the SNP position or determining the identity of a genetic variant in linkage disequilibrium with the allele present at the SNP position,

b. wherein the identification of the alternative allele of at least one SNP selected from the list shown in Table 1 is an indication that the cattle are susceptible to MAP; or

c. wherein the identification of the alternative allele of at least one SNP selected from the list shown in Table 2 is an indication that the cattle are resistant to MAP; or

d. wherein the identification of the alternative allele of at least one SNP selected from the list shown in Table 3 is an indication that the cattle are tolerant to paratuberculosis (PTB).

2. *In vitro* method, according to claim 1, for classifying and/or selecting cattle according to their susceptibility to MAP, wherein susceptibility is determined according to their probability of testing positive for MAP infection, which comprises:

a. determining the identity of at least one SNP selected from the list shown in Table 1A in a biological sample obtained from cattle, wherein determining the identity of the at least one SNP comprises determining the identity of the allele present at the SNP position or determining the identity of a genetic variant in linkage disequilibrium with the allele present at the SNP position,

b. wherein the identification of the alternative allele of at least one SNP selected from the list shown in Table 1A, is an indication that the cattle have a high probability of testing positive for MAP infection and are therefore susceptible to MAP.

3. *In vitro* method, according to claim 1, for classifying and/or selecting cattle according to their susceptibility to MAP, wherein susceptibility is determined according to their probability of presenting diffuse lesions in gut and associated lymphoid tissues, which comprises:

a. determining the identity of at least one SNP selected from the list shown in Table 1B in a biological sample obtained from cattle, wherein determining the identity of the at least one SNP comprises determining the identity of the allele present at the SNP position or determining the identity of a genetic variant in linkage disequilibrium with the allele present at the SNP position,

b. wherein the identification of the alternative allele of at least one SNP selected from the list shown in Table 1B is an indication that the cattle have a high probability of presenting diffuse lesions in gut tissues and are therefore susceptible to MAP.

4. *In vitro* method, according to claim 1, for classifying and/or selecting cattle according to their resistance to MAP, wherein resistance is determined according to their probability of having low MAP load in macrophages, which comprises:

a. determining the identity of at least one SNP selected from the list shown in Table 2A in a biological sample obtained from cattle, wherein determining the identity of the at least one SNP comprises determining the identity of the allele present at the SNP position or determining the identity of a genetic variant in linkage disequilibrium with the allele present at the SNP position,

b. wherein the identification of the alternative allele of at least one SNP selected from the list shown in Table 2A is an indication that the cattle have a high probability of having low MAP load in macrophages and are therefore resistant to MAP.

5. *In vitro* method, according to claim 1, for classifying and/or selecting cattle according to their resistance to MAP, wherein resistance is determined according to their probability of producing high levels of IFN-gamma in blood stimulated with avian tuberculin, which comprises:

a. determining the identity of at least one SNP selected from the list shown in Table 2B in a biological sample obtained from cattle, wherein determining the identity of the at least one SNP comprises determining the identity of the allele present at the SNP position or determining the identity of a genetic variant in linkage disequilibrium with the allele present at the SNP position,

b. wherein the identification of the alternative allele of at least one SNP selected from the list shown in Table 2B is an indication that blood samples from the cattle have a high probability of producing high levels of IFN-gamma after stimulation with avian tuberculin and are therefore resistant to MAP.

6. *In vitro* method, according to claim 1, for classifying and/or selecting cattle according to their tolerance to PTB, wherein tolerance is determined according to their probability of having no lesions in gut tissues despite testing positive for MAP infection, which comprises:

    a. determining the identity of at least one SNP selected from the list shown in Table 3A in a biological sample obtained from cattle, wherein determining the identity of the at least one SNP comprises determining the identity of the allele present at the SNP position or determining the identity of a genetic variant in linkage disequilibrium with the allele present at the SNP position,
    b. wherein the identification of the alternative allele of at least one SNP selected from the list shown in Table 3A is an indication that the cattle have a high probability of presenting no lesions in gut tissues despite testing positive for MAP infection and are therefore tolerant to PTB.

7. *In vitro* method, according to claim 1, for classifying and/or selecting cattle according to their tolerance to PTB, wherein tolerance is determined according to their probability of having multifocal, but not diffuse, lesions in gut tissues, which comprises:

    a. determining the identity of at least one SNP selected from the list shown in Table 3B in a biological sample obtained from cattle, wherein determining the identity of the at least one SNP comprises determining the identity of the allele present at the SNP position or determining the identity of a genetic variant in linkage disequilibrium with the allele present at the SNP position,
    b. wherein the identification of the alternative allele of at least one SNP selected from the list shown in Table 3B is an indication that the cattle have a high probability of having multifocal, but not diffuse, lesions in gut tissues and are therefore tolerant to PTB.

8. *In vitro* method, according to any of the previous claims, which comprises:

    a. determining the identity of all the SNPs selected from the list shown in Tables 1, 2 or 3 in a biological sample obtained from cattle, wherein determining the identity of the at least one SNP comprises determining the identity of the allele present at the SNP position or determining the identity of a genetic variant in linkage disequilibrium with the allele present at the SNP position,
    b. wherein the identification of the alternative allele of all the SNPs of Table 1, is an indication that the cattle are susceptible to MAP; or
    c. wherein the identification of the alternative allele of all the SNPs of Table 2, is an indication that the cattle are resistant to MAP; or
    d. wherein the identification of the alternative allele of all the SNPs of Table 3, is an indication that the cattle are tolerant to PTB.

9. *In vitro* method, according to any of the previous claims, wherein the test for MAP infection is performed by ELISA, tissue PCR, bacteriological culture for MAP detection, histopathological analysis, Interferon gamma release assay (IGRA) and/or MGIA (Mycobacterial growth inhibition assay), and wherein step a) is performed by using PCR, qPCR, allele-specific PCR, allele-specific qPCR, SNP array, whole genome sequencing, genomic prediction and/or genotype imputation.

10. *In vitro* method, according to any of the previous claims, wherein the cattle are selected from the group comprising: a cow, a bull, heifers, bullocks, steers, beef cattle and dairy cattle.

11. *In vitro* method, according to any of the previous claims, wherein the biological sample is a nucleic acid sample, preferably a nucleic acid sample derived from peripheral blood.

12. *In vitro* use of at least one or all the SNPs selected from the list shown in Tables 1, 2 or 3, or of a genetic variant in linkage disequilibrium with the at least one or all the SNPs, or of a kit comprising reagents for the determination of at least one or all the SNPs selected from the list shown in Tables 1, 2 or 3, or of a genetic variant in linkage disequilibrium with the at least one or all the SNPs, for classifying and/or selecting cattle according to their susceptibility, tolerance and/or resistance to MAP infection.

13. Use, according to claim 12, of at least one or all the SNPs selected from the list shown in Table 1A, or of a genetic variant in linkage disequilibrium with the at least one or all the SNPs, or of a kit comprising reagents for the determination of at least one or all the SNPs selected from the list shown in Table 1A, or of a genetic variant in linkage disequilibrium with the at least one or all the SNPs, for classifying and/or selecting cattle according to their susceptibility to MAP infection by assessing their probability of testing positive for MAP infection; or use of at least one or all the SNPs selected from the list shown in Table 1B, or of a genetic variant in linkage disequilibrium with the at least one or all the SNPs, or of a kit comprising reagents for the determination of at least one or all the SNPs selected from the list shown in Table 1B, or of a genetic variant in linkage disequilibrium with the at least one or all the SNPs, for classifying and/or selecting cattle according to their susceptibility to MAP infection by assessing their probability of presenting diffuse lesions in gut tissues.

14. Use, according to claim 12, of at least one or all the SNPs selected from the list shown in Table 2A, or of a genetic variant in linkage disequilibrium with the at least one or all the SNPs, or of a kit comprising reagents for the determination of at least one or all the SNPs selected from the list shown in Table 2A, or of a genetic variant in linkage disequilibrium with the at least one or all the SNPs, for classifying and/or selecting cattle according to their resistance to MAP infection by assessing their probability of having low MAP load in macrophages; or use of at least one or all the SNPs selected from the list shown in Table 2B, or of a genetic variant in linkage disequilibrium with the at least one or all the SNPs, or of a kit comprising reagents for the determination of at least one or all the SNPs selected from the list shown in Table 2B, or of a genetic variant in linkage disequilibrium with the at least one or all the SNPs, for classifying and/or selecting cattle according to their resistance to MAP infection by assessing their probability of producing high levels of IFN-gamma in response to avian tuberculin.

15. Use, according to claim 12, of at least one or all the SNPs selected from the list shown in Table 3A, or of a genetic variant in linkage disequilibrium with the at least one or all the SNPs, or of a kit comprising reagents for the determination of at least one or all the SNPs selected from the list shown in Table 3A, or of a genetic variant in linkage disequilibrium with the at least one or all the SNPs, for classifying and/or selecting cattle according to their tolerance to PTB by assessing their probability of having no lesions in gut tissues despite testing positive for MAP infection; or use of at least one or all the SNPs selected from the list shown in Table 3B, or of a genetic variant in linkage disequilibrium with the at the least one or all the SNPs, or of a kit comprising reagents for the determination of at least one or all the SNPs selected from the list shown in Table 3B, or of a genetic variant in linkage disequilibrium with the at least one or all the SNPs, for classifying and/or selecting cattle according to their tolerance to PTB infection by assessing their probability of having multifocal, but not diffuse, lesions in gut tissues.

**Figure 1**

gEBVs of the target population and categorization

**Plink1.9**
compute the polygenic risk scores of the animals (gEBVs) based on the b value of the PTB-associated SNPs

PTB-associated SNPs effects

Allelic variants in the SNPs associated with the PTB phenoypes

+

gEBVs of the training population

genotyping

PTB phenotypes

**GCTA 1.93.3**
estimate SNP effects by GBLUP

Genomic relationship matrix

Target population

Reference population

genotyping

## EUROPEAN SEARCH REPORT

Application Number

EP 23 38 2757

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/223687 A1 (NEIBERGS HOLLY L [US] ET AL) 2 September 2010 (2010-09-02) * Claims, examples * | 1-3,8-13 | INV. C12Q1/6883 |
| X | CA 2 675 242 A1 (UNIV GUELPH [CA]) 11 February 2011 (2011-02-11) * Claims; paragraph [0160]; table 7 * | 1-3,8-13 | |
| X | CANIVE MARIA ET AL: "Identification of loci associated with pathological outcomes in Holstein cattle infected with Mycobacterium avium subsp. paratuberculosis using whole-genome sequence data", SCIENTIFIC REPORTS, vol. 11, no. 1, 11 October 2021 (2021-10-11), XP093118888, US ISSN: 2045-2322, DOI: 10.1038/s41598-021-99672-4 Retrieved from the Internet: URL:https://www.nature.com/articles/s41598-021-99672-4> * the whole document * | 1-3,8-13 | |
| X | ALONSO-HEARN MARTA ET AL: "Genome-wide association studies for the identification of cattle susceptible and resilient to paratuberculosis", FRONTIERS IN VETERINARY SCIENCE, vol. 9, 12 September 2022 (2022-09-12), XP093118895, Lausanne ISSN: 2297-1769, DOI: 10.3389/fvets.2022.935133 * the whole document * | 1-3,8-13 | |

TECHNICAL FIELDS SEARCHED (IPC)

C12Q

-/--

The present search report has been drawn up for all claims

| Place of search | Date of completion of the search | Examiner |
|---|---|---|
| Munich | 15 January 2024 | Bradbrook, Derek |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 23 38 2757

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/215338 A1 (KIRKPATRICK BRIAN W [US] ET AL) 28 July 2016 (2016-07-28) * paragraph [0014] - paragraph [0019]; claims 24-51; tables A-D * | 1-3,8-13 | |
| X | BADIA-BRINGUÉ GERARD ET AL: "Control of Mycobacterium avium subsp. paratuberculosis load within infected bovine monocyte-derived macrophages is associated with host genetics", FRONTIERS IN IMMUNOLOGY, vol. 14, 22 February 2023 (2023-02-22), XP093118929, Lausanne, CH ISSN: 1664-3224, DOI: 10.3389/fimmu.2023.1042638 * the whole document * | 1-3,8-13 | |
| X | CANIVE MARÍA ET AL: "A Genome-Wide Association Study for Tolerance to Paratuberculosis Identifies Candidate Genes Involved in DNA Packaging, DNA Damage Repair, Innate Immunity, and Pathogen Persistence", FRONTIERS IN IMMUNOLOGY, vol. 13, 6 April 2022 (2022-04-06), XP093118927, Lausanne, CH ISSN: 1664-3224, DOI: 10.3389/fimmu.2022.820965 * the whole document * | 1-3,8-13 | **TECHNICAL FIELDS SEARCHED (IPC)** |

-/--

The present search report has been drawn up for all claims

| Place of search | Date of completion of the search | Examiner |
|---|---|---|
| Munich | 15 January 2024 | Bradbrook, Derek |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

**EUROPEAN SEARCH REPORT**

Application Number

EP 23 38 2757

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KIRKPATRICK B W ET AL: "Whole-Genome association analysis of susceptibility to paratuberculosis in Holstein cattle", ANIMAL GENETICS, BLACKWELL SCIENTIFIC PUBLICATIONS, LONDON, GB, vol. 42, no. 2, 9 March 2011 (2011-03-09), pages 149-160, XP071536401, ISSN: 0268-9146, DOI: 10.1111/J.1365-2052.2010.02097.X * the whole document * | 1-3,8-13 | |

-----

TECHNICAL FIELDS
SEARCHED (IPC)

~~The present search report has been drawn up for all claims~~

| Place of search | Date of completion of the search | Examiner |
|---|---|---|
| Munich | 15 January 2024 | Bradbrook, Derek |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

**Application Number**

**EP 23 38 2757**

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

**1-3, 8-13(all partially)**

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION**

**SHEET B**

Application Number

EP 23 38 2757

The Search Division considers that the present European patent application does not comply with the requirements of unity of invention and relates to several inventions or groups of inventions, namely:

1. claims: 1-3, 8-13(all partially)

   Methods for classifying and/or selecting cattle according to their susceptibility, tolerance and/or resistance to Mycobacterium avium subsp. paratuberculosis (MAP) infection, comprising determining the identity of at least one single nucleotide polymorphism (SNP) selected from the list shown in Tables 1, 2 or 3 in a biological sample obtained from cattle, wherein determining the identity of the at least one SNP comprises determining the identity of the allele present at the SNP position or determining the identity of a genetic variant in linkage disequilibrium with the allele present at the SNP position, wherein said SNP is the first shown in Table 1, namely 23:15594386: CCATGGGGT:C, and is associated with susceptibility.
   ---

2. claims: 1-3, 8-13(all partially)

   Methods for classifying and/or selecting cattle according to their susceptibility, tolerance and/or resistance to Mycobacterium avium subsp. paratuberculosis (MAP) infection, comprising determining the identity of at least one single nucleotide polymorphism (SNP) selected from the list shown in Tables 1, 2 or 3 in a biological sample obtained from cattle, wherein determining the identity of the at least one SNP comprises determining the identity of the allele present at the SNP position or determining the identity of a genetic variant in linkage disequilibrium with the allele present at the SNP position, wherein said SNP is the second shown in Table 1, namely 23:19329213: C:A, and is associated with susceptibility.
   ---

3-2045. claims: 1-15(partially)

   Methods for classifying and/or selecting cattle according to their susceptibility, tolerance and/or resistance to Mycobacterium avium subsp. paratuberculosis (MAP) infection, comprising determining the identity of at least one single nucleotide polymorphism (SNP) selected from the list shown in Tables 1, 2 or 3 in a biological sample obtained from cattle, wherein determining the identity of the at least one SNP comprises determining the identity of the allele present at the SNP position or determining the identity of a genetic variant in linkage disequilibrium with the allele present at the SNP position, wherein for each invention said SNP is at least one of the further SNPs shown in Tables 1, 2 and 3.
   ---

## ANNEX TO THE EUROPEAN SEARCH REPORT
## ON EUROPEAN PATENT APPLICATION NO.

**EP 23 38 2757**

This annex lists the patent family members relating to the patent documents cited in the above-mentioned European search report.
The members are as contained in the European Patent Office EDP file on
The European Patent Office is in no way liable for these particulars which are merely given for the purpose of information.

**15-01-2024**

| Patent document cited in search report | Publication date | Patent family member(s) | Publication date |
|---|---|---|---|
| US 2010223687 A1 | 02-09-2010 | NONE | |
| CA 2675242 A1 | 11-02-2011 | NONE | |
| US 2016215338 A1 | 28-07-2016 | CA 2716302 A1 | 23-02-2012 |
| | | US 2016215338 A1 | 28-07-2016 |
| | | US 2019226028 A1 | 25-07-2019 |

EPO FORM P0459

For more details about this annex : see Official Journal of the European Patent Office, No. 12/82